# EUROPÄISCHE PATENTSCHRIFT

(11) **EP 1 212 318 B1**
(45) Veröffentlichungstag und Bekanntmachung des Hinweises auf die Patenterteilung: **25.01.2006**
(21) Anmeldenummer: 00958481.4
(22) Anmeldetag: 22.08.2000
(51) Int. Cl.: C07D 401/12, A61K 31/404, A61P 43/00, C07D 209/34, C07D 403/12, C07D 491/04, C07D 401/14, C07D 317/00, C07D 209/00

(54) **SUBSTITUIERTE INDOLINONE ALS TYROSINKINASE INHIBITOREN**
SUBSTITUTED INDOLINONES AS TYROSINE KINASE INHIBITORS
INDOLINONES SUBSTITUEES UTILISEES COMME INHIBITEURS DE LA TYROSINE KINASE

(30) Priorität: 27.08.1999 DE 19940829; 14.06.2000 DE 10029285
(43) Veröffentlichungstag der Anmeldung: 12.06.2002
(73) Patentinhaber: Boehringer Ingelheim Pharma GmbH & Co.KG, 55218 Ingelheim am Rhein (DE)
(72) Erfinder: ROTH, Gerald, Jürgen, 88400 Biberach (DE); HECKEL, Armin, 88400 Biberach (DE); WALTER, Rainer, 88400 Biberach (DE); TONTSCH-GRUNT, Ulrike, A-2500 Baden (AT); SPEVAK, Walter, A-2105 Oberrohrbach (AT); VAN MEEL, Jacobus, C., A., A-2340 Mödling (AT)
(86) Internationale Anmeldenummer: PCT/EP2000/008149
(87) Internationale Veröffentlichungsnummer: WO 2001/016130

(56) Entgegenhaltungen:
- WO-A-99/15500
- DE-A- 19 815 020
- DE-A- 19 816 624

## Beschreibung

Aus WO 99/15500 sind substituierte Oxindole Derivate mit inhibierender Wirkung auf verschiedene Kinasen bekannt. Die Mehrzahl der Ausführungsbeispiele betrifft Verbindungen.die zwingend einen Sulfamid-Rest in der über N oder CH gebundenen Anilin-Teilstruktur enthalten.

Die vorliegende Erfindung betrifft neue substituierte Indolinone der allgemeinen Formel 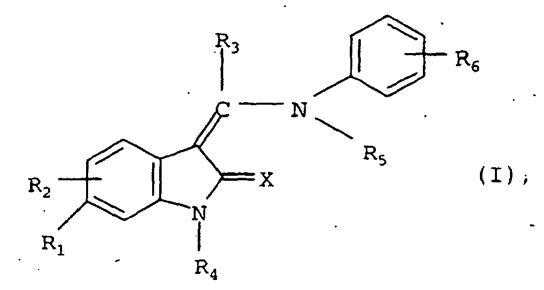 deren Isomere, deren Salze, insbesondere deren physiologisch verträgliche Salze, welche wertvolle Eigenschaften aufweisen.

Die obigen Verbindungen der allgemeinen Formel I, in der R₄ ein Wasserstoffatom oder einen Prodrugrest darstellt, weisen wertvolle pharmakologische Eigenschaften auf, insbesondere eine inhibierende Wirkung auf verschiedene Kinasen, vor allem auf Rezeptor-Tyrosinkinasen wie VEGFR2, PDGFRα, PDGFRβ, FGFR1, FGFR3, EGFR, HER2, IGF1R und HGFR, sowie auf Komplexe von CDK's (Cyclin Dependent Kinases) wie CDK1, CDK2, CDK3, CDK4, CDK5, CDK6, CDK7, CDK8 und CDK9 mit ihren spezifischen Cyclinen (A, B1, B2, C, D1, D2, D3, E, F, G1, G2, H, I und K) und auf virales Cyclin (siehe L. Mengtao in J. Virology 71(3), 1984-1991 (1997)), auf die Proliferation kultivierter humaner Zellen, insbesondere die von Endothelzellen, z.B. bei der Angiogenese, aber auch auf die Proliferation anderer Zellen, insbesondere von Tumorzellen.

Die übrigen Verbindungen der obigen allgemeinen Formel I, in der R₄ kein Wasserstoffatom und keinen Prodrugrest darstellt, stellen wertvolle Zwischenprodukte zur Herstellung der vorstehend erwähnten Verbindungen dar.

Gegenstand der vorliegenden Erfindung sind somit die obigen Verbindungen der allgemeinen Formel I, wobei die Verbindungen, in denen R₄ ein Wasserstoffatom oder einen Prodrugrest darstellt, wertvolle pharmakologische Eigenschaften aufweisen, die die pharmakologisch wirksamen Verbindungen enthaltenden Arzneimittel, deren Verwendung und Verfahren zu ihrer Herstellung.

In der obigen allgemeinen Formel I bedeuten
X ein Sauerstoff- oder Schwefelatom,
R₁ eine C₂₋₃-Alkenyl-, C₂₋₃-Alkinyl-, Aryl-,-Aryl-C₁₋₃-alkyl-, Heteroaryl-, Heteroaryl-C₁₋₃-alkyl-, Trifluormethyl- oder Cyanogruppe,
eine Hydroxy-, C₁₋₃-Alkoxy-, Hydroxy-C₁₋₃-alkyl-, C₁₋₃-Alkoxy-C₁₋₃-alkyl-, Aryloxy- oder Heteroaryloxygruppe,
eine Mercapto-, C₁₋₃-Alkylsulfenyl-, Phenylsulfenyl-, Benzylsulfenyl-, C₁₋₃-Alkylsulfinyl-, Phenylsulfinyl-, Benzylsulfinyl-, C₁₋₃-Alkylsulfonyl-, Phenylsulfonyl-, Benzylsulfonyl-, Sulfo-, C₁₋₃-Alkoxysulfonyl-, Phenoxysulfonyl- oder Benzyloxysulfonylgruppe,
eine Amino-, C₁₋₃-Alkylamino-, Di-(C₁₋₃-Alkyl)-amino-, Hydroxycarbonyl-C₁₋₃-alkylamino-, N-(C₁₋₃-Alkyl)-hydroxycarbonyl-C₁₋₃-alkylamino-, C₁₋₃-Alkoxycarbonyl-C₁₋₃-alkylamino-, N-(C₁₋₃-Alkyl)-C₁₋₃-alkoxycarbonyl-C₁₋₃-alkylamino-, Phenylamino-, N-Phenyl-C₁₋₃-alkylamino-, N,N-Diphenylamino-, Benzylamino-, N-Benzyl-C₁₋₃-alkylamino-, N,N-Dibenzylamino-, C₁₋₃-Alkylcarbonylamino-, Benzoylamino- oder Benzylcarbonylaminogruppe oder eine N-(C₁₋₃-Alkyl)-C₁₋₃-alkylcarbonylaminogruppe, in der die beiden Alkylgruppen durch eine C₂₋₅-n-Alkylenbrücke ersetzt sein können oder in der eine oder beide Alkylgruppen durch eine Phenyl- oder Benzylgruppe ersetzt sein können,
eine C₁₋₃-Alkylsulfonyamino-, Phenylsulfonyamino- oder Benzylsulfonyaminogruppe oder eine N-(C₁₋₃-Alkyl)-C₁₋₃-alkylsulfonylaminogruppe, in der die beiden Alkylgruppen durch eine C₂₋₅-n-Alkylenbrücke ersetzt sein können oder in der eine oder beide Alkylgruppen durch eine Phenyl- oder Benzylgruppe ersetzt sein können,
eine Aminosulfonyl-, C₁₋₃-Alkylaminosulfonyl-, Phenylaminosulfonyl-, Benzylaminosulfonyl-, Di-(C₁₋₃-Alkyl)-aminosulfonyl-, N,N-Diphenyl-aminosulfonyl- oder N,N-Dibenzyl-aminosulfonylgruppe,
eine Phosphono-, (C₁₋₃-Alkoxy)PO(H)-, (C₁₋₃-Alkoxy)PO(C₁₋₃-Alkyl)-, (C₁₋₃-Alkoxy)PO(OH)-, Di-(C₁₋₃-Alkoxy)-PO- oder (C₂₋₄-n-Alkylendioxy)-PO-Gruppe,
eine gegebenenfalls durch C₁₋₃-Alkylgruppen mono-, di- oder trisubstituierte Ureidogruppe,
eine 4- bis 7-gliedrige Cycloalkylenimino- oder Cycloalkyleniminosulfonylgruppe, wobei jeweils die Methylengruppe in Position 4 einer 6- oder 7-gliedrigen Cycloalkyleniminogruppe durch ein Sauerstoff- oder Schwefelatom, durch eine Sulfinyl-, Sulfonyl-, -NH- oder -N(C₁₋₃-Alkyl)-Gruppe ersetzt sein kann,
R₂ ein Wasserstoff-, Fluor-, Chlor-, Brom- oder Jodatom,
eine C₁₋₆-Alkyl- oder Trifluormethylgruppe,
eine Hydroxy-, C₁₋₃-Alkoxy-, Mercapto-, C₁₋₃-Alkylsulfenyl-, C₁₋₃-Alkylsulfinyl-, C₁₋₃-Alkylsulfonyl-, Sulfo-, C₁₋₃-Alkoxysulfonyl-, Aminosulfonyl-, C₁₋₃-Alkylaminosulfonyl- oder Di-(C₁₋₃-Alkyl)-aminosulfonylgruppe,
eine Nitro-, Amino-, C₁₋₃-Alkylamino- oder Di-(C₁₋₃-Alkyl)-aminogruppe,
eine C₁₋₃-Alkylcarbonyl-, Cyano-, Carboxy-, C₁₋₃-Alkoxycarbonyl-, Aminocarbonyl-, C₁₋₃-Alkylaminocarbonyl- oder Di-(C₁₋₃-Alkyl)-aminocarbonylgruppe,
eine Phosphono-, (C₁₋₃-Alkoxy)PO(H)-, (C₁₋₃-Alkoxy)PO(C₁₋₃-Alkyl)-, (C₁₋₃-Alkoxy)PO(OH)- oder Di-(C₁₋₃-Alkoxy) -PO-Gruppe,
eine 4- bis 7-gliedrige Cycloalkylenimino-, Cycloalkyleniminocarbonyl- oder Cycloalkyleniminosulfonylgruppe, wobei jeweils die Methylengruppe in Position 4 einer 6- oder 7-gliedrigen Cycloalkyleniminogruppe durch ein Sauerstoff- oder Schwefelatom, durch eine Sulfinyl-, Sulfonyl-, -NH- oder -N(C₁₋₃-Alkyl)-Gruppe ersetzt sein kann, oder
R₁ und R₂ zusammen eine Methylendioxy-, Ethylendioxy-; n-Propylen-, n-Butylen- oder 1,4-Butadienylengruppe,
R₃ eine Phenylgruppe ,
R₄ ein Wasserstoffatom, eine C₁₋₃-Alkylgruppe oder einen Prodrugrest,
R₅ ein Wasserstoffatom oder eine C₁₋₃-Alkylgruppe und
R₆ ein Wasserstoff-, Fluor-, Chlor-, Brom- oder Jodatom,
eine Trifluormethyl- oder Heteroarylgruppe, eine gegebenenfalls durch 1 bis 3 Fluoratome substituierte C₁₋₃-Alkoxygruppe, eine Amino-C₁₋₃-alkoxy-, C₁₋₃-Alkylamino-C₂₋₃-alkoxy- oder Benzylamino-C₂₋₃-alkoxygruppe, eine Cycloalkylenimino-C₂₋₃-alkoxygruppe mit 4 bis 7 Ringgliedern, eine Di-(C₁₋₃-Alkyl)-amino-C₂₋₃-alkoxy- oder C₁₋₃-Alkylmercaptogruppe,
eine Nitro-, Cyano-, Carboxy-, C₁₋₃-Alkoxycarbonyl-, Aminocarbonyl-, C₁₋₃-Alkylaminocarbonyl-, Di-(C₁₋₃-Alkyl)-aminocarbonyl-, Piperidinocarbonyl- oder Tetrazolylgruppe,
eine am Stickstoffatom gegebenenfalls durch eine C₁₋₃-Alkylgruppe substituierte C₁₋₃-Alkylcarbonylaminogruppe,
eine gegebenenfalls an der Iminogruppe durch eine C₁₋₃-Alkylgruppe substituierte Imidazolyl- oder Piperazinogruppe,
eine C₁₋₄-Alkylgruppe, die terminal
durch eine Hydroxy-, C₁₋₃-Alkoxy-, Carboxy-, C₁₋₃-Alkoxycarbonyl-, Amino-, C₁₋₄-Alkylamino-, Di-(C₁₋₄-Alkyl)-amino-, Phenylamino-, N-Phenyl-C₁₋₃-alkylamino-, Phenyl-n-C₁₋₃-alkylamino-, N-(C₁₋₃-Alkyl)-Phenyl-n-C₁₋₃-alkylamino- oder Di-(Phenyl-n-C₁₋₃-alkyl)-aminogruppe,
durch eine 4- bis 7-gliedrige Cycloalkyleniminogruppe, in der
eine mit der Iminogruppe verknüpfte Methylengruppe durch eine Carbonyl-oder Sulfonylgruppe ersetzt sein kann oder
ein oder zwei Wasserstoffatome jeweils durch eine C₁₋₃-Alkylgruppe ersetzt sein können oder/und
jeweils die Methylengruppe in Position 4 einer 6- oder 7-gliedrigen Cycloalkyleniminogruppe durch eine Carboxy-, C₁₋₃-Alkoxycarbonyl-, Aminocarbonyl-, C₁₋₃-Alkylaminocarbonyl-, Di-(C₁₋₃-Alkyl)-aminocarbonyl-, Phenyl-n-C₁₋₃-alkylamino- oder N-(C₁₋₃-Alkyl)-Phenyl-n-C₁₋₃-alkylaminogruppe substituiert oder
durch ein Sauerstoff- oder Schwefelatom, durch eine Sulfinyl-, Sulfonyl-, -NH- oder -N(C₁₋₃-Alkyl)-Gruppe ersetzt sein kann,
durch eine 5- bis 7-gliedrige Cycloalkenyleniminogruppe, in der die Doppelbindung vom Stickstoffatom isoliert ist,
durch eine C₄₋₇-Cycloalkylamino-, N-(C₁₋₃-Alkyl) - C₄₋₇-cycloalkylamino- oder C₅₋₇-Cycloalkenylaminogruppe, in der die Position 1 des Rings nicht an der Doppelbindung beteiligt ist und in der das Stickstoffatom durch eine C₁₋₃-Alkylgruppe substituiert sein kann,
durch eine C₁₋₃-Alkylcarbonylamino-, N-(C₁₋₃-Alkyl)-C₁₋₃-alkylcarbonylamino-, Aminocarbonyl-, C₁₋₃-Alkylaminocarbonyl- oder Di-(C₁₋₃-Alkyl)-aminocarbonylgruppe substituiert sein kann,
oder R₆ eine Gruppe der Formel

-N(Rₐ)-CO-(CH₂)ₙ-R_{b} (II),

in der
Rₐ eine C₁₋₃-Alkylgruppe,
n eine der Zahlen 0, 1 oder 2 und
R_{b} eine Amino-, C₁₋₄-Alkylamino-, Phenylamino-, N-(C₁₋₄-Alkyl)-phenylamino-, Benzylamino-, N-(C₁₋₄-Alkyl)-benzylamino- oder Di-(C₁₋₄-Alkyl)-aminogruppe oder eine 4- bis 7-gliedrige Cycloalkyleniminogruppe, wobei jeweils die Methylengruppe in position 4 einer 6- oder 7-gliedrigen Cycloalkyleniminogruppe durch ein Sauerstoff- oder Schwefelatom, durch eine Sulfinyl-, Sulfonyl-, -NH- oder -N(C₁₋₃-Alkyl)-Gruppe ersetzt sein kann, bedeuten,
eine Gruppe der Formel

-N(R_{c})-(CH₂)ₘ-(CO)ₒ-R_{d} (III),

in der
R_{c} eine C₁₋₃-Alkyl-, C₁₋₃-Alkylcarbonyl-, Arylcarbonyl-, Benzylcarbonyl-, C₁₋₃-Alkylsulfonyl-, Arylsulfonyl- oder Benzylsulfonylgruppe,
m eine der Zahlen 1, 2, 3 oder 4,
o eine der Zahlen 0 oder 1 bedeuten und
R_{d} die für R_{b} vorstehend erwähnten Bedeutungen besitzt oder eine in 1-Position gegebenenfalls durch eine C₁₋₃-Alkylgruppe substituierte Di-(C₁₋₄-Alkyl)-amino-C₁₋₃-alkylaminogruppe darstellt,
oder eine N-(C₁₋₃-Alkyl)-C₁₋₃-alkylsulfonylaminogruppe,
wobei zusätzlich eine vorhandene Carboxy-, Amino- oder Iminogruppe durch einen in-vivo abspaltbaren Rest (Prodrugrest) substituiert sein kann,
und unter einem von einer Imino- oder Aminogruppe in-vivo abspaltbaren Rest beispielsweise eine Hydroxygruppe, eine Acylgruppe wie die Benzoyl- oder Pyridinoylgruppe oder eine C₁₋₁₆-Alkanoylgruppe wie die Formyl-, Acetyl-, Propionyl-, Butanoyl-, Pentanoyl- oder Hexanoylgruppe, eine Allyloxycarbonylgruppe, eine C₁₋₁₆-Alkoxycarbonylgruppe wie die Methoxycarbonyl-, Ethoxycarbonyl-, Propoxycarbonyl-, Isopropoxycarbonyl-, Butoxycarbonyl-, tert.Butoxycarbonyl-, Pentoxycarbonyl-, Hexyloxycarbonyl-, Octyloxycarbonyl-, Nonyloxycarbonyl-, Decyloxycarbonyl-, Undecyloxycarbonyl-, Dodecyloxycarbonyl- oder Hexadecyloxycarbonylgruppe, eine Phenyl-C₁₋₆-alkoxycarbonylgruppe wie die Benzyloxycarbonyl-, Phenylethoxycarbonyl- oder Phenylpropoxycarbonylgruppe, eine C₁₋₃-Alkylsulfonyl-C₂₋₄-alkoxycarbonyl-, C₁₋₃-Alkoxy-C₂₋₄-alkoxy-C₂₋₄-alkoxycarbonyl- oder RₑCO-O-(R_{f}CR_{g})-O-CO-Gruppe, in der
Rₑ eine C₁₋₈-Alkyl-, C₅₋₇-Cycloalkyl-, Phenyl- oder Phenyl-C₁₋₃-alkylgruppe,
R_{f} ein Wasserstoffatom, eine C₁₋₃-Alkyl-, C₅₋₇-Cycloalkyl- oder Phenylgruppe und
R_{g} ein Wasserstoffatom, eine C₁₋₃-Alkyl- oder RₑCO-O-(R_{f}CR_{g})-O-Gruppe, in der Rₑ bis R_{g} wie vorstehend erwähnt definiert sind, darstellen,
wobei zusätzlich für eine Aminogruppe die Phthalimidogruppe in Betracht kommt, zu verstehen ist, wobei die vorstehend erwähnten Esterreste ebenfalls als in-vivo in eine Carboxygruppe überführbare Gruppe verwendet werden können,
desweiteren sind unter dem Ausdruck eine Arylgruppe eine gegebenenfalls durch ein Fluor-, Chlor-, Brom- oder Iodatom, durch eine Trifluormethyl-, C₁₋₃-Alkyl- oder C₁₋₃-Alkoxygruppe mono- oder disubstituierte Phenyl- oder Naphthylgruppe, wobei im Fall der Disubstitution die Substituenten gleich oder verschieden sein können, und
unter dem Ausdruck eine Heteroarylgruppe eine gegebenenfalls durch eine oder zwei C₁₋₃-Alkylgruppen substituierte monocyclische 5- oder 6-gliedrige Heteroarylgruppe, wobei die 6-gliedrige Heteroarylgruppe ein, zwei oder drei Stickstoffatome und die 5-gliedrige Heteroarylgruppe eine gegebenenfalls durch eine C₁₋₃-Alkylgruppe substituierte Iminogruppe, ein Sauerstoff- oder Schwefelatom oder eine gegebenenfalls durch eine C₁₋₃-Alkylgruppe substituierte Iminogruppe und ein Sauerstoff- oder Schwefelatom oder ein oder zwei Stickstoffatome enthält, und außerdem an die vorstehend erwähnten monocyclischen heterocyclischen Gruppen über zwei benachbarte Kohlenstoffatome ein Phenylring ankondensiert sein kann, zu verstehen.

Hierbei seien die Verbindungen der vorstehend erwähnten allgemeinen Formel I besonders erwähnt, in denen
X und R₁ bis R₅ wie eingangs erwähnt definiert sind und
R₆ mit Ausnahme einer Aminocarbonyl-, C₁₋₃-Alkylaminocarbonyl-, Di-(C₁₋₃-Alkyl)-aminocarbonyl-, Piperidinocarbonyl- oder Tetrazolylgruppe,
einer gegebenenfalls an der Iminogruppe durch eine C₁₋₃-Alkylgruppe substituierten Imidazolyl- oder Piperazinogruppe,
einer C₁₋₄-Alkylgruppe, die terminal Carboxy-, C₁₋₃-Alkoxycarbonylgruppe,
einer N-(C₁₋₃-Alkyl)-C₁₋₃-alkylsulfonylaminogruppe
oder einer Gruppe der Formel

-N(R_{c})-(CH₂)ₘ-(CO)ₒ-R_{d} (III),

in der
R_{c} eine C₁₋₃-Alkylgruppe darstellt, wie eingangs erwähnt definiert ist.
Bevorzugte Verbindungen der obigen allgemeinen Formel I sind diejenigen, in denen
X ein Sauerstoffatom,
R₁ eine C₁₋₃-Alkoxy-, Trifluormethyl-, Di-(C₁₋₃-Alkyl)-amino-, Pyrrolidino- oder Pyrrologruppe,
eine Amino- oder C₁₋₃-Alkylaminogruppe, in denen jeweils ein Aminwasserstoffatom durch eine C₁₋₃-Alkylcarbonyl-, Phenyl-C₁₋₃-alkylcarbonyl-, Benzoyl-, Aminocarbonyl-, C₁₋₃-Alkylsulfonyl-, Phenylsulfonyl-, Carboxy-C₁₋₃-alkyl- oder C₁₋₃-Alkyloxycarbonyl-C₁₋₃-alkylgruppe ersetzt sein kann, oder
eine gegebenenfalls durch eine C₁₋₃-Alkylgruppe substituierte Phenylgruppe,
R₂ ein Wasserstoffatom oder eine C₁₋₃-Alkoxygruppe oder
R₁ und R₂ zusammen eine Methylendioxygruppe,
R₃ eine Phenylgruppe
R₄ ein Wasserstoffatom,
R₅ ein Wasserstoffatom und
R₆ ein Wasserstoff-, Fluor-, Chlor-, Brom-oder Jodatom,
eine Trifluormethyl-, 4-(C₁₋₃-Alkyl)-piperazino-, Pyridinyl-, Imidazolyl-, Tetrazolyl-, C₁₋₃-Alkoxy- oder C₁₋₃-Alkylmercaptogruppe,
eine Nitro-, Cyano-, Carboxy- oder C₁₋₃-Alkyloxycarbonylgruppe oder eine am Stickstoffatom gegebenenfalls durch eine C₁₋₃-Alkylgruppe substituierte C₁₋₃-Alkylcarbonylaminogruppe
eine Piperidinocarbonylgruppe oder eine gegebenenfalls durch eine oder.zwei C₁₋₃-Alkylgruppen substituierte Aminocarbonylgruppe ;
eine C₁₋₃-Alkylgruppe, die terminal
durch eine Amino-, C₁₋₄-Alkylamino-, Di-(C₁₋₄-Alkyl)-amino-, Phenylamino-, N-Phenyl-C₁₋₃-alkylamino-, Phenyl-n-C₁₋₃-alkylamino-, N-(C₁₋₃-Alkyl)-Phenyl-n-C₁₋₃-alkylamino- oder Di-(Phenyl-n-C₁₋₃-alkyl)-aminogruppe, durch eine Pyrrolidino-, Piperidino-, Hexamethylenimino-, Morpholino-, Thiomorpholino-, 1-Oxido-thiomorpholino- oder Piperazinogruppe,
wobei die Piperidinogruppe zusätzlich durch eine oder zwei C₁₋₃-Alkylgruppen oder durch eine Carboxy-, C₁₋₃-Alkoxycarbonyl-, Aminocarbonyl-, C₁₋₃-Alkylaminocarbonyl- Di-(C₁₋₃-Alkyl)-aminocarbonyl- oder N-(C₁₋₃-Alkyl)-Phenyl-n-C₁₋₃-alkylaminogruppe substituiert sein kann,
durch eine C₅₋₇-Cycloalkylamino- oder C₅₋₇-Cycloalkenylaminogruppe, in der die Position 1 des Rings.nicht an der Doppelbindung beteiligt ist,
durch eine C₁₋₃-Alkylcarbonylamino-, N-(C₁₋₃-Alkyl)-C₁₋₃-alkylcarbonylamino-, Carboxy-, C₁₋₃-Alkoxycarbonyl-, Aminocarbonyl-, C₁₋₃-Alkylaminocarbonyl- oder Di-(C₁₋₃-Alkyl)-aminocarbonylgruppe substituiert sein kann,
eine C₁₋₃-Alkoxygruppe, die terminal durch eine Amino-, C₁₋₃-Alkylamino- oder Di-(C₁₋₃-Alkyl)-aminogruppe substituiert ist,
eine Gruppe der Formel

-N(Rₐ)-CO-(CH₂)ₙ-R_{b} (II),

in der
Rₐ eine C₁₋₃-Alkylgruppe,
n eine der Zahlen 0, 1 oder 2 und
R_{b} eine Amino-, C₁₋₄-Alkylamino- oder Di- (C₁₋₄-Alkyl)-aminogruppe oder eine Pyrrolidino-, Piperidino-, Hexamethylenimino-, Morpholino-, Thiomorpholino-, 1-Oxido-thiomorpholino- oder Piperazinogruppe bedeuten,
eine Gruppe der Formel

-N(R_{c})-(CH₂)ₘ-(CO)ₒ-R_{d} (III),

in der
R_{c} eine C₁₋₃-Alkyl-, C₁₋₃-Alkylcarbonyl- oder C₁₋₃-Alkylsulfonylgruppe,
m eine der Zahlen 1, 2, 3 oder 4,
o eine der Zahlen 0 oder 1 bedeuten und
R_{d} die für R_{b} vorstehend erwähnten Bedeutungen besitzt oder eine in 1-Position gegebenenfalls durch eine C₁₋₃-Alkylgruppe substituierte Di-(C₁₋₄-Alkyl)-amino-C₁₋₃-alkylaminogruppe darstellt,
oder eine N-(C₁₋₃-Alkyl)-C₁₋₃-alkylsulfonylaminogruppe,
bedeuten, deren Isomere und deren Salze.

Besonders bevorzugte Verbindungen der obigen allgemeinen Formel I sind diejenigen, in denen
X ein Sauerstoffatom,
R₁ eine Methoxy-, Ethoxy-, Trifluormethyl-, Phenyl-, Methylphenyl-, Dimethylamino-, Pyrrolidino- oder Pyrrologruppe,
eine Aminogruppe, die durch eine Methyl-, Carboxymethyl-, Methoxycarbonylmethyl-, Acetyl-, Phenylacetyl-, Benzoyl-, Methansulfonyl-, Benzolsulfonyl- oder Aminocarbonylgruppe substituiert sein kann,
R₂ ein Wasserstoffatom, eine Methoxy- oder Ethoxygruppe oder
R₁ und R₂ zusammen eine Methylendioxygruppe,
R₃ eine Phenylgruppe,
R₄ ein Wasserstoffatom,
R₅ ein Wasserstoffatom und
R₆ ein Wasserstoff-, Fluor-, Chlor-, Brom- oder Jodatom,
eine Methyl-, Trifluormethyl-, Methoxy-, Ethoxy-, Methylmercapto-, Cyano-, Carboxy-, Methoxycarbonyl-, Ethoxycarbonyl-, Aminocarbonyl-, Dimethylaminocarbonyl-, Piperidinocarbonyl-, Nitro-, 4-Methyl-piperazino-, Imidazolyl-, Pyridinyl- oder Tetrazolylgruppe,
eine endständig durch eine Dimethylaminogruppe substituierte Ethyloxy- oder n-Propyloxygruppe,
eine durch eine Carboxy, Methoxycarbonyl-, Ethoxycarbonyl-, Aminocarbonyl- oder Dimethylaminocarbonylgruppe substituierte Methyl- oder Ethylgruppe,
eine C₁₋₃-Alkylgruppe, die endständig
durch eine Amino-, C₁₋₄-Alkylamino-, Cyclohexylamino-, Benzylamino- oder Phenylaminogruppe, in denen jeweils ein Wasserstoffatom des Aminstickstoffatoms durch eine C₁₋₃-Alkyl-, Benzyl-, Acetyl- oder Dimethylaminocarbonylgruppe ersetzt sein kann,
durch eine gegebenenfalls durch eine oder zwei Methylgruppen substituierte Piperidinogruppe,
durch eine durch eine Carboxy-, Methoxycarbonyl-, Ethoxycarbonyl- oder Dimethylaminocarbonylgruppe substituierte Piperidinogruppe,
durch eine Pyrrolidino-, 3,4-Dehydro-piperidino-, Hexamethylenimino-, Morpholino-, Thiomorpholino-, 1-Oxo-thiomorpholino- oder Piperazinogruppe substituiert ist,
eine C₁₋₃-Alkylaminogruppe, in der das Wasserstoffatom des Aminstickstoffatoms
durch eine Ethyl- oder n-Propylgruppe, die jeweils endständig durch eine Dimethylaminogruppe substuituiert ist,
durch eine C₂₋₃-Alkanoylgruppe, die in 2- oder 3-Stellung durch eine Amino-, C₁₋₃-Alkylamino-, Di-(C₁₋₃-Alkyl)-amino-, Pyrrolidino-, Piperidino-, Morpholino- oder Piperazinogruppe substituiert sein kann,
durch eine Aminocarbonyl-, Methylaminocarbonyl-, Dimethylaminocarbonyl-, Piperidinocarbonyl- oder Methansulfonylgruppe ersetzt ist,
und zusätzlich der C₁₋₃-Alkylteil der C₁₋₃-Alkylaminogruppe
durch eine Aminocarbonylgruppe,
durch eine C₁₋₃-Alkylaminocarbonyl- oder Di-(C₁₋₃-Alkyl)-aminocarbonylgruppe, in denen ein C₂₋₃-Alkylteil endständig zusätzlich durch eine Dimethylaminogruppe substituiert sein kann,
durch eine Pyrrolidinocarbonyl-, Piperidinocarbonyl-, Morpholinocarbonyl- oder Piperazinocarbonylgruppe substituiert sein kann,
wobei der C₂₋₃-Alkylteil der vorstehend erwähnten C₁₋₃-Alkylaminogruppe endständig außerdem durch eine Amino-, C₁₋₃-Alkylamino-, Di-(C₁₋₃-Alkyl)-amino-, Pyrrolidino-, Piperidino-, Morpholino- oder Piperazinogruppe substituiert sein kann,
bedeuten,
insbesondere diejenigen Verbindungen der obigen allgemeinen Formel I sind diejenigen, in denen
entweder X, R₁ und R₃ bis R₆ wie vorstehend erwähnt definiert sind und
R₂ ein Wasserstoffatom darstellt,
oder X und R₃ bis R₆ wie vorstehend erwähnt definiert sind, R₁ und R₂, die gleich oder verschieden sein können, jeweils eine C₁₋₃-Alkoxygruppe darstellen,
deren Isomere und deren Salze.

Ganz besonders bevorzugte Verbindungen der obigen allgemeinen Formel I sind diejenigen, in denen
X ein Sauerstoffatom,
R₁ eine Amino-, Methoxy- oder Ethoxygruppe,
R₂ ein Wasserstoffatom oder in Position 5 eine Methoxy- oder Ethoxygruppe,
R₃ eine Phenylgruppe,
R₄ und R₅ jeweils ein Wasserstoffatom und
R₆ eine durch eine Methylamino-, Ethylamino-, Piperidino- oder 4-(Dimethylaminocarbonyl)-piperidinogruppe substituierte Methyl- oder Ethylgruppe, wobei das Amino-Wasserstoffatom der Methylamino- und Ethylaminogruppe durch eine Methyl- oder Benzylgruppe ersetzt ist, eine N-Dimethylaminomethylcarbonyl-N-methyl-aminogruppe oder eine N-Acetyl-N-(C₂₋₃-alkyl)-aminogruppe, in der der C₂₋₃-Alkylteil jeweils endständig durch eine Dimethylaminogruppe substituiert ist, bedeuten,
und deren Salze.

Beispielsweise seien folgende besonders wertvolle Verbindungen der allgemeinen Formel I erwähnt:
(a) 3-(Z)-{1-[4-(Piperidin-1-yl-methyl)-anilino]-1-phenyl-methyliden}-5,6-dimethoxy-2-indolinon,
(b) 3-(Z)-(1-{4-[(N-Benzyl-N-methyl-amino)-methyl]-anilino}-1-phenyl-methyliden)-5,6-dimethoxy-2-indolinon,
(c) 3-(Z)-{1-(4-(Dimethylamino-methyl)-anilino]-1-phenyl-methyliden}-5,6-dimethoxy-2-indolinon,
(d) 3-(Z)-{1-[4-(N-Dimethylaminomethylcarbonyl-N-methyl-amino)-anilino]-1-phenyl-methyliden}-5,6-dimethoxy-2-indolinon,
(e) 3-(Z)-(1-{4-[2-(4-Dimethylcarboxamid-piperidin-1-yl)-ethyl]-anilino}-1-phenyl-methyliden)-5,6-dimethoxy-2-indolinon,
(f) 6-Amino-3-(Z)-{1-[4-(piperidin-1-yl-methyl)-anilino]-1-phenyl-methyliden}-2-indolinon,
(g) 3-(Z)-(1-{4-[N-Acetyl-N-(2-dimethylamino-ethyl)-amino]-anilino}-1-phenyl-methyliden)-5,6-dimethoxy-2-indolinon und
(h) 3-(Z)-(1-{4-[N-Acetyl-N-(3-dimethylamino-propyl) -amino] - anilino}-1-phenyl-methyliden)-5,6-dimethoxy-2-indolinon

sowie deren Salze.

Erfindungsgemäß erhält man die neuen Verbindungen beispielsweise nach folgenden.im Prinzip literaturbekannten Verfahren:
a. Umsetzung einer Verbindung der allgemeinen Formel 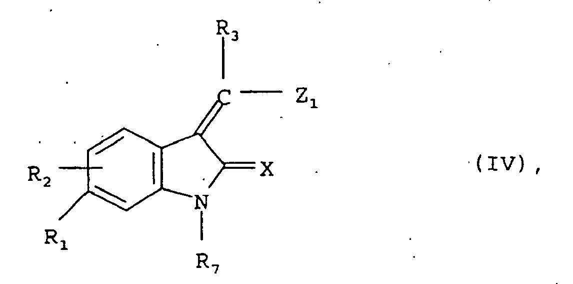
   in der
   X und R₁ bis R₃ wie in den Ansprüchen 1 bis 4 erwähnt definiert sind,
   R₇ ein Wasserstoffatom, eine Schutzgruppe für das Stickstoffatom der Lactamgruppe oder eine Bindung an eine Festphase und
   Z₁ ein Halogenatom, eine Hydroxy-, Alkoxy- oder Arylalkoxygruppe, z.B. ein Chlor- oder Bromatom, eine Methoxy-, Ethoxy- oder Benzyloxygruppe, bedeuten,
   mit einem Amin der allgemeinen Formel 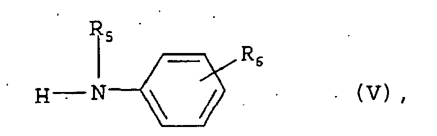
   in der
   R₅ und R₆ wie in den Ansprüchen 1 bis 4 erwähnt definiert
   sind,
   und erforderlichenfalls anschließende Abspaltung einer verwendeten Schutzgruppe für das Stickstoffatom der Lactamgruppe
   oder von einer Festphase.
   Als Schutzgruppe für das Stickstoffatom der Lactamgruppe kommt beispielsweise eine Acetyl-, Benzoyl-, Ethoxycarbonyl-, tert.Butyloxycarbonyl- oder Benzyloxycarbonylgruppe und
   als Festphase ein Rink-Harz wie ein p-Benzyloxybenzylalkoholharz, wobei die Bindung zweckmäßigerweise über ein Zwischenglied wie ein 2,5-Dimethoxy-4-hydroxy-benzylderivat erfolgt, in Betracht.
   Die Umsetzung wird zweckmäßigerweise in einem Lösungsmittel wie Dimethylformamid, Toluol, Acetonitril, Tetrahydrofuran, Dimethylsulfoxid, Methylenchlorid oder deren Gemischen gegebenenfalls in Gegenwart einer inerten Base wie Triethylamin, N-Ethyl-diisopropylamin oder Natriumhydrogencarbonat bei Temperaturen zwischen 20 und 175°C durchgeführt, wobei eine verwendete Schutzgruppe infolge Umamidierung gleichzeitig abgespalten werden kann.
   Bedeutet Z₁ in einer Verbindung der allgemeinen Formel IV ein Halogenatom, dann wird die Umsetzung vorzugsweise in Gegenwart einer inerten Base bei Temperaturen zwischen 20 und 120°C, durchgeführt.
   Bedeutet Z₁ in einer Verbindung der allgemeinen Formel IV eine Hydroxy-, Alkoxy- oder Arylalkoxygruppe, dann wird die Umsetzung vorzugsweise bei Temperaturen zwischen 20 und 200°C, durchgeführt.
   Die gegebenenfalls erforderliche anschließende Abspaltung einer verwendeten Schutzgruppe wird zweckmäßigerweise entweder hydrolytisch in einem wäßrigen oder alkoholischen Lösungsmittel, z.B. in Methanol/Wasser, Ethanol/Wasser, Isopropanol/Wasser, Tetrahydrofuran/Wasser, Dioxan/Wasser, Dimethylformamid/Wasser, Methanol oder Ethanol in Gegenwart einer Alkalibase wie Lithiumhydroxid, Natriumhydroxid oder Kaliumhydroxid bei Temperaturen zwischen 0 und 100°C, vorzugsweise bei Temperaturen zwischen 10 und 50°C,
   oder vorteilhafterweise durch Umamidierung mit einer primären oder sekundären organischen Base wie Butylamin, Dimethylamin oder Piperidin in einem Lösungsmittel wie Methanol,' Ethanol, Dimethylformamid und deren Gemischen oder in einem Überschuß des eingesetzten Amins bei Temperaturen zwischen 0 und 100°C, vorzugsweise bei Temperaturen zwischen 10 und 50°C, durchgeführt.
   Die Abspaltung von einer verwendeten Festphase erfolgt vorzugsweise mittels Trifluoressigsäure und Wasser in Gegenwart von einem Dialkylsulfid wie Dimethylsulfid bei Temperaturen zwischen 0 und 35°C, vorzugsweise bei Raumtemperatur.
b. Zur Herstellung einer Verbindung der allgemeinen Formel I, in der R₁ eine Aminogruppe darstellt:
   Reduktion einer Verbindung der allgemeinen Formel 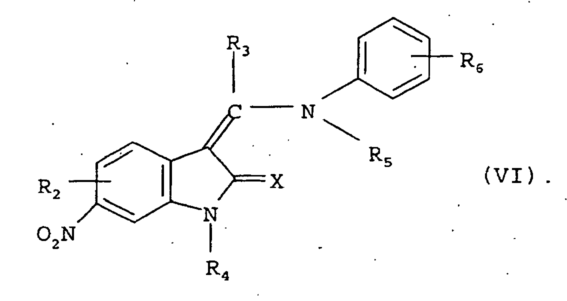
   Die Reduktion einer Nitrogruppe erfolgt vorzugsweise hydrogenolytisch, z.B. mit Wasserstoff in Gegenwart eines Katalysators wie Palladium/Kohle oder Raney-Nickel in einem Lösungsmittel wie Methanol, Ethanol, Essigsäureethylester, Dimethylformamid, Dimethylformamid/Aceton oder Eisessig gegebenenfalls unter Zusatz einer Säure wie Salzsäure oder Eisessig bei Temperaturen zwischen 0 und 50°C, vorzugsweise jedoch bei Raumtemperatur, und bei einem Wasserstoffdruck von 1 bis 7 bar, vorzugsweise jedoch von 3 bis 5 bar.
c. Zur Herstellung einer Verbindung der allgemeinen Formel I, in der R₁ oder/und R₂ eine der eingangs erwähnten substituierten Sulfinyl- oder Sulfonylgruppen darstellt:
   Oxidation einer Verbindung der allgemeinen Formel 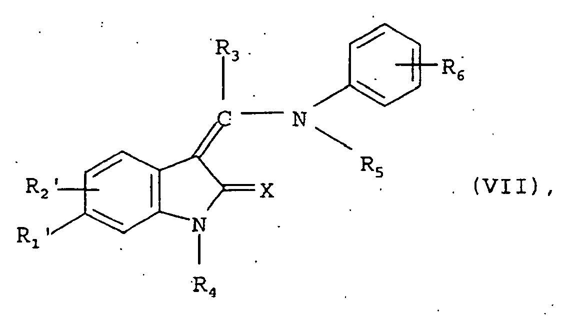
   in der
   R₃ bis R₆ wie in den Ansprüchen 1 bis 4 erwähnt definiert sind und
   eine der Gruppen R₁' und R₂' eine der für R₁ und R₂ eingangs erwähnten substituierten Mercapto- oder Sulfinylgruppen bedeutet und die andere die für R₁ oder R₂ mit Ausnahme der Mercapto- oder Sulfinylgruppen eingangs erwähnten Bedeutungen annimmt oder beide Gruppen R₁ und R₂' eine der für R₁ und R₂ eingangs erwähnten substituierten Mercapto- oder Sulfinylgruppen darstellen.

Die Oxidation wird vorzugsweise in einem Lösungsmittel oder Lösungsmittelgemisch, z.B. in Wasser, Wasser/Pyridin, Aceton, Methylenchlorid, Essigsäure, Essigsäure/Acetanhydrid, verdünnter Schwefelsäure oder Trifluoressigsäure, je nach dem verwendeten Oxidationsmittel zweckmäßigerweise bei Temperaturen zwischen -80 und 100°C durchgeführt.

Zur Herstellung einer entsprechenden Sulfinylverbindung der allgemeinen Formel I wird die Oxidation zweckmäßigerweise mit einem Äquivalent des verwendeten Oxidationsmittels durchgeführt, z.B. mit Wasserstoffperoxid in Eisessig, Trifluoressigsäure oder Ameisensäure bei 0 bis 20°C oder in Aceton bei 0 bis 60°C, mit einer Persäure wie Perameisensäure in Eisessig oder Trifluoressigsäure bei 0 bis 50°C oder mit m-Chlorperbenzoesäure in Methylenchlorid, Chloroform oder Dioxan bei -20 bis 80°C, mit Natriummetaperjodat in wäßrigem Methanol oder Ethanol bei -15 bis 25°C, mit Brom in Eisessig oder wäßriger Essigsäure gegebenenfalls in Gegenwart einer schwachen Base wie Natriumacetat, mit N-Bromsuccinimid in Ethanol, mit tert.Butylhypochlorit in Methanol bei -80 bis -30°C, mit Jodbenzodichlorid in wäßrigem Pyridin bei 0 bis 50°C, mit Salpetersäure in Eisessig bei 0 bis 20°C, mit Chromsäure in Eisessig oder in Aceton bei 0 bis 20°C und mit Sulfurylchlorid in Methylenchlorid bei -70°C, der hierbei erhaltene Thioether-Chlor-Komplex wird zweckmäßigerweise mit wäßrigem Ethanol hydrolysiert.

Zur Herstellung einer Sulfonylverbindung der allgemeinen Formel I wird die Oxidation ausgehend von einer entsprechenden Sulfinylverbindung zweckmäßigerweise mit einem oder mehr Äquivalenten des verwendeten Oxidationsmittels oder ausgehend von einer entsprechenden Mercaptoverbindung zweckmäßigerweise mit.zwei oder mehr Äquivalenten des verwendeten Oxidationsmittels durchgeführt, z.B. mit Wasserstoffperoxid in.Eisessig/-Acetanhydrid, Trifluoressigsäure oder in Ameisensäure bei 20 bis 100°C oder in Aceton bei 0 bis 60°C, mit einer Persäure wie Perameisensäure oder m-Chlorperbenzoesäure in Eisessig, Trifluoressigsäure, Methylenchlorid oder Chloroform bei Temperaturen zwischen 0 und 60°C, mit Salpetersäure in Eisessig bei 0 bis 20°C, mit Chromsäure, Natriumperjodat oder Kaliumpermanganat in Essigsäure, Wasser/Schwefelsäure oder in Aceton bei 0 bis 20°C.

Erhält man erfindungsgemäß eine Verbindung der allgemeinen Formel I, die eine Alkoxycarbonylgruppe enthält, so kann diese mittels Hydrolyse in eine entsprechende Carboxyverbindung übergeführt werden, oder
eine Verbindung der allgemeinen Formel I, die eine Amino- oder Alkylaminogruppe enthält, so kann diese mittels Alkylierung oder reduktiver Alkylierung in eine.entsprechende Alkylamino-, Dialkylamino- oder Pyrrolidinoverbindung übergeführt werden, oder
eine Verbindung der allgemeinen Formel I, die eine Amino- oder Alkylaminogruppe enthält, so kann diese mittels Acylierung in eine entsprechende Acylverbindung übergeführt werden, oder
eine Verbindung der allgemeinen Formel I, die eine Amino- oder Alkylaminogruppe enthält, so kann diese mittels Sulfonierung in eine entsprechende Sulfonylverbindung übergeführt werden, beispielsweise in die entsprechende Alkylsulfonyamino-, Phenylsulfonyamino-, Benzylsulfonyamino- oder N-Alkyl-alkylsulfonylaminogruppe, oder
eine Verbindung der allgemeinen Formel I, die eine Aminogruppe enthält, so kann diese mittels einer Kondensationreaktion in eine entsprechende Pyrroloverbindung übergeführt werden, oder
eine Verbindung der allgemeinen Formel I, die eine Carboxygruppe enthält, so kann diese mittels Veresterung oder Amidierung in eine entsprechende Ester- oder Aminocarbonylverbindung übergeführt werden, oder
eine Verbindung der allgemeinen Formel I, die eine Cyanogruppe enthält, so kann diese mittels Reduktion in eine entsprechende Aminomethylverbindung übergeführt werden, oder
eine Verbindung der allgemeinen Formel I, die eine Amino- oder Alkylaminogruppe enthält, so kann diese mittels Umsetzung mit Cyansäure oder einem entsprechenden Isocyanat in eine entsprechende Ureidoverbindung übergeführt werden.

Die anschließende Hydrolyse erfolgt vorzugsweise in einem wäßrigen Lösungsmittel, z.B. in Wasser, Methanol/Wasser, Ethanol/Wasser, Isopropanol/Wasser, Tetrahydrofuran/Wasser oder Dioxan/Wasser, in Gegenwart einer Säure wie Trifluoressigsäure, Salzsäure oder Schwefelsäure oder in Gegenwart einer Alkalibase wie Lithiumhydroxid, Natriumhydroxid oder Kaliumhydroxid bei Temperaturen zwischen 0 und 100°C, vorzugsweise bei Temperaturen zwischen 10 und 50°C.

Die anschließende reduktive Alkylierung wird vorzugsweise in einem geeigneten Lösungsmittel wie Methanol, Methanol/Wasser, Methanol/Wasser/Ammoniak, Ethanol, Ether, Tetrahydrofuran, Dioxan, Methylenchlorid oder Dimethylformamid gegebenenfalls unter Zusatz einer Säure wie Salzsäure in Gegenwart von katalytisch angeregtem Wasserstoff, z.B. von Wasserstoff in Gegenwart von Raney-Nickel, Platin oder Palladium/Kohle, oder in Gegenwart eines Metallhydrids wie Natriumborhydrid, Natriumcyanoborhydrid, Lithiumborhydrid oder Lithiumaluminiumhydrid bei Temperaturen zwischen 0 und 100°C, vorzugsweise bei Temperaturen zwischen 20 und 80°C, durchgeführt.

Die anschließende Alkylierung wird mit einem Alkylierungsmittel wie einem Alkylhalogenid oder Dialkylsulfat wie Methyljodid, Dimethylsulfat oder Propylbromid vorzugsweise in einem Lösungsmittel wie Methanol, Ethanol, Methylenchlorid, Tetrahydrofuran, Toluol, Dioxan, Dimethylsulfoxid oder Dimethylformamid gegebenenfalls in Gegenwart einer anorganischen oder einer tertiären organischen Base wie Kaliumcarbonat, Triethylamin, N-Ethyl-diisopropylamin, Pyridin oder Dimethylaminopyridin, vorzugsweise bei Temperaturen zwischen 20°C und der Siedetemperatur des verwendeten Lösungsmittel, durchgeführt.

Die anschließende Acylierung wird vorzugsweise in einem Lösungsmittel wie Methylenchlorid, Diethylether, Tetrahydrofuran, Toluol, Dioxan, Acetonitril, Dimethylsulfoxid oder Dimethylformamid gegebenenfalls in Gegenwart einer anorganischen oder einer tertiären organischen Base, vorzugsweise bei Temperaturen zwischen 20°C und der Siedetemperatur des verwendeten Lösungsmittel, durchgeführt. Hierbei wird die Acylierung mit einer entsprechenden Säure vorzugsweise in Gegenwart eines wasserentziehenden Mittels, z.B. in Gegenwart von Chlorameisensäureisobutylester, Orthokohlensäuretetraethylester, Orthoessigsäuretrimethylester, 2,2-Dimethoxypropan, Tetramethoxysilan, Thionylchlorid, Trimethylchlorsilan, Phosphortrichlorid, Phosphorpentoxid, N,N'-Dicyclohexylcarbodiimid, N,N'-Dicyclohexylcarbodiimid/N-Hydroxysuccinimid, N,N'-Dicyclohexylcarbodiimid/1-Hydroxy-benztriazol, 2-(1H-Benzotriazol-1-yl)-1,1,3,3-tetramethyluronium-tetrafluorborat, 2-(1H-Benzotriazol-1-yl)-1,1,3,3-tetramethyluronium-tetrafluorborat/1-Hydroxy-benztriazol, N,N'-Carbonyldiimidazol oder Triphenylphosphin/Tetrachlorkohlenstoff, und gegebenenfalls unter Zusatz einer Base wie Pyridin, 4-Dimethylamino-pyridin, N-Methyl-morpholin oder Triethylamin zweckmäßigerweise bei Temperaturen zwischen 0 und 150°C, vorzugsweise bei Temperaturen zwischen 0 und 100°C, und die Acylierung mit einer entsprechenden reaktionsfähigen Verbindung wie deren Anhydrid, Ester, Imidazolide oder Halogenide gegebenenfalls in Gegenwart einer tertiären organischen Base wie Triethylamin, N-Ethyl-diisopropylamin, N-Methyl-morpholin oder Pyridin bei Temperaturen zwischen 0 und 150°C, vorzugsweise bei Temperaturen zwischen 50 und 100°C, durchgeführt.

Die anschließende Sulfonierung wird vorzugsweise in einem Lösungsmittel wie Methylenchlorid, Diethylether, Tetrahydrofuran, Toluol, Dioxan, Acetonitril, Dimethylsulfoxid oder Dimethylformamid gegebenenfalls in Gegenwart einer anorganischen oder einer tertiären organischen Base, vorzugsweise bei Temperaturen zwischen 20°C und der Siedetemperatur des verwendeten Lösungsmittel, durchgeführt. Hierbei wird die Sulfonierung mit einer entsprechenden reaktionsfähigen Verbindung, beispielsweise den Sulfonylhalogeniden, gegebenenfalls in Gegenwart einer tertiären organischen Base wie Triethylamin, N-Ethyl-diisopropylamin, N-Methyl-morpholin, Pyridin oder Dimethylaminopyridin bei Temperaturen zwischen 0 und 150°C, vorzugsweise bei Temperaturen zwischen 50 und 100°C, durchgeführt.

Die anschließende Kondensation zur Überführung einer Aminogruppe in eine Pyrrologruppe wird zweckmäßigerweise in einem Lösungsmittel wie Dimethylformamid, Toluol; Acetonitril, Tetrahydrofuran, Dimethylsulfoxid, Methylenchlorid oder.deren Gemischen, vorzugsweise in Gegenwart einer Säure wie Ameisensäure, Eisessig oder Trifluoressigsäure oder unter Verwendung der Säure als alleiniges Lösungsmittel mit kondensationsfähigen 1,4-difunktionellen n-Butylen- oder Tetrahydrofuranderivaten, beispielsweise mit 2,5-Dimethoxytetrahydrofuran oder 2,5-Hexandion, bei Temperaturen zwischen 0 und 120°C oder der Siedetemperatur der Reaktionsmischung durchgeführt.

Die anschließende Veresterung oder Amidierung wird zweckmäßigerweise durch Umsetzung eines reaktionsfähigen entsprechenden Carbonsäurederivates mit einem entsprechenden Alkohol oder Amin wie vorstehend beschrieben durchgeführt.

Die anschließende Reduktion einer Cyanogruppe wird vorzugsweise in einem geeigneten Lösungsmittel wie Methanol, Methanol/Wasser, Methanol/Wasser/Ammoniak, Ethanol, Ether, Tetrahydrofuran, Dioxan, Methylenchlorid oder Dimethylformamid gegebenenfalls unter Zusatz von methanolischem Ammoniak mit Wasserstoff in Gegenwart eines Hydrierungskatalysators, z.B. in Gegenwart von Raney-Nickel oder Palladium/Kohle, bei einem Wasserstoffdruck von 1 bis 5 bar, vorzugsweise bei Temperaturen zwischen 20°C und der Siedetemperatur des verwendeten Lösungsmittel, durchgeführt.

Die anschließende Umsetzung mit einer Cyansäure oder einem entsprechenden Isocyanat wird vorzugsweise in einem geeigneten Lösungsmittel wie Ethanol, Tetrahydrofuran oder Dioxan bei Temperaturen zwischen 0°C und der Siedetemperatur des Reaktionsgemisches durchgeführt, hierbei wird die eingesetzte Cyansäure zweckmäßigerweise durch Umsetzung eines Salzes der Cyansäure mit einer Säure, beispielsweise durch Umsetzung von Kaliumcyanat mit Eisessig, im Reaktionsgemisch hergestellt.

Bei den vorstehend beschriebenen Umsetzungen können gegebenenfalls vorhandene reaktive Gruppen wie Carboxy-, Amino-, Alkylamino- oder Iminogruppen während der Umsetzung durch übliche Schutzgruppen geschützt werden, welche nach der Umsetzung wieder abgespalten werden.

Beispielsweise kommt als Schutzrest für eine Carboxylgruppe die Trimethylsilyl-, Methyl-, Ethyl-, tert.Butyl-, Benzyl- oder Tetrahydropyranylgruppe und
als Schutzrest für eine Amino-, Alkylamino- oder Iminogruppe die Acetyl-, Trifluoracetyl-, Benzoyl-, Ethoxycarbonyl-, tert.Butoxycarbonyl-, Benzyloxycarbonyl-, Benzyl-, Methoxybenzyl- oder 2,4-Dimethoxybenzyl-gruppe und für die Aminogruppe zusätzlich die Phthalylgruppe in Betracht.

Die gegebenenfalls anschließende Abspaltung eines verwendeten Schutzrestes erfolgt beispielsweise hydrolytisch in einem wäßrigen Lösungsmittel, z.B. in Wasser, Isopropanol/Wasser, Tetrahydrofuran/Wasser oder Dioxan/Wasser, in Gegenwart einer Säure wie Trifluoressigsäure, Salzsäure oder Schwefelsäure oder in Gegenwart einer Alkalibase wie Lithiumhydroxid, Natriumhydroxid oder Kaliumhydroxid bei Temperaturen zwischen 0 und 100°C, vorzugsweise bei Temperaturen zwischen 10 und 50°C.

Die Abspaltung eines Benzyl-, Methoxybenzyl- oder Benzyloxycarbonylrestes erfolgt jedoch beispielsweise hydrogenolytisch, z.B. mit Wasserstoff in Gegenwart eines Katalysators wie Palladium/Kohle in einem Lösungsmittel wie Methanol, Ethanol, Essigsäureethylester, Dimethylformamid, Dimethylformamid/Aceton oder Eisessig gegebenenfalls unter Zusatz einer Säure wie Salzsäure oder Eisessig bei Temperaturen zwischen 0 und 50°C, vorzugsweise jedoch bei Raumtemperatur, und bei einem Wasserstoffdruck von 1 bis 7 bar, vorzugsweise jedoch von 3 bis 5 bar.

Die Abspaltung einer Methoxybenzylgruppe kann auch in Gegenwart eines Oxidationsmittels wie Cer(IV)ammoniumnitrat in einem Lösungsmittel wie Methylenchlorid, Acetonitril oder Acetonitril/Wasser bei Temperaturen zwischen 0 und 50°C, vorzugsweise jedoch bei Raumtemperatur, erfolgen.

Die Abspaltung eines 2,4-Dimethoxybenzylrestes erfolgt jedoch vorzugsweise in Trifluoressigsäure in Gegenwart von Anisol.

Die Abspaltung eines tert.Butyl- oder tert.Butyloxycarbonylrestes erfolgt vorzugsweise durch Behandlung mit einer Säure wie Trifluoressigsäure oder Salzsäure gegebenenfalls unter Verwendung eines Lösungsmittels wie Methylenchlorid, Dioxan, Essigester oder Ether.

Die Abspaltung eines Phthalylrestes erfolgt vorzugsweise in Gegenwart von Hydrazin oder eines primären Amins wie Methylamin, Ethylamin oder n-Butylamin in einem Lösungsmittel wie Methanol, Ethanol, Isopropanol, Toluol/Wasser oder Dioxan bei Temperaturen zwischen 20 und 50°C.

Ferner können erhaltene chirale Verbindungen der allgemeinen Formel I in ihre Enantiomeren und/oder Diastereomeren aufgetrennt werden.

So lassen sich beispielsweise die erhaltenen Verbindungen der allgemeinen Formel I, welche in Racematen auftreten, nach an sich bekannten Methoden (siehe Allinger N. L. und Eliel E. L. in "Topics in Stereochemistry", Vol. 6, Wiley Interscience, 1971) in ihre optischen Antipoden und Verbindungen der allgemeinen Formel I mit mindestes 2 asymmetrischen Kohlenstoffatomen auf Grund ihrer physikalisch-chemischen Unterschiede nach an sich bekannten Methoden, z.B. durch Chromatographie und/oder fraktionierte Kristallisation, in ihre Diastereomeren auftrennen, die, falls sie in racemischer Form anfallen, anschließend wie oben erwähnt in die Enantiomeren getrennt werden können.

Die Enantiomerentrennung erfolgt vorzugsweise durch Säulentrennung an chiralen Phasen oder durch Umkristallisieren aus einem optisch aktiven Lösungsmittel oder durch Umsetzen mit einer, mit der racemischen Verbindung Salze oder Derivate wie z.B. Ester oder Amide bildenden optisch aktiven Substanz, insbesondere Säuren und ihre aktivierten Derivate oder Alkohole, und Trennen des auf diese Weise erhaltenen Gemisches diastereomerer Salze oder Derivate, z.B. auf Grund von verschiedenen Löslichkeiten, wobei aus den reinen diastereomeren Salzen oder Derivaten die freien Antipoden durch Einwirkung geeigneter Mittel freigesetzt werden können. Besonders gebräuchliche, optisch aktive Säuren sind z.B. die D- und L-Formen von Weinsäure, Dibenzoylweinsäure, Di-o-Tolylweinsäure, Apfelsäure, Mandelsäure, Camphersulfonsäure, Glutaminsäure, N-Acetylglutaminsäure, Asparaginsäure, N-Acetyl-asparäginsäure oder Chinasäure. Als optisch aktiver Alkohol kommt beispielsweise (+)- oder (-)-Menthol und als optisch aktiver Acylrest in Amiden beispielsweise der (+)- oder (-)-Menthyloxycarbonylrest in Betracht.

Desweiteren können die erhaltenen Verbindungen der Formel I in ihre Salze, insbesondere für die pharmazeutische Anwendung in ihre physiologisch verträglichen Salze mit anorganischen oder organischen Säuren, übergeführt werden. Als Säuren kommen hierfür beispielsweise Salzsäure, Bromwasserstoffsäure, Schwefelsäure, Phosphorsäure, Fumarsäure, Bernsteinsäure, Milchsäure, Zitronensäure, Weinsäure, Maleinsäure oder Methansulfonsäure in Betracht.

Außerdem lassen sich die so erhaltenen neuen Verbindungen der Formel I, falls diese eine Carboxygruppe enthalten, gewünschtenfalls anschließend in ihre Salze mit anorganischen oder organischen Basen, insbesondere für die pharmazeutische Anwendung in ihre physiologisch verträglichen Salze, überführen. Als Basen kommen hierbei beispielsweise Natriumhydroxid, Kaliumhydroxid, Cyclohexylamin, Ethanolamin, Diethanolamin und Triethanolamin in Betracht.

Die als Ausgangsprodukte verwendeten Verbindungen der allgemeinen Formeln IV bis VII sind teilweise literaturbekannt oder man erhält diese nach literaturbekannten Verfahren oder werden in den Beispielen beschrieben.

Wie bereits eingangs erwähnt, weisen die neuen Verbindungen der allgemeinen Formel I, in der R₄ ein Wasserstoffatom oder einen Prodrugrest darstellt, wertvolle pharmakologische Eigenschaften auf, insbesondere eine inhibierende Wirkung auf verschiedene Kinasen, vor allem auf Rezeptor-Tyrosinkinasen wie VEGFR2, PDGFRα, PDGFRβ, FGFR1, FGFR3, EGFR, HER2, IGF1R und HGFR, sowie auf Komplexe von CDK's (Cyclin Dependent Kinases) wie CDK1, CDK2, CDK3, CDK4, CDK5, CDK6, CDK7, CDK8 und CDK9 mit ihren spezifischen Cyclinen (A, B1, B2, C, D1, D2, D3, E, F, G1, G2, H, I und K) und auf virales Cyclin, auf die Proliferation kultivierter humaner Zellen, insbesondere die von Endothelzellen, z.B. bei der Angiogenese, aber auch auf die Proliferation anderer Zellen, insbesondere von Tumorzellen.

Die biologischen Eigenschaften der neuen Verbindungen wurde nach folgendem Standardverfahren wie folgt geprüft:

Humane Nabelschnur Endothelzellen (HUVEC) wurden in IMDM (Gibco BRL), supplementiert mit 10% foetalem Rinderserum (FBS) (Sigma), 50 *µ*M ß-Mercaptoeethanol (Fluka), Standardantibiotika, 15 *µ*g/ml Endothelzellwachstumsfaktor (ECGS, Collaborative Biomedical Products) und 100 *µ*g/ml Heparin (Sigma) auf Gelatine-beschichteten Kulturflaschen (0.2 % Gelatine, Sigma) bei 37°C, 5 % CO₂ in wassergesättigter Atmosphäre kultiviert.

Zur Untersuchung der inhibitorischen Aktivität der erfindungsgemäßen Verbindungen wurden die Zellen für 16 Stunden "gehungert", d.h. in Kulturmedium.ohne Wachstumsfaktoren (ECGS + Heparin) gehalten. Die Zellen wurden mittels Trypsin/EDTA von den Kulturflaschen abgelöst und einmal in serumhaltigem Medium gewaschen. Anschließend wurden 2,5 x 10³ Zellen pro well ausgesät.

Die Proliferation der Zellen wurde mit 5 ng/ml VEGF₁₆₅ (vascular endothelial growth factor; H. Weich, GBF Braunschweig) und 10 *µ*g/ml Heparin stimuliert. Pro Platte wurden jeweils 6 wells als Kontrollwert nicht stimuliert.

Die erfindungsgemäßen Verbindungen wurden in 100 % Dimethylsulfoxid gelöst und in verschiedenen Verdünnungen als Dreifachbestimmungen den Kulturen zugefügt, wobei die maximale Dimethylsulfoxid-Konzentration 0.3 % betrug.

Die Zellen wurden für 76 Stunden bei 37°C inkubiert, dann wurde für weitere 16 Stunden ³H-Thymidin (0.1 µ Ci/well, Amersham) zugegeben, um die DNA Synthese zu bestimmen. Anschließend wurden die radioaktiv markierten Zellen auf Filtermatten immobilisiert und die eingebaute Radioaktivität in einem ßcounter bestimmt. Zur Bestimmung der inhibitorischen Aktivität der erfindungsgemäßen Verbindungen wurde der Mittelwert der nicht-stimulierten Zellen vom Mittelwert der Faktor-stimulierten Zellen (in Anwesenheit oder Abwesenheit der erfindungsgemäßen Verbindungen) subtrahiert.

Die relative Zellproliferation wurde in Prozent der Kontrolle (HUVEC ohne Inhibitor) berechnet und die Wirkstoffkonzentration, die die Proliferation der Zellen zu 50 % hemmt (IC₅₀), abgeleitet.

Die nachfolgende Tabelle enthält die gefundenen Ergebnisse:

| Verbindung (Beispiel Nr.) | Ic₅₀ [*µ*M] |
|---|---|
| 1 | 0.03 |
| 1(3) | 0.27 |
| 1(6) | 0.02 |
| 1(8) | 0.05 |
| 1(17) | 0.04 |
| 1(18) | 0.04 |
| 3 | 0.05 |
| 4 | 0.04 |

Außerdem wurde die Verbindung des Beispiels 1(6) an Mäusen auf ihre Verträglichkeit geprüft. Die approximative perorale LD₅₀ für diese Verbindung liegt bei über 1.000 mg/kg. Die Verbindungen der allgemeinen Formel I sind daher gut verträglich.

Auf Grund ihrer Hemmwirkung auf die Proliferation von Zellen, insbesondere von Endothelzellen und von Tumorzellen, eignen sich die Verbindungen der allgemeinen Formel I zur Behandlung von Krankheiten, in denen die Proliferation von Zellen, insbesondere die von Endothelzellen, eine Rolle spielt.

So stellt beispielsweise die Proliferation von Endothelzellen und die damit verbundene Neovaskularisierung einen entscheidenden Schritt bei der Tumorprogression dar (Folkman J. et al., Nature 339, 58-61, (1989); Hanahan D. und Folkman J., Cell 86, 353-365, (1996)). Weiterhin ist die Proliferation von Endothelzellen auch bei Hämangiomen, bei der Metastasierung, der rheumatischen Arthritis, der Psoriasis und der okularen Neovaskularisierung von Bedeutung (Folkman J., Nature Med. 1, 27-31, (1995)). Der therapeutische Nutzen von Inhibitoren der Endothelzellproliferation wurde im Tiermodell beispielsweise von O'Reilly et al. und Parangi et al. gezeigt (O'Reilly M.S. et al., Cell 88, '277-285, (1997) ; Parangi S. et al. , Proc Natl Acad Sci USA 93, 2002-2007, (1996)).

Die Verbindungen der allgemeinen Formel I, deren Tautomeren, deren Stereoisomere oder deren physiologisch verträglichen Salze eignen sich somit beispielsweise zur Behandlung von soliden Tumoren, der diabetischen Retinopathie, der rheumatischen Arthritis und der Psioriasis, oder anderen Erkrankungen, bei denen Zellproliferation oder Angiogenese eine Rolle spielen.

Auf Grund ihrer biologischen Eigenschaften können die erfindungsgemäßen Verbindungen allein oder in Kombination mit anderen pharmakologisch wirksamen Verbindungen angewendet werden, beispielsweise in der Tumortherapie in Monotherapie oder in Kombination mit anderen Anti-Tumor Therapeutika, beispielsweise in Kombination mit Topoisomerase-Inhibitoren (z.B. Etoposide), Mitoseinhibitoren (z.B. Vinblastin, Taxol), mit Nukleinsäuren interagierenden Verbindungen (z.B. cis-Platin, Cyclophosphamid, Adriamycin), Hormon-Antagonisten (z.B. Tamoxifen), Inhibitoren metabolischer Prozesse (z.B. 5-FU etc.), Zytokinen (z.B. Interferonen), Kinase-Inhibitoren, Antikörpern, oder auch in Kombination mit Strahlentherapie etc. Diese Kombinationen können entweder simultan oder sequentiell verabreicht werden.

Bei der pharmazeutischen Anwendung werden die erfindungsgemäßen Verbindungen in der Regel bei warmblütigen Wirbeltieren, insbesondere beim Menschen, in Dosierungen von 0,01-100 mg/kg Körpergewicht, vorzugsweise bei 0,1-15 mg/kg verwendet. Zur Verabreichung werden diese mit einem oder mehreren üblichen inerten Trägerstoffen und/oder Verdünnungsmitteln, z.B. mit Maisstärke, Milchzucker, Rohrzucker, mikrokristalliner Zellulose, Magnesiumstearat, Polyvinylpyrrolidon, Zitronensäure, Weinsäure, Wasser, Wasser/Äthanol, Wasser/Glycerin, Wasser/-Sorbit, Wasser/Polyäthylenglykol, Propylenglykol, Stearylalkohol, carboxymethylcellulose oder fetthaltigen Substanze wie Hartfett oder deren geeigneten Gemischen in übliche galenische Zubereitungen wie Tabletten, Dragées, Kapseln, Pulver, Injektionslösungen, Ampullen, Suspensionen, Lösungen, Sprays oder Zäpfchen eingearbeitet.

Die nachfolgenden Beispiele sollen die vorliegende Erfindung näher erläutern ohne diese zu beschränken:

### Abkürzungen:

- TBTU =: O-(Benzotriazol-1-yl)-N,N,N',N'-bis(tetramethylen)-uroniumhexafluorophosphat
- HOBt =: 1-Hydroxy-1H-benzotriazol

### Herstellung der Ausgangsverbindungen:

### Beispiel I

### 1-Hydroroxy-6-nitro-2-indolinon

2.0 g 2,4-Dinitrophenylessigsäure (hergestellt nach J. Chem. Soc. 1948, 1717) werden in 40 ml Ethanol und 6.0 ml konz. Salzsäure gelöst und 4.1 g Zinn-(II)-chlorid-dihydrat bei Raumtemperatur portionsweise zugegeben. Das Gemisch wird 12 Stunden bei Raumtemperatur und 4 Stunden bei 60°C gerührt. Nach dem Abkühlen werden weitere 2.0 g Zinn-(II)-chlorid-dihydrat zugegeben und nochmals 12 Stunden bei Raumtemperatur gerührt. Das Reaktionsgemisch wird mit Wasser verdünnt, mit Methylenchlorid extrahiert und die organische Phase über Natriumsulfat getrocknet. Nach Abziehen des Lösungsmittels wird der Rückstand mit Ether verrieben und der Niederschlag abfiltriert.
Ausbeute: 0.6 g (35 % der Theorie),
R_{f}-Wert: 0.7 (Kieselgel, Methylenchlorid/Methanol = 9:1)
C₈H₆N₂O₄
ESI-Massenspektrum: m/z = 193 [M-H⁻]

### Beispiel II

### 3,4-Diethoxy-phenylessigsäure

0.8 g 3,4-Dihydroxy-phenylessigsäure und 6.9 g Bariumhydroxid-Oktahydrat werden in 50 ml Wasser gelöst und bei Raumtemperatur 2.9 ml Diethylsulfat zugetropft. Die Lösung wird 2 Stunden bei Raumtemperatur und 2 Stunden bei 40°C gerührt. Nach dieser Zeit wird mit gesättigter Kaliumhydrogensulfat-Lösung angesäuert, mit Essigester versetzt und die Suspension über Celite filtriert. Die Phasen werden getrennt und die Essigesterphase über Natriumsulfat getrocknet und eingedampft. Der erhaltene Rückstand wird über eine Kieselgelsäule mit Toluol/Essigester/Ethanol (4:2:1) als Laufmittel gereinigt.
Ausbeute: 0.5 g (42 % der Theorie),
R_{f}-Wert: 0.5 (Kieselgel, Toluol/Essigester/Ethanol = 4:2:1)
C₁₂H₁₆O₄
ESI-Massenspektrum: m/z = 223 [M-H⁻]

### Beispiel III

### 4, 5-Diethoxy-2-nitro-phenylessigsäure

0.5.g 3,4-Diethoxy-phenylessigsäure werden in 10 ml Essigsäure p.a. vorgelegt und 0.5 ml 65%ige Salpetersäure zugetropft, so dass die Innentemperatur nicht über 15°C ansteigt. Anschliessend wird für 0.5 Stunden auf 40°C erwärmt. Nach dieser Zeit wird die Lösung auf Eiswasser gegossen und der ausgefallene Niederschlag abgesaugt. Das Produkt wird mit Wasser gewaschen und bei 100°C getrocknet.
Ausbeute: 0.3 g (57 % der Theorie),
R_{f}-Wert: 0.5 (Kieselgel, Methylenchlorid/Methanol = 10:1)
C₁₂H₁₅NO₆
ESI-Massenspektrum: m/z = 292 [M+Na⁺]

### Beispiel IV

### 2-Nitro-4-trifluormethyl-phenylessigsäure-tert, butylester

2.6 g Kalium-tert.butylat werden in 45 ml Dimethylformamid vorgelegt und bei einer Innentemperatur von -5°C 1.3 ml 3-Nitro-trifluortoluol und 1.5. ml Chloressigsäure-tert.-butylester in 1 ml Dimethylformamid zugetropft. Es wird für weitere 5 Minuten gerührt und die Lösung dann auf Eiswasser gegossen und der ausgefallene Niederschlag abgesaugt. Ausbeute: 2.5 g (82 % der Theorie),
R_{f}-Wert: 0.4 (Kieselgel, Petrolether/Essigester = 10:1)
Schmelzpunkt: 45°C

### Beispiel V

### 2-Nitro-4-trifluormethyl-phenylessigsäure

16.7 g 2-Nitro-4-trifluormethyl-phenylessigsäure-tert.butylester und 50 ml Trifluoressigsäure werden in 50 ml Methylenchlorid gelöst und 3 Stunden bei Raumtemperatur gerührt. Nach Abziehen des Lösungsmittels wird der Rückstand in Petrolether/Ether (10:1) aufgenommen, abgesaugt und im Vakuum bei 80°C getrocknet.
Ausbeute: 13.4 g (98 % der Theorie),
R_{f}-Wert: 0.5 (Kieselgel, Toluol/Essigester/Ethanol = 4:2:1)
C₉H₆F₃NO₄
ESI-Massenspektrum: m/z = 248 [M-H⁻]

### Beispiel VI

### 5,6-Diethoxy-2-indolinon

1.4 g 4,5-Diethoxy-2-nitro-phenylessigsäure werden in 50 ml Essigsäure gelöst, 0.3 g 10%iges Palladium auf Kohlenstoff zugegeben und der Ansatz 1 Stunde bei Raumtemperatur und 50 psi hydriert. Der Katalysator wird abfiltriert und das Filtrat eingedampft.
Ausbeute: 1.15 g (100 % der Theorie),
R_{f}-Wert: 0.5 (Kieselgel, Toluol/Essigester/Ethanol =4:2:1)

Analog Beispiel VI werden folgende Verbindungen hergestellt:
(1) 6-Trifluormethyl-2-indolinon
   Hergestellt aus 2-Nitro-4-trifluormethyl-phenylessigsäure
(2) 6-Brom-2-indolinon
   Hergestellt aus 4-Brom-6-nitro-phenylessigsäure (nach Chem. Pharm. Bull. (1985), 33, 1414-1418)

### Beispiel VII

### 6-Phenyl-2-indolinon

2.4 g 6-Brom-2-indolinon werden in 60 ml Dimethoxyethan vorgelegt, 1.9 g Phenylboronsäure in 8 ml Ethanol und 0.3 g Tetrakistriphenylphosphinpalladium zugegeben und zu dieser Mischung 12 ml 2N Natriumcarbonat-Kösung bei Raumtemperatur zugetropft. Der Ansatz wird 6 Stunden bei 85°C gerührt. Nach dem Abkühlen wird der Katalysator abfiltriert, das Lösungsmittel abgezogen und der Rückstand mit 100 ml Wasser und 20 ml 1N Natronlauge gewaschen. Der Rückstand wird über eine Kieselgelsäule mit Petrolether/Essigester (8:2) als Laufmittel gereinigt.
Ausbeute: 1.5 g (65 % der Theorie),
R_{f}-Wert: 0.45 (Kieselgel, Petrolether/Essigester = 1:1)
Schmelzpunkt: 167-170°C

Analog Beispiel VII wird folgende Verbindung hergestellt:
(1)6-(2-Tolyl)-2-indolinon
   Hergestellt aus 6-Brom-2-indolinon und 2-Tolylboronsäure

### Beispiel VIII

### 1-Acetyl-5,6-dimethoxy-2-indolinon

16.0 g 5,6-Dimethoxy-2-indolinon (nach Hahn; Tulus; Chem. Ber. 74, 500 (1941)) werden in.170 ml Acetanhydrid gelöst und über 3 Stunden bei 130°C gerührt. Nach dem Abkühlen wird der Rückstand abfiltriert, mit Ether gewaschen und bei 100°C im Vakuum getrocknet.
Ausbeute: 15.8 g (81 % der Theorie),
R_{f}-Wert: 0.8 (Kieselgel, Methylenchlorid/Methanol = 10:1)
C₁₂H₁₃NO₄
ESI-Massenspektrum: m/z = 234 [M-H⁻]

Analog Beispiel VIII werden folgende Verbindungen hergestellt:
(1) 1-Acetyl-6-methoxy-2-indolinon
   Hergestellt aus 6-Methoxy-2-indolinon (hergestellt nach Quallich, G. J.; Morrissey, P. M.; Synthesis 1993, 51) und Acetanhydrid
(2) 1-Acetyl-5,6-diethoxy-2-indolinon
   Hergestellt aus 5,6-Diethoxy-2-indolinon und Acetanhydrid
(3) 1-Acetyl-6-trifluormethyl-2-indolinon
   Hergestellt aus 6-Trifluormethyl-2-indolinon und Acetanhydrid

### Beispiel IX

### 1-Acetyl-3-(1-ethoxy-1-phenylmethyliden)-5,6-dimethoxy-2-indolinon

10.0 g 1-Acetyl-5,6-dimethoxy-2-indolinon, 29.2 ml Orthobenzoesäuretriethylester und 100 ml Acetanhydrid werden 48 Stunden bei 120°C gerührt. Das Lösungsmittel wird abgezogen und der Rückstand mit Toluol abgedampft, mit Ether versetzt und der ausgefallene Niederschlag (Ausgangsverbindung) abgesaugt. Das Filtrat wird eingedampft und über eine Kieseigelsäule mit Toluol, dann mit Toluol/Essigester (10:1), getrennt. Das Produkt wird mit Ether verrieben, abgesaugt und bei 100°C im Vakuum getrocknet.
Ausbeute: 1.4 g (9 % der Theorie),
R_{f}-Wert: 0.5 (Kieselgel, Toluol/Essigester = 5:1)
C₂₁H₂₁NO₅
ESI-Massenspektrum: m/z = 390 [M+Na⁺]

Analog Beispiel IX werden folgende Verbindungen hergestellt:
(1) 1-Acetyl-3-(1-ethoxy-1-phenyl-methyliden)-6-methoxy-2-indolinon
   Hergestellt aus 1-Acetyl-6-methoxy-2-indolinan, Orthobenzoesäuretriethylester und Acetanhydrid
(2) 1-Acetyl-3-(1-ethoxy-1-ethyl-methyliden)-5,6-dimethoxy-2-indolinon
   Hergestellt aus 1-Acetyl-5,6-dimethoxy-2-indolinon, Orthopropionsäuretriethylester und Acetanhydrid
(3) 1-Acetyl-3-(1-ethoxy-1-phenyl-methyliden)-5,6-diethoxy-2-indolinon
   Hergestellt aus 1-Acetyl-5,6-diethoxy-2-indolinon, Orthopropionsäuretriethylester und Acetanhydrid
(4) 1-Acetyl-3-(1-ethoxy-1-phenyl-methyliden)-6-phenyl-2-indolinon
   Hergestellt aus 6-Phenyl-2-indolinon, Orthopropionsäuretriethylester und Acetanhydrid
(5) 1-Acetyl-3-(1-ethoxy-1-phenyl-methyliden)-6-(2-tolyl)-2-indolinon
   Hergestellt aus 6-(2-Tolyl)-2-indolinon, Orthopropionsäuretriethylester und Acetanhydrid

### Beispiel X

### 1-Acetyl-3-[1-hydroxy-1-(3-cyanophenyl)-methyliden]-5,6-dimethoxy-2-indolinon

2.0 g 1-Acetyl-5,6-dimethoxy-2-indolinon, 1.3 g 2-Cyanobenzoesäure, 3.3 g TBTU, 1.6 g HOBt und 7.5 ml N-Ethyl-diiso-propylamin werden in 30 ml Dimethylformamid gelöst und zwei Tage bei Raumtemperatur gerührt. Nach dieser Zeit wird die Lösung mit 400 ml Wasser und 20 ml gesättigter Kaliumhydrogensulfat-Lösung versetzt. Der ausgefallene Niederschlag wird abgesaugt, mit Wasser, wenig Methanol und wenig Ether nachgewaschen und bei 100°C im Vakuum getrocknet.
Ausbeute: 2.6 g (84 % der Theorie),
R_{f}-Wert: 0.8 (Kieselgel, Methylenchlorid/Methanol = 5:1)
C₂₀H₁₆N₂O₅
ESI-Massenspektrum: m/z = 363 [M-H⁻]

Analog Beispiel X wird folgende verbindung hergestellt:
(1) 1-Acetyl-3-(1-hydroxy-1-phenyl-methyliden)-6-trifluormethyl-2-indolinon
   Hergestellt aus 1-Acetyl-6-trifluormethyl-2-indolinon und Benzoesäure

### Beispiel XI

### 1-Acetyl-3-[1-chlor-1-(3-cyanophenyl)-methyliden]-5,6-dimethoxy-2-indolinon

2.5 g 1-Acetyl-3-[1-chlor-1-(3-cyanophenyl)-methyliden]-5,6-dimethoxy-2-indolinon und 1.6 g Phosphorpentachlorid werden in 30 ml Toluol gelöst und 1 Stunde bei 80°C gerührt. Die Suspension wird abgekühlt, mit 50 ml Ether versetzt und der ausgefallene Niederschlag abgesaugt. Nach Waschen mit Ether wird der Rückstand bei 50°C im Vakuum getrocknet. Ausbeute: 1.5 g (56 % der Theorie),
R_{f}-Wert: 0.6 (Kieselgel, Methylenchlorid/Methanol = 40:1)
C₂₀H₁₅ClN₂O₄
ESI-Massenspektrum: m/z.= 405/407 [M+Na⁺]

Analog Beispiel XI wird folgende Verbindung hergestellt:
(1) 1-Acetyl-3-(1-chlor-1-phenyl-methyliden)-6-trifluormethyl-2-indolinon
   Hergestellt aus 1-Acetyl-3-(1-hydroxy-1-phenyl-methyliden)-6-trifluormethyl-2-indolin und Phosphorpentachlorid

### Beispiel XII

### 3-(1-Methoxy-1-phenyl-methyliden)-5,6-methylendioxy-2-indolinon

Unter Stickstoffatmosphäre werden 4.5 g Diethyl-1-methoxybenzylphosphonat (nach Burkhouse, D.; Zimmer, H.; Synthesis 1984, 330) in 40 ml absolutem Dimethylformamid gelöst und bei -40°C 4.0 g Kalium-tert-butylat portionsweise zugegeben und 15 Minuten bei -10°C nachgerührt. Zur klaren Lösung werden 3.0 g 5,6-Methylendioxyisatin (nach Lackey, K.; Sternbach, D. D.; Synthesis 1993, 993) zugegeben und 1 Stunde bei Raumtemperatur nachgerührt. Nach dieser Zeit wird die Mischung in 20 ml eiskalte gesättigte Kaliumhydrogensulfat-Lösung gegossen und mit Essigester extrahiert. Die Essigesterphase wird mit Wasser gewaschen, über Natriumsulfat getrocknet und eingedampft. Schließlich wird der Rückstand über eine Kieselgelsäule mit Toluol/Essigester (5:1) getrennt. Das erhaltene Produkt kann aus Ether umkristallisiert werden.
Ausbeute: 1.6 g (35 % der Theorie),
R_{f}-Wert: 0.3 (Kieselgel, Toluol/Essigester = 5:1)
C₁₇H₁₃NO₄
ESI-Massenspektrum: m/z = 318 [M+Na⁺]

### Beispeil XIII

### 1-Acetoxy-3-(1-ethoxy-1-phenylmethyliden)-6-nitro-2-indolinon

300 mg 1-Hydroxy-6-nitro-2-indolinon werden in 4.0 ml Acetanhydrid und 4.0 ml Orthobenzoesäuretriethylester gelöst, 6 Stunden bei 110°C gerührt, eingedampft und über eine Kieselgelsäule mit Methylenchlorid als Laufmittel gereinigt. Ausbeute: 0..28 g (49 % der Theorie),
R_{f}-Wert: 0.23 (Kieselgel, Methylenchlorid)
C₁₉H₁₆N₂O₆
Massenspektrum: m/z = 368 [M⁺]

### Beispiel XIV

### 3-(Z)-{1-[4-(Piperidin-1-yl-methyl)-anilino]-1-phenyl-methyliden}-6-nitro-2-indolinon

1.25 g 1-Acetoxy-3-(1-ethoxy-1-phenyl-methyliden)-6-nitro-2-indolinon und 0.65 g 4-(Piperidin-1-yl-methyl)-anilin werden in 10 ml Dimethylformamid gelöst und 30 Minuten bei Raumtemperatur gerührt. Nach dieser Zeit werden 46 mg Palladium auf Kohlenstoff (10%ig) zugegeben und über 1 Stunde bei Raumtemperatur mit 3 bar Wasserstoff vorsichtig hydriert. Der Katalysator wird abfiltriert, das Filtrat eingedampft und der Rückstand über eine Kieselgelsäule mit Methylenchlorid/Methanol (9:1) gereinigt.
Ausbeute: 16 mg (4 % der Theorie),
R_{f}-Wert: 0.4 (Kieselgel, Methylenchlorid/Methanol = 9:1)
C₂₇H₂₆N₄O₃.

Analog Beispiel XIV wird folgende Verbindung hergestellt:
(1) 1-Acetoxy-3-(Z)-[1-(4-methoxycarbonyl-anilino)-1-phenylmethyliden]-6-nitro-2-indolinon
   Hergestellt aus 1-Acetoxy-3-(1-ethoxy-1-phenyl-methyliden) - 6-nitro-2-indolinon und 4-Aminobenzoesäuremethylester ohne anschließende Hydrierung ESI-Massenspektrum: m/z = 472 [M-H⁻]

### Beispiel XV

### N-(2-Dimethylamino-ethyl)-N-methylsulfonyl-4-nitroanilin

38.9 g N-Methylsulfonyl-4-nitroanilin werden in 2.0 1 Aceton gelöst, 51.9 g 1-Chlor-2-dimethylamino-ethan, 77.4 g Kaliumcarbonat und 5.0 g Natriumiodid zugesetzt und das Gemisch insgesamt 4 Tage bei 50°C gerührt, wobei nach 12 Stunden weitere 25.9 g 1-Chlor-2-dimethylamino-ethan, 49.8 g Kaliumcarbonat und 5.0 g Natriumiodid in 500 ml Aceton und nach 36 Stunden weitere 26.0 g 1-Chlor-2-dimethylamino-ethan, 50.0 g Kaliumcarbonat und 5.0 g Natriumiodid in 100 ml Aceton zugesetzt werden. Nach dieser Zeit wird der Ansatz filtriert und das Filtrat eingeengt. Der Rückstand wird mit Ether verrührt, abgesaugt und bei 40°C getrocknet.
Ausbeute: 25.3 g (49 % der Theorie),
R_{f}-Wert: 0.5 (Kieselgel, Methylenchlorid/Methanol/Ammoniak = 9:1:0.1)
C₁₁H₁₇N₃O₄S
ESI-Massenspektrum: m/z = 288 [M+H⁺]

Analog Beispiel XV wird folgende Verbindung hergestellt:
(1) N-Carboxymethyl-N-methylsulfonyl-4-nitroanilin

### Beispiel XVI

### N-(Dimethylaminocarbonyl-methyl)-N-methylsulfonyl-4-nitroanilin

7.0 g N-Carboxymethyl-N-methylsulfonyl-4-nitroanilin, 2.5 g Dimethylaminhydrochlorid, 8.1 g TBTU und 3.9 g HOBT werden in 125 ml Dimethylformamid gelöst und bei 0°C 17.6 ml N-Ethyl-diisopropylamin zugegeben. Das Gemisch wird 4 Stunden bei Raumtemperatur gerührt, mit 1 l Wasser verdünnt und der ausgefallene Niederschlag abgesaugt. Nach Waschen mit Wasser, Ethanol und Ether wird der Rückstand bei 70°C im Vakuum getrocknet.
Ausbeute: 5.3 g (69 % der Theorie),
R_{f}-Wert: 0.40 (Kieselgel, Methylenchlorid/Methanol = 9:1)
C₁₁H₁₅N₃O₅S
ESI-Massenspektrum: m/z = 300 [M-H⁻]

Analog Beispiel XVI wird folgende Verbindung hergestellt:
(1) N-[(2-Dimethylamino-ethylamino)-carbonylmethyl]-N-methylsulfonyl-4-nitroanilin

### Beispiel XVII

### N-(Dimethylaminomethylcarbonyl)-N-methyl-4-nitro-anilin

1.8 g Dimethylaminhydrochlorid und 5.5 g Kaliumcarbonat werden in 80 ml Aceton vorgelegt und 4.2 g N-(2-Brommethylcarbonyl)-N-methyl-4-nitroanilin (hergestellt nach Chem. Ber. 119, 2430 (1986)) in drei Portionen bei Raumtemperatur zugegeben. Das Gemisch wird für 12 Stunden bei Raumtemperatur gerührt. Nach dieser Zeit wird das Gemisch filtriert und das Filtrat eingeengt. Der Rückstand wird in Essigester gelöst, zweimal mit Wasser gewaschen, über Natriumsulfat getrocknet und schließlich einrotiert.
Ausbeute: 2.8 g (79% der Theorie),
R_{f}-Wert: 0.5 (Kieselgel, Essigester/Methanol = 7:3)
Schmelzpunkt: 121-122°C

### Beispiel XVIII

### 4-(Piperidin-1-yl-methyyl)-nitrobenzol

40.0 g 4-Nitrobenzylbromid werden in 500 ml Methylenchlorid gelöst, 51.5 ml Triethylamin zugegeben und 18.3 ml Piperidin vorsichtig zugetropft. Nach Ende der exothermen Reaktion wird für weitere 30 Minuten unter Rückfluß erhitzt. Nach dem Abkühlen wird mit Wasser gewaschen und die organische Phase über Natriumsulfat getrocknet. Schließlich wird die organische Phase eingeengt.
Ausbeute: 36.3 g (89 % der Theorie),
R_{f}-Wert: 0.6 (Kieselgel, Methylenchlorid/Methanol = 9:1)
C₁₂H₁₆N₂O₂
Massenspektrum: m/z = 221 [M⁺]

Analog Beispiel XVIII werden folgende Verbindungen hergestellt:'
(1) 4-[(N-Benzyl-N-methyl-amino)-methyl]-nitrobenzol
(2) 3-(Dimethylaminomethyl)-nitrobenzol
(3) 4-(Dimethylaminomethyl)-nitrobenzol
(4) 4-(2-Dimethylamino-ethyl)-nitrobenzol
(5) 4-[2-(4-Ethoxycarbonyl-piperidin-1-yl)-ethyl]-nitrobenzol
(6) 4-[(2,6-Dimethyl-piperidin-1-yl)-methyl]-nitrobenzol
(7) 4-(Pyrrolidin-1-yl-methyl)-nitrobenzol

### Beispiel IXX

### 4-(4-Methyl-piperazin-1yl)-nitrobenzol

31.5 g 4-Chlor-1-nitrobenzol und 44.4 ml 1-Methyl-piperazin werden zusammengegeben und 18 Stunden bei 90°C gerührt. Anschließend wird die Lösung auf Eiswasser gegossen und der ausgefallene Niederschlag abgesaugt, mit Wasser gewaschen und aus Ethanol/Wasser (1:1) umkristallisiert. Der Rückstand wird im Vakuum bei 75°C getrocknet.
Ausbeute: 44.0 g (99 % der Theorie),
R_{f}-Wert: 0.5 (Kieselgel, Methylenchlorid/Methanol = 10:1)
Schmelzpunkt: 108-112°C.

Analog Beispiel IX werden folgende Verbindungen hergestellt:
(1) N-(2-Dimethylaminoethyl)-N-methyl-4-nitroanilin
   Hergestellt aus 1-Fluor-4-nitrobenzol und 1-Dimethylamino-2-methylamino-ethan
(2) N-(3-Dimethylaminopropyl)-N-methyl-4-nitroanilin
   Hergestellt aus 1-Fluor-4-nitrobenzol und 1-Dimethylamino-3-methylamino-propan

### Beispiel XX

### 4-[N-Acetyl-(2-dimethylaminoethy)-amino]-nitrobenzol

3.6 g 4-(2-Dimethylamino-ethylamino)-nitrobenzol (nach Gabbay et al., J. Am. Chem. Soc. 91, 5136 (1969)) werden in 50 ml Methylenchlorid gelöst und 5.0 ml Triethylamin zugegeben. Zu dieser Mischung werden langsam 1.3 ml Acetylchlorid bei Raumtemperatur zugetropft und das Gemisch 2 Stunden bei Raumtemperatur gerührt. Nach dieser Zeit werden nochmals 5.0 ml Triethylamin und 1.3 ml Acetylchlorid zugegeben und weitere 2 Stunden am Rückfluß gekocht. Das Lösungsmittel wird im Vakuum entfernt, der Rückstand in Essigester aufgenommen und die organische Phase zweimal mit Wasser ausgeschüttelt. Nach Trocknen über Magnesiumsulfat wird das Lösungsmittel abgezogen und der Rückstand im Vakuum getrocknet.
Ausbeute: 2.0 g (45 % der Theorie),
R_{f}-Wert: 0.55 (Kieselgel, Methylenchlorid/Methanol/Ammoniak = 9:1:0.1)
C₁₂H₁₇N₃O₃
ESI-Massenspektrum: m/z = 252 [M+H⁺]

Analog Beispiel XX werden folgende Verbindungen hergestellt:
(1) 4-[N-Acetyl-N-(3-dimethylaminopropyl)-amino]-nitrobenzol
   Hergestellt aus 4-(3-Dimethylamino-propylamino)-nitrobenzol und Acetylchlorid
(2) 4-[N-Propionyl-N-(2-dimethylaminoethyl)-amino]-nitrobenzol Hergestellt aus 4-(2-Dimethylamino-ethylamino)-nitrobenzol und Propionylchlorid
(3) 4-[N-Propionyl-N-(3-dimethylaminopropyl)-amino]-nitrobenzol
   Hergestellt aus 4-(3-Dimethylamino-propylamino)-nitrobenzol und Propionylchlorid

### Beispiel XXI

### 4-Nitro-N,N-dimethylbenzamid

10.0 g 4-Nitrobenzoesäure, 8.4 g Dimethylaminhydrochlorid und 18.3 g TBTU werden in 50 ml Dimethylformamid vorgelegt, 78.4 ml N-Ethyl-diisopropylamin bei Raumtemperatur zugegeben und die Mischung 15 Stunden bei Raumtemperatur gerührt. Nach dieser Zeit wird der Ansatz mit Wasser versetzt und mit Essigsäureethylester extrahiert. Die organische Phase wird nochmals mit Wasser und mit 1N Salzsäure ausgeschüttelt, über Natriumsulfat getrocknet und eingeengt. Das Produkt wird aus Ether umkristallisiert.
Ausbeute: 5.6 g (48 % der Theorie),
R_{f}-Wert: 0.6 (Kieselgel, Methylenchlorid/Methanol = 9:1)
Schmelzpunkt: 61-63°C

Analog Beispiel XXI wird folgende Verbindung hergestellt:
(1) 4-(Piperidin-1-yl-carbonyl)-nitrobenzol
   Hergestellt aus 4-Nitrobenzoesäure und Piperidin

### Beispiel XXII

### 4-(Piperidin-1-yl-methyl)-anilin

37.0 g 4-(Piperidin-1-yl-methyl)-nitrobenzol werden in 300 ml Methanol gelöst, 8.0 g Raney-Nickel zugegeben und für 1 Stunde 25 Minuten mit 3 bar Wasserstoff bei Raumtemperatur hydriert. Der Katalysator wird abfiltriert und das Filtrat eingedampft. Ausbeute: 24.0 g (75 % der Theorie),
R_{f}-wert: 0.4 (Kieselgel, Methylenchlorid/Methanol = 9:1)
C₁₂H₁₈N₂
ESI-Massenspektrum: m/z = 191 [M+H⁺]

Analog Beispiel XXII werden folgende Verbindungen hergestellt:
(1) 4-[(N-Benzyl-N-methyl-amino)-methyl]-anilin
(2) N-(2-Dimethylamino-ethyl)-N-methylsulfonyl-p-phenylendiamin
(3) 3-(Dimethylaminomethyl)-anilin
(4) 4-(Dimethylaminomethyl)-anilin
(5) 4-(2-Dimethylamino-ethyl)-anilin
(6) N-(Dimethylaminomethylcarbonyl)-N-methyl-p-phenylendiamin
(7) 4- [2-(4-Ethoxycarbonyl-piperidin-1,yl) -ethyl]-anilin
(8) N-(Dimethylaminocarbonylmethyl)-N-methylsulfonyl-p-phenylendiamin
(9) 4-[(2,6-Dimethyl-piperidin-1-yl)-methyl]-anilin
(10) N- [(2-Dimethylamino-ethylamino) -carbonylmethyl] -N-methylsul-fonyl-p-phenylendiamin
(11) 4-(Pyrrolidin-1-yl-methyl)-anilin
(12) 4-(4-Methyl-piperazin-1-yl)-anilin
(13) N-(2-Dimethylaminoethyl)-N-methyl-p-phenylendiamin
(14) N-(3-Dimethylaminopropyl)-N-methyl-p-phenylendiamin
(15) N-Acetyl-N- (2-dimethylaminoethyl)-2-phenylendiamin
(16) N-Acetyl-N-(3-dimethylaminopropyl)-p-phenylendiamin
(17) N-Propionyl-N-(2-dimethylaminoethyl)-p-phenylendiamin
(18) N-Propionyl-N-(3-dimethylaminopropyl)-p-phenylendiamin
(19) N-Methylsulfonyl-p-phenylendiamin
(20) 4-Amino-N,N-dimethylbenzamid
(21) 4-(Piperidin-1-yl-carbonyl)-anilin
(22) 4-Tetrazol-5-yl-anilin

Herstellung der Endverbindungen:

### Beispiel 1

### 3-(Z)-{1-[4-(Piperidin-1-yl-methyl)-anilino]-1-phenyl-methyliden}-5,6-dimethoxy-2-indolinon

700 mg 1-Acetyl-3-(1-ethoxy-1-phenyl-methyliden)-5,6-di-methoxy-2-indolinon und 300 mg 4-(Piperidin-1-yl-methyl)-anilin werden in 3.0 ml Dimethylformamid suspendiert und 2 Stunden bei 110°C gerührt. Nach dem Abkühlen wird 1.0 ml Piperidin zugegeben und 3 Stunden bei Raumtemperatur gerührt, mit Wasser versetzt und der ausgefallene Niederschlag abgesaugt. Der Niederschlag wird über eine Kieselgelsäule mit Methylenchlorid/Methanol/Ammoniak (10:1:0.01) getrennt, mit Ether verrührt, abgesaugt und bei 100°C getrocknet.
Ausbeute: 300 mg (55 % der Theorie),
R_{f}-Wert: 0.4 (Kieselgel, Methylenchlorid/Methanol = 5:1)
C₂₉H₂₉N₃O₄
Massenspektrum: m/z = 469 [M⁺]

Analog Beispiel 1 werden folgende Verbindungen hergestellt:
(1) 3-(Z)-[1-(4-Methoxy-anilino)-1-phenyl-methyliden]-5,6-dimethoxy-2-indolinon
   Hergestellt aus 1-Acetyl-3-(2-ethoxy-1-phenyl-methyliden)-5,6-dimethoxy-2-indolinon und p-Anisidin
   R_{f}-Wert: 0.4 (Kieselgel, Methylenchlorid/Methanol = 10:1)
   C₂₄H₂₂N₂O₄
   Massenspektrum: m/z = 402 [M⁺]
(2) 3- (Z)- [1-(4-Chlor-anilino)-1-phenyl-methyliden]-5,6-dimethoxy-2-indolinon
   Hergestellt aus 1-Acetyl-3-(1-ethoxy-1-phenyl-methyliden)-5,6-dimethoxy-2-indolinon und 4-Chloranilin
   R_{f}-Wert: 0.5 (Kieselgel, Methylenchlorid/Methanol = 10:1)
   C₂₃H₁₉ClN₂O₃
   Massenspektrum: m/z = 406/408 [M⁺]
(3) 3-(Z)-(1-{4-[(N-Benzyl-N-methyl-amino)-methyl]-anilino}-1-phenyl-methyliden)-5,6-dimethoxy-2-indolinon
   Hergestellt aus 1-Acetyl-3-(1-ethoxy-1-phenyl-methyliden)-5,6-dimethoxy-2-indolinon und 4-[(N-Benzyl-N-methyl-amino)-methyl]-anilin
   R_{f}-Wert: 0.5 (Kieselgel, Methylenchlorid/Methanol = 10:1)
   C₃₂H₃₁N₃O₃
   Massenspektrum: m/z = 505 [M⁺]
(4) 3- (Z)- (1={4- [N- (2-Dimethylaminoethyl) -N-methylsulfonylamino]-anilino}-1-phenyl-methyliden)-5,6-dimethoxy-2-indolinon
   Hergestellt aus 1-Acetyl-3-(1-ethoxy-1-phenyl-methyliden)-5,6-dimethoxy-2-indolinon und N-(2-Dimethylamino-ethyl)-N-methylsulfonyl-p-phenylendiamin
   R_{f}-Wert: 0.5 (Aluminiumoxid, Methylenchlorid/Methanol = 20:1)
   C₂₈H₃₂N₄O₅S
   Massenspektrum: m/z = 536 [M⁺]
(5) 3-(Z)-{1- [3-(Dimethylamino-methyl)-anilino]-1-phenylmethyliden}-5,6-dimethoxy-2-indolinon
   Hergestellt aus 1-Acetyl-3-(1-ethoxy-1-phenyl-methyliden)-5,6-dimethoxy-2-indolinon und 3-(Dimethylaminomethyl)-anilin R_{f}-Wert: 0.4 (Kieselgel, Methylenchlorid/Methanol = 5:1)
   C₂₆H₂₇N₃O₃
   Massenspektrum: m/z = 429 [M⁺]
(6) 3-(Z)-{1-(4-(Dimethylaminomethyl)-anilino]-1-phenylmethyliden}-5,6-dimethoxy-2-indolinon
   Hergestellt aus 1-Acetyl-3-(1-ethoxy-1-phenyl-methyliden)-5,6-dimethoxy-2-indolinon und 4-(Dimethylaminomethyl)-anilin
   R_{f}-Wert: 0.5 (Kieselgel, Methylenchlorid/Methanol/Ammoniak = 10:1:1)
   C₂₆H₂₇N₃O₃
   Massenspektrum: m/z = 429 [M⁺]
(7) 3-(Z)-{1-[4-(2-Dimethylamino-ethyl)-anilino]-1-phenylmethyliden}-5,6-dimethoxy-2-indolinon
   Hergestellt aus 1-Acetyl-3-(1-ethoxy-1-phenyl-methyliden) - 5,6-dimethoxy-2-indolinon und 4-(2-Dimethylamino-ethyl)-anilin R_{f}-Wert: 0.6 (Kieselgel, Methylenchlorid/Methanol/Ammoniak = 5:1:0:01)
   C₂₇H₂₉N₃O₃
   Massenspektrum: m/z = 443 [M⁺]
(8) 3-(Z)-{1-[4-{N-Dimethylaminomethylcarbonyl-N-methylamino)-anilino]-1-phenyl-methyliden}-5,6-dimethoxy-2-indolinon Hergestellt aus 1-Acetyl-3-(1-ethoxy-1-phenyl-methyliden)-5,6-dimethoxy-2-indolinon und N-(Dimethylaminomethylcarbonyl)-N-methyl-p-phenylendiamin
   R_{f}-Wert: 0.5 (Kieselgel, Methylenchlorid/Methanol = 10:1)
   C₂₈H₃₀N₄O₄
   Massenspektrum: m/z = 486 [M⁺]
(9) 3-(Z)-{1-[4-(1H-Imidazol-4-yl)-anilino]-1-phenylmethyliden}-5,6-dimethoxy-2-indolinon
   Hergestellt aus 1-Acetyl-3-(1-ethoxy-1-phenyl-methyliden)-5,6-dimethoxy-2-indolinon und 4-(1H-Imidazo-4-yl)-anilin (hergestellt nach Chem. Pharm. Bull. 38, 1803 (1990))
   R_{f}-Wert: 0.5 (Kieselgel, Methylenchlorid/Methanol = 10:1)
   C₂₆F₂₂N₄O₃
   ESI-Massenspektrum: m/z = 439 [M+H⁺]
(10) 3-(Z)-(1-{4-[2-(4-Carboxyethyl-piperidin-1-yl)-ethyl]-anilino}-1-phenyl-methyliden)-5,6-dimethoxy-2-indolinon
   Hergestellt aus 1-Acetyl-3-(1-ethoxy-1-phenyl-methyliden)-5,6-dimethoxy-2-indolinon und 4-[2-(4-Ethoxycarbonyl-piperidin-1-yl)-ethyl]-anilin
   R_{f}-Wert: 0.5 (Kieselgel, Methylenchlorid/Methanol = 10:1)
   C₃₃H₃₇N₃O₅
   ESI-Massenspektrum: m/z = 554 [M-H⁻]
(11) 3-(Z)-(1-{4-[N-(Dimethylaminocarbonylmethyl)-N-methylsulfonyl-amino]-anilino}-1-phenyl-methyliden)-5',6-dimethoxy-2-indolinon
   Hergestellt aus 1-Acetyl-3-(1-ethoxy-1-phenyl-methyliden)-5,6-dimethoxy-2-indolinon und N-(Dimethylaminocarbonylmethyl)-N-methylsulfonyl-p-phenylendiamin
   R_{f}-Wert: 0.5 (Kieselgel, Methylenchlorid/Methanol = 10:1)
   C₂₈H₃₀N₄O₆S
   Massenspektrum: m/z = 550 [M⁺]
(12) 3-(Z)-[1-(4-Ethoxycarbonyl-anilino)-1-phenyl-methyliden]-5,6-dimethoxy-2-indolinon
   Hergestellt aus 1-Acetyl-3-(1-ethoxy-1-phenyl-methyliden)-5,6-dimethoxy-2-indolinon und 4-Aminobenzoesäureethylester
   R_{f}-Wert: 0.5 (Kieselgel, Methylenchlorid/Ethanol = 20:1)
   C₂₆H₂₄N₂O₅
   Massenspektrum: m/z = 444 [M⁺]
(13) 3-(Z)-(1-{4-[(2,6-Dimethyl-piperidin-1-yl)-methyl]-anilino}-1-phenyl-methyliden)-5,6-dimethoxy-2-indolinon
   Hergestellt aus 1-Acetyl-3-(1-ethoxy-1-phenyl-methyliden)-5,6-dimethoxy-2-indolinon und 4-[(2,6-Dimethyl-piperidin-1-yl)-methyl]-anilin
   R_{f}-Wert: 0.5 (Kieselgel, Methylenchlorid/Methanol/Ammoniak = 10:1:0.01)
   C₃₁H₃₅N₃O₃
   Massenspektrum: m/z = 497 [M⁺]
(14) 3-(Z)-[1-(4-{N-[(2-Dimethylamino-ethylamino)-carbonylmethyl]-N-methylsulfonyl-amino}-anilino)-1-phenyl-methyliden]-5,6-dimethoxy-2-indolinon
   Hergestellt aus 1-Acetyl-3-(1-ethoxy-1-phenyl-methyliden)-5,6-dimethoxy-2-indolinon und N-[(2-Dimethylamino-ethylamino)-carbonylmethyl]-N-methylsulfonyl-p-phenylendiamin
   R_{f}-Wert: 0.3 (Kieselgel, Methylenchlorid/Methanol = 4:1)
   C₃₀H₃₅N₅O₆S
   Massenspektrum: m/z = 593 [M⁺]
(15) 3-(Z)-{1-[4-(Pyrrolidin-1-yl-methyl)-anilino]-1-phenylmethyliden}-5,6-dimethoxy-2-indolinon
   Hergestellt aus 1-Acetyl-3-(1-ethoxy-1-phenyl-methyliden) - 5,6-dimethoxy-2-indolinon und 4-(Pyrrolidin-1-yl-methyl)-anilin
   R_{f}-Wert: 0.4 (Kieselgel, Methylenchlorid/Methanol = 5:1)
   C₂₈H₂₉N₃O₃.
   Massenspektrum: m/z = 455 [M⁺]
(16) 3-(Z)-{1-[4-(4-Methyl-piperazin-1-yl)-anilino]-1-phenylmethyliden}-5,6-dimethoxy-2-indolinon
   Hergestellt aus 1-Acetyl-3-(1-ethoxy-1-phenyl-methyliden)-5,6-dimethoxy-2-indolinon und 4-(4-Methyl-piperazin-1-yl)-anilin
   R_{f}-Wert: 0.4 (Kieselgel, Methylenchlorid/Methanol = 10:1)
   C₂₈H₃₀N₄O₃
   Massenspektrum: m/z = 470 [M⁺]
(17) 3-(Z)-(1-{4-[N-Acetyl-N-(2-dimethylamino-ethyl)-amino]-anilino}-1-phenyl-methyliden)-5,6-dimethoxy-2-indolinon
   Hergestellt aus 1-Acetyl-3-(1-ethoxy-1-phenyl-methyliden)-5,6-dimethoxy-2-indolinon und N-Acetyl-N-(2-dimethylaminoethyl)-p-phenylendiamin
   R_{f}-Wert: 0.5 (Kieselgel, Methylenchlorid/Methanol = 9:1)
   C₂₉H₃₂N₄O₄
   ESI-Massenspektrum: m/z = 499 [M-H⁻]
(18) 3-(Z)-(1-{4-[N-Acetyl-N-(3-dimethylamino-propyl)-amino]-anilino}-1-phenyl-methyliden)-5,6-dimethoxy-2-indolinon
   Hergestellt aus 1-Acetyl-3-(1-ethoxy-1-phenyl-methyliden)-5,6-dimethoxy-2-indolinon und N-Acetyl-N-(3-dimethylaminopropyl)-p-phenylendiamin
   R_{f}-Wert: 0.45 (Kieselgel, Methylenchlorid/Methanol = 10:1)
   C₃₀H₃₄N₄O₄
   ESI-Massenspektrum: m/z = 515 [M+H⁺]
(19) 3-(Z)-(1-{4-[N-Propionyl-N-(2-dimethylamino-ethyl)-amino]-anilino}-1-phenyl-methyliden)-5,6-dimethoxy-2-indolinon
   Hergestellt aus 1-Acetyl-3-(1-ethoxy-1-phenyl-methyliden)-5,6-dimethoxy-2-indolinon und N-Propionyl-N-(2-dimethylaminoethyl)-p-phenylendiamin
   R_{f}-Wert: 0.5 (Kieselgel, Methylenchlorid/Methanol = 9:1)
   C₃₀H₃₄N₄O₄
   ESI-Massenspektrum: m/z = 515 [M+H⁺]
(20) 3-(Z)-(1-{4-[N-Propionyl-N-(3-dimethylamino-propyl)-amino]-anilino}-1-phenyl-methyliden)-5,6-dimethoxy-2-indolinon
   Hergestellt aus 1-Acetyl-3-(1-ethoxy-1-phenyl-methyliden)-5,6-dimethoxy-2-indolinon und N-Propionyl-N-(3-dimethylaminopropyl)-p-phenylendiamin
   R_{f}-Wert: 0.5 (Kieselgel, Methylenchlorid/Methanol = 10:1)
   C₃₁H₃₆N₄O₄
   ESI-Massenspektrum: m/z = 529 [M+H⁺]
(21) 3-(Z)-[1-(4-Aminocarbonyl-anilino)-1-phenyl-methyliden]-5,6-dimethoxy-2-indolinon
   Hergestellt aus 1-Acetyl-3-d-ethoxy-1-phenyl-methyliden)-5,6-dimethoxy-2-indolinon und 4-Aminobenzamid
   R_{f}-Wert: 0.4 (Kieselgel, Toluol/Essigester/Ethanol = 4:2:1)
   C₂₄H₂₁N₃O₄
   ESI-Massenspektrum: m/z = 414 [M-H⁻]
(22) 3-(Z)-[1-(4-Dimethylaminocarbonyl-anilino)-1-phenylmethyliden]-5,6-dimethoxy-2-indolinon
   Hergestellt aus 1-Acetyl-3-(1-ethoxy-1-phenyl-methyliden)-5,6-dimethoxy-2-indolinon und 4-Amino-dimethylbenzamid
   R_{f}-Wert: 0.4 (Kieselgel, Toluol/Essigester/Ethanol = 4:2:1)
   C₂₆H₂₅N₃O₄
   ESI-Massenspektrum: m/z = 442 [M-H⁻]
(23) 3-(Z)-{1-[4-(Piperidin-1-yl-carbonyl)-anilino]-1-phenylmethyliden}-5,6-dimethoxy-2-indolinon
   Hergestellt aus 1-Acetyl-3-(1-ethoxy-1-phenyl-methyliden)-5,6-dimethoxy-2-indolinon und 4-(Piperidin-1-yl-carbonyl)-anilin
   R_{f}-Wert: 0.4 (Kieselgel, Toluol/Essigester/Ethanol = 4:2:1)
   C₂₉H₂₉N₃O₄
   ESI-Massenspektrum: m/z = 482 [M-H⁻]
(24) 3- (Z) - [1-(4-Ethoxycarbonylmethyl-anilino)-1-phenyl - methyliden]-5,6-dimethoxy-2-indolinon
   Hergestellt aus 1-Acetyl-3-(1-ethoxy-1-phenyl-methyliden)-5,6-dimethoxy-2-indolinon und und 4-Aminophenyl-essigsäure-ethylester
   R_{f}-Wert: 0.5 (Kieselgel, Petrolether/Essigester/Ethanol = 7:2:1)
   C₂₂₇H₂₆N₂O₅
   ESI-Massenspektrum: m/z = 457 [M-H⁻]
(25) 3-(Z)-{1-[4-(Tetrazol-5-yl)-anilino]-1-phenyl-methyliden}-5,6-dimethoxy-2-indolinon
   Hergestellt aus 1-Acetyl-3-(1-ethoxy-1-phenyl-methyliden)-5,6-dimethoxy-2-indolinon und 4-Tetrazol-5-yl-anilin
   R_{f}-Wert: 0.4 (Kieselgel, Toluol/Essigester/Ethanol = 4:2:1)
   C₂₄H₂₀N₆O₃
   ESI-Massenspektrum: m/z = 439 [M-H⁻]
(26) 3-(Z)-{1-[4-(Piperidin-1-yl-methyl)-anilino]-1-phenyl-methyliden}-5,6-methylendioxy-2-indolinon
   Hergestellt aus 3-(1-Methoxy-1-phenyl-methyliden)-5,6-methylendioxy-2-indolinon und 4-(Piperidin-1-yl-methyl)-anilin
   R_{f}-Wert: 0.5 (Kieselgel, Methylenchlorid/Methanol = 5:1)
   C₂₈H₂₇N₃O₃
   Massenspektrum: m/z = 453 [M⁺]
(27) 3-(Z)-{1-[4-(Dimethylaminomethyl)-anilino]-1-phenyl-methyliden}-5,6-methylendioxy-2-indolinon
   Hergestellt aus 3-(1-Methoxy-1-phenyl-methyliden)-5,6-methylendioxy-2-indolinon und 4-(Dimethylaminomethyl)-anilin R_{f}-Wert: 0.5 (Kieselgel, Methylenchlorid/Methanol = 5:1)
   C₂₅H₂₃N₃O₃
   Massenspektrum: m/z = 413 [M⁺]
(28) 3-(Z)-(1-{4-[(N-Benzyl-N-methyl-amino)-methyl]-anilino}-1-phenyl-methyliden}-5,6-methylendioxy-2-indolinon
   Hergestellt.aus 3-(1-Methoxy-1-phenyl-methyliden)-5,6-methylendioxy-2-indolinon und 4-[(N-Benzyl-N-methyl-amino)-methyl]-anilin
   R_{f}-Wert: 0.5 (Kieselgel, Methylenchlorid/Methanol = 5:1)
   C₃₁H₂₇N₃O₃
   Massenspektrum: m/z = 489 [M⁺]
(29) 3-(Z)-(1-{4-[N-(2-Dimethylaminoethyl)-N-methylsulfonylamino]-anilino}-1-phenyl-methyliden)-5,6-methylendioxy-2-indolinon
   Hergestellt aus 3-(1-Methoxy-1-phenyl-methyliden)-5,6-methylendioxy-2-indolinon und N-(2-Dimethylamino-ethyl)-N-methylsulfonyl-p-phenylendiamin
   R_{f}-Wert: 0.6 (Kieselgel, Methylenchlorid/Methanol = 5:1)
   C₂₇H₂₈N₄O₅S
   Massenspektrum: m/z = 520 [M⁺]
(30) 3-(Z)-{1-[4-(N-Dimethylaminomethylcarbonyl-N-methylamino)-anilino]-1-phenyl-methyliden}-5,6-methylendioxy-2-indolinon
   Hergestellt aus 3-(1-Methoxy-1-phenyl-methyliden)-5,6-methylendioxy-2-indolinon und N-(Dimethylaminomethylcarbonyl)-N-methyl-p-phenylendiamin
   R_{f}-Wert: 0.3 (Kieselgel, Methylenchlorid/Methanol = 5:1)
   C₂₇H₂₆N₄O₄
   Massenspektrum: m/z = 470 [M⁺]
(31) 3-(Z)-{1-[4-(Piperidin-1-yl-methyl)-anilino]-1-phenylmethyliden}-6-methoxy-2-indolinon
   Hergestellt aus 1-Acetyl-3-(1-ethoxy-1-phenyl-methyliden)-6-methoxy-2-indol.inon und 4-(Piperidin-1-yl-methyl)-anilin R_{f}-Wert: 0.4 (Aluminiumoxid, Toluol/Essigester/Methanol = 4:2:0.25)
   C₂₈H₂₉N₃O₂
   Massenspektrum: m/z = 439 [M⁺]
(32) 3- (Z) - (1-{4- [(N-Benzyl-N-methyl-amino)-methyl] -anilino}-1-phenyl-methyliden)-6-methoxy-2-indolinon
   Hergestellt aus 1-Acetyl-3-(1-ethoxy-1-phenyl-methyliden)-6-methoxy-2-indolinon und 4-[(N-Benzyl-N-methyl-amino)-methyl]-anilin
   R_{f}-Wert: 0.5 (Aluminiumoxid, Toluol/Essigester/Ethanol = 4 :2:0.25)
   C₃₁H₂₉N₃O₂
   Massenspektrum: m/z = 475 [M⁺]
(33) 3-(Z)-{1- [3-(Dimethylaminomethyl)-anilino]-1-phenyl-methyliden}-6-methoxy-2-indolinon
   Hergestellt aus 1-Acetyl-3-(1-ethoxy-1-phenyl-methyliden)-6-methoxy-2-indolinon und 3-(Dimethylaminomethyl)-anilin
   R_{f}-Wert: 0.5 (Kieselgel, Methylenchlorid/Methanol = 5:1)
   C₂₅H₂₅N₃O₂
   Massenspektrum: m/z = 399 [M⁺]
(34) 3-(Z)-{1-[4-(Piperidin-1-yl-methyl)-anilino]-1-phenyl-methyliden}-5,6-diethoxy-2-indolinon
   Hergestellt aus 1-Acetyl-3-(1-ethoxy-1-phenyl-methyliden)-5,6-diethoxy-2-indolinon und 4-(Piperidin-1-yl-methyl)-anilin R_{f}-Wert: 0.4 (Aluminiumoxid, Toluol/Essigester = 2:1)
   C₃₁H₃₅N₃O₃
   ESI-Massenspektrum: m/z = 498 [M+H⁺]
(35) 3-(Z)-{1-[4-(Piperidin-1-yl-methyl)-anilino]-1-phenyl-methyliden}-6-trifluormethyl-2-indolinon
   Hergestellt aus 1-Acetyl-3-(1-chlor-1-phenyl-methyliden)-6-trifluormethyl-2-indolinon und 4-(Piperidin-1-yl-methyl)-anilin
   R_{f}-Wert: 0.5 (Kieselgel, Methylenchlorid/Methanol = 10:1)
   C₂₈H₂₆F₃N₃O
   ESI-Massenspektrum: m/z = 476 [M-H⁻]
(36) 3-(Z)-(1-{4-[N-(2-Dimethylamino-ethyl)-N-methylsulfonyl-amino]-anilino}-1-phenyl-methyliden)-6-trifluormethyl-2-indolinon
   Hergestellt aus 1-Acetyl-3-(1-chlor-1-phenyl-methyliden)-6-trifluormethyl-2-indolinon und N-(2-Dimethylamino-ethyl)-N-methylsulfonyl-p-phenylendiamin
   R_{f}-Wert: 0.5 (Kieselgel, Methylenchlorid/Methanol = 10:1)
   C₂₇H₂₇F₃N₄O₃S
   ESI-Massenspektrum: m/z = 543 [M-H⁻]
(37) 3-(Z)-{1-[4-(N-Dimethylaminomethylcarbonyl-N-methyl-amino)-anilino]-1-phenyl-methyliden}-6-trifluormethyl-2-indolinon
   Hergestellt aus 1-Acetyl-3-(1-chlor-1-phenyl-methyliden)-6-trifluormethyl-2-indolinon und N-(Dimethylaminomethylcarbonyl)-N-methyl-p-phenylendiamin
   R_{f}-Wert: 0.4 (Kieselgel, Methylenchlorid/Methanol = 10:1)
   C₂₇H₂₅F₃N₄O₂
   ESI-Massenspektrum: m/z = 493 [M-H⁻]
(38) 3-(Z)-{1-[4-(Piperidin-1-yl-methyl)-anilino]-1-phenyl-methyliden}-6-phenyl-2-indolinon
   Hergestellt aus 1-Acetyl-3-(1-ethoxy-1-phenyl-methyliden)-6-phenyl-2-indolinon und 4-(Piperidin-1-yl-methyl)-anilin
   R_{f}-Wert: 0.45 (Kieselgel, Methylenchlorid/Methanol = 9:1)
   C₃₃H₃₁N₃O
   ESI-Massenspektrum: m/z = 486 [M+H⁺]
(39) 3-(Z)-(1-{4-[(N-Benzyl-N-methyl-amino)-methyl]-anilino}-1-phenyl-methyliden)-6-phenyl-2-indolinon
   Hergestellt aus 1-Acetyl-3-(1-ethoxy-1-phenyl-methyliden)-6-phenyl-2-indolinon und 4-[(N-Benzyl-N-methyl-amino)-methyl]-anilin
   R_{f}-Wert: 0.45 (Kieselgel, Methylenchlorid/Methanol = 9:1)
   C₃₃H₃₁N₃O
   ESI-Massenspektrum: m/z = 486 [M+H⁺]
(40) 3-(Z)-(1-{4-[N-(2-Dimethylamino-ethyl)-N-methylsulfonyl-amino]-anilino}-1-phenyl-methyliden)-6-phenyl-2-indolinon
   Hergestellt aus 1-Acetyl-3-(1-ethoxy-1-phenyl-methyliden)-6-phenyl-2-indolinon und N-(2-Dimethylamino-ethyl)-N-methylsulfonyl-p-phenylendiamin
   R_{f}-Wert: 0.4 (Kieselgel, Methylenchlorid/Methanol = 9:1)
   C₃₂H₃₂N₄O₃S
   ESI-Massenspektrum: m/z = 553 [M+H⁺]
(41) 3- (Z)-{1-[3-(Dimethylaminomethyl)-anilino]-1-phenyl-methyliden}-6-phenyl-2-indolinon
   Hergestellt aus 1-Acetyl-3-(1-ethoxy-1-phenyl-methyliden)-6-phenyl-2-indolinon und 3-(Dimethylaminomethyl)-anilin
   R_{f}-Wert: 0.55 (Kieselgel, Methylenchlorid/Methanol = 10:1)
   C₃₀H₂₇N₃O
   ESI-Massenspektrum: m/z = 446 [M+H⁺]
(42) 3-(Z)-{1-[4-(N-Dimethylaminomethylcarbonyl-N-methyl-amino)-anilino]-1-phenyl-methyliden}-6-phenyl-2-indolinon
   Hergestellt aus 1-Acetyl-3-(1-ethoxy-1-phenyl-methyliden)-6-phenyl-2-indolinon und N-(Dimethylaminomethylcarbonyl)-N-methyl-p-phenylendiamin
   R_{f}-Wert: 0.45 (Kieselgel, Methylenchlorid/Methanol = 9:1)
   C₃₂H₃₀N₄O₂
   Massenspektrum: m/z = 502 [M⁺]
(43) 3-(Z)-{1-[4-(Piperidin-1-yl-methyl)-anilino]-1-phenyl-methyliden}-6-(2-tolyl)-2-indolinon
   Hergestellt aus 1-Acetyl-3-(1-ethoxy-1-phenyl-methyliden)-6-(2-tolyl)-2-indolinon und 4-(Piperidin-1-yl-methyl)-anilin R_{f}-Wert: 0.5 (Kieselgel, Methylenchlorid/Methanol = 9:1)
   C₃₄H₃₃N₃O
   ESI-Massenspektrum: m/z = 500 [M+H⁺]
(44) 3-(Z)-{1-[4-(Dimethylaminomethyl)-anilino]-1-phenyl-methyliden}-6-(2-tolyl)-2-indolinon
   Hergestellt aus 1-Acetyl-3-(1-ethoxy-1-phenyl-methyliden)-6-(2-tolyl)-2-indolinon und 4-(Dimethylaminomethyl)-anilin R_{f}-Wert: 0.45 (Kieselgel, Methylenchlorid/Methanol = 9:1)
   C₃₁H₂₉N₃O
   Massenspektrum: m/z = 459 [M⁺]
(45) 3-(Z)-[1-(4-Ethoxycarbonyl-anilino)-1-phenyl-methyliden)-6-(2-tolyl)-2-indolinon
   Hergestellt aus 1-Acetyl-3-(1-ethoxy-1-phenyl-methyliden)-6-(2-tolyl)-2-indolinon und 4-Aminobenzoesäureethylester
   R_{f}-Wert: 0.5 (Kieselgel, Toluol/Essigester/Ethanol = 4:2:1)
   C₃₁H₂₆N₂O₃
   Massenspektrum: m/z = 474 [M⁺]

### Beispiel 2

### 3-(Z)-(1-{4-[2-(4-Carboxy-piperidin-1-yl)-ethyl]-anilino}-1-phenyl-methyliden)-5,6-dimethoxy-2-indolinon

0.4 g 3-(Z)-(1-{4-[2-(4-Ethoxycarbonyl-piperidin-1-yl)-ethyl]-anilino}-1-phenyl-methyliden)-5,6-dimethoxy-2-indolinon werden in 8.0 ml Ethanol gelöst und 2.0 ml 1N Natronlauge zugegeben. Das Gemisch wird für 1 Stunde bei 50°C gerührt. Zur klaren Lösung werden 2.0 ml 1N HCl zugesetzt und der entstehende Niederschlag abgesaugt. Der Niederschlag wird mit Wasser und Ethanol gewaschen und im Vakuum bei 100°C getrocknet.
Ausbeute: 0.2 g (64 % der Theorie),
R_{f}-Wert: 0.2 (Kieselgel, Methylenchlorid/Methanol = 2:1)
C₃₁H₃₃N₃O₅
ESI-Massenspektrum: m/z = 526 [M-H⁻]

Analog Beispiel 2 werden folgende Verbindungen hergestellt:
(1) 3-(Z)-[1-(4-Carboxy-anilino)-1-phenyl-methyliden]-5,6-dimethoxy-2-indolinon
   Hergestellt aus 3-(Z)-[1-(4-Ethoxycarbonyl-anilino)-1-phenyl-methyliden]-5,6-dimethoxy-2-indolinon
   R_{f}-Wert: 0.4 (Kieselgel, Methylenchlorid/Methanol = 10:1)
   C₂₄H₂₀N₂O₅
   ESI-Massenspektrum: m/z = 415 [M-H⁻]
(2) 3-(Z)-{1-[4-(Carboxymethyl)-anilino]-1-phenyl-methyliden}-5,6-dimethoxy-2-indolinon
   Hergestellt aus 3-(Z)-{1-[4-(Ethoxycarbonylmethyl)-anilino]-1-phenyl-methyliden}-5,6-dimethoxy-2-indolinon
   R_{f}-Wert: 0.1 (Kieselgel, Petrolether/Essigester/Ethanol = 7:2:1)
   C₂₅H₂₂N₂O₅
   ESI-Massenspektrum: m/z = 429 [M-H⁻]
(3) 3- (Z) - [1-(4 -carboxy-anilino)-1-phenyl-methyliden] -6-ureido-2-indolinon
   Hergestellt aus 3-(Z)-[1-(4-Methoxycarbonyl-anilino)-1-phenyl-methyliden]-6-ureido-2-indolinon
   R_{f}-Wert: 0.45 (Kieselgel, Methylenchlorid/Methanol = 4:1)
   C₂₃H₁₈N₄O₄
   ESI-Massenspektrum: m/z = 413 [M-H⁻]

### Beispiel 3

### 3-(Z)-(1-{4-[2-(4-Dimethylcarbamoyl-piperidin-1-yl)-ethyl]-anilino}-1-phenyl-methyliden)-5,6-dimethoxy-2-indolinon

0.2 g 3-(Z)-(1-{4-[2-(4-Carboxy-piperidin-1-yl)-ethyl]-anilino}-1-phenyl-methyliden)-5,6-dimethoxy-2-indolinon werden in 5.0 ml Dimethylformamid gelöst und 0.2 g TBTU, 0.1 g HOBT und 0.5 ml Triethylamin zugegeben. Schließlich werden 0.2 g Dimethylaminhydrochlorid zugegeben und das Gemisch 2 Stunden bei Raumtemperatur gerührt. Das Lösungsmittel wird abgezogen und der Rückstand mit Wasser verrührt, abgesaugt und mit Isopropanol und Ether gewaschen. Der Rückstand wird im Vakuum bei 100°C getrocknet.
Ausbeute: 0.2 g (95 % der Theorie),
R_{f}-Wert: 0.5 (Kieselgel, Methylenchlorid/Methanol = 10:1)
C₃₃H₃₈N₄O₄
Massenspektrum: m/z = 554 [M⁺]

Analog Beispiel 3 werden folgende Verbindungen hergestellt:
(1) 3-(Z)-{1-[4-(Aminocarbonylmethyl)-anilino]-1-phenyl-methyliden}-5,6-dimethoxy-2-indolinon
   Hergestellt aus 3-(Z)-{1-[4-(Carboxymethyl)-anilino]-1-phenyl-methyliden}-5,6-dimethoxy-2-indolinon und N-Hydroxy-succinimid-ammoniumsalz
   R_{f}-Wert: 0.4 (Kieselgel, Petrolether/Essigester/Ethanol = 4:2:1)
   C₂₅H₂₃N₃O₄
   ESI-Massenspektrum: m/z = 428 [M-H⁻]
(2) 3-(Z)-{1-[4-(Dimethylaminocarbonylmethyl)-anilino]-1-phenyl-methyliden}-5,6-dimethoxy-2-indolinon
   Hergestellt aus 3-(Z)-{1-[4-(Carboxymethyl)-anilino]-1-phenyl-methyliden}-5,6-dimethoxy-2-indolinon und Dimethylamin-hydrochlorid
   R_{f}-Wert: 0.45 (Kieselgel, Petrolether/Essigester/Ethanol = 4:2:1)
   C₂₇H₂₇N₃O₄
   ESI-Massenspektrum: m/z = 456 [M-H⁻]

### Beispiel 4

### 6-Amino-3-(Z)-{1-[4-(piperidin-1-yl-methyl)-anilino]-1-phenyl-methyliden}-2-indolinon

11.2 g 3-(Z)-{1-[4-(Piperidin-1-yl-methyl)-anilino]-1-phenyl-methyliden}-6-nitro-2-indolinon werden in 120 ml Methanol und 60 ml Methylenchlorid gelöst, 1.1 g Palladium auf Kohlenstoff (10%ig) zugegeben und über 2 Stunden 45 Minuten bei Raumtemperatur mit 3 bar Wasserstoff hydriert. Der Katalysator wird abfiltriert, das Filtrat eingedampft und der Rückstand über eine Kieselgelsäule mit Methylenchlorid/Methanol (4:1) gereinigt.
Ausbeute: 5.2 g (52 % der Theorie),
R_{f}-wert: 0.25 (Kieselgel, Methylenchlorid/Methanol = 4:1)
C₂₇H₂₈N₄O
ESI-Massenspektrum: m/z = 425 [M+H⁺]

Analog Beispiel 4 wird folgende Verbindung hergestellt:
(1) 6-Amino-3-(Z)-[1-(4-methoxycarbonyl-anilino)-1-phenyl-methyliden]-2-indolinon
   Hergestellt aus 1-Acetoxy-3-(Z)-[1-(4-methoxycarbonyl-anilino)-1-phenyl-methyliden]-6-nitro-2-indolinon
   R_{f}-Wert: 0.74 (Kieselgel, Methylenchlorid/Methanol = 4:1)
   C₂₃H₁₉N₃O₃
   ESI-Massenspektrum: m/z = 384 [M-H⁻]

### Beispiel 5

### 3-(Z) - [1- (4-Methoxycarbonyl-anilino)-1-phenyl-methyliden]-6-ureido-2-indolinon

560 mg 6-Amino-3-(Z)-[1-(4-methoxycarbonyl-anilino)-1-phenyl-methyliden]-2-indolinon, 236 mg Kaliumcyanat und 3.8 ml Eisessig werden in 20 ml Ethanol gelöst und für 1 Stunde am Rückfluß gekocht. Nach dieser Zeit wird das Lösungsmittel abgedampft, der Rückstand in Methylenchlorid aufgenommen, mit Wasser gewaschen und über Natriumsulfat getrocknet. Nach dem Eindampfen wird das Produkt im Exsikator getrocknet.
Ausbeute: 280 mg (45 % der Theorie),
R_{f}-Wert: 0.25 (Kieselgel, Essigester/Cyclohexan/Methanol = 4.5:4.5:1)
C₂₄H₂₀N₄O₄
ESI-Massenspektrum: m/z = 427 [M-H⁻]

### Beispiel 6

### 6-Benzoylamino-3-(Z)-{1-[4-(piperidin-1-yl-methyl)-anilino]-1-phenyl-methyliden}-2-indolinon

0.4 g 6-Amino-3-(Z)-{1-[4-{piperidin-1-yl-methyl)-anilino]-1-phenyl-methyliden}-2-indolinon werden in 25 ml Methylenchlorid gelöst, 12.5 ml Pyridin zugegeben und bei Raumtemperatur 0.1 ml Benzoylchlorid zugetropft. Das Reaktionsgemisch wird 12 Stunden bei Raumtemperatur gerührt, eingedampft und über eine Kieselgelsäule mit Methylenchlorid/Methanol = 4:1 als Laufmittel gereinigt.
Ausbeute: 160 mg (32 % der Theorie),
R_{f}-Wert: 0.3 (Kieselgel, Methylenchlorid/Methanol = 9:1)
C₃₄H₃₂N₄O₂
ESI-Massenspektrum: m/z = 529 [M+H⁺]

Analog Beispiel 6 werden folgende Verbindungen hergestellt:
(1) 6-Acetylamino-3-(Z)-{1-[4-(piperidin-1-yl-methyl)-anilino]-1-phenyl-methyliden}-2-indolinon
   Hergestellt aus 6-Amino-3-(Z)-{1-[4-(piperidin-1-yl-methyl)-anilino]-1-phenyl-methyliden}-2-indolinon und Acetylchlorid R_{f}-Wert: 0.48 (Kieselgel, Methylenchlorid/Methanol = 4:1)
   C₂₉H₃₀N₄O₂
   ESI-Massenspektrum: m/z = 467 [M+H⁺]
(2) 6-(2-Phenylacetylamino)-3-(Z)-{1-[4-(piperidin-1-yl-methyl)-anilino]-1-phenyl-methyliden}-2-indolinon
   Hergestellt aus 6-Amino-3-(Z)-{1-[4-(piperidin-1-yl-methyl)-anilino)-1-phenyl-methylidenl-2-indolinon und Phenylessigsäurechlorid
   R_{f}-Wert: 0.67 (Kieselgel, Methylenchlorid/Methanol = 4:1)
   C₃₅H₃₄N₄O₂
   ESI-Massenspektrum: m/z = 543 [M+H⁺]
(3) 6-Methansulfonylamino-3-(Z)-{1-[4-(piperidin-1-yl-methyl)-anilino]-1-phenyl-methyliden}-2-indolinon
   Hergestellt aus 6-Amino-3-(Z)-{1-[4-(piperidin-1-yl-methyl)-anilino]-1-phenyl-methyliden}-2-indolinon und Methansulfonylchlorid
   R_{f}-Wert: 0.56 (Kieselgel, Methylenchlorid/Methanol = 4:1)
   C₂₈H₃₀N₄O₃S
   ESI-Massenspektrum: m/z = 503 [M+H⁺]
(4) 6-Benzolsulfonylamino-3-(Z)-{1-[4-(piperidin-1-yl-methyl)-anilino]-1-phenyl-methyliden}-2-indolinon
   Hergestellt aus 6-Amino-3-(Z)-(1-[4-(piperidin-1-yl-methyl)-anilino]-1-phenyl-methyliden)-2-indolinon und Benzolsulfonylchlorid
   R_{f}-Wert: 0.53 (Kieselgel, Methylenchlorid/Methanol = 4:1)
   C₃₃H₃₂N₄O₃S
   ESI-Massenspektrum: m/z = 565 [M+H⁺]

### Beispiel 7

### 6-Methylamino-3-(Z)-{1-[4-(piperidin-1-yl-methyl)-anilino]-1-phenyl-methyliden}-2-indolinon und 6-Dimethylamino-3-(Z)-{1-[4-(piperidin-1-yl-methyl)-anilino]-1-phenyl-methyliden}-2-indolinon

0.1 ml Formaldehyd-Lösung (37%ig) werden in 20 ml Methanol gelöst, 0.5 g 6-Amino-3-(Z)-{1-[4-(piperidin-1-yl-methyl)-anilino]-1-phenyl-methyliden}-2-indolinon zugegeben und 1 Stunde bei Raumtemperatur gerührt. Es bildet sich ein Niederschlag, der durch Zugabe von 5 ml Methylenchlorid in Lösung gebracht wird. Zu dieser Mischung werden 75 mg Natriumcyanoborhydrid zugegeben und das Gemisch 12 Stunden bei Raumtemperatur gerührt. Nach dieser Zeit werden 5 ml konz. HCl zugegeben, das Lösungsmittel weitgehend abgezogen, der Rückstand in Wasser aufgenommen und mit Natronlauge basisch gestellt. Die wäßrige Phase wird mit Methylenchlorid extrahiert, über Natriumsulfat getrocknet, eingedampft und über eine Kieselgelsäule mit Methylenchlorid/Methanol (4:1) gereinigt. Ausbeute: 70 mg (13 % der Theorie). 6-Methylamino-3-(Z)-{1-[4-(piperidin-1-yl-methyl)-anilino]-1-phenyl-methyliden}-2-indolinon,
R_{f}-Wert: 0.64 (Kieselgel, Methylenchlorid/Methanol = 4:1)
C₂₈H₃₀N₄O
Massenspektrum: m/z = 438 [M⁺]

35 mg (6 % der Theorie) 6-Dimethylamino-3-(Z)-{1-[4-(piperidin-1-yl-methyl)-anilino)-1-phenyl-methyliden}-2-indolinon, R_{f}-wert: 0.58 (Kieselgel, Methylenchlorid/Methanol = 4:1)
C₂₉H₃₄N₄O
ESI-Massenspektrum: m/z = 453 [M+H⁺]

### Beispiel 8

### 3-(Z)-{1-[4-(Piperidin-1-yl-methyl)-anilino]-1-phenyl-methyliden}-6- (pyrrol-1-yl)-2-indolinon

0.5 g 6-Amino-3-(Z)-{1-[4-(piperidin-1-yl-methyl)-anilino]-1-phenyl-methyliden}-2-indolinon werden in 20 ml Eisessig gelöst und 0.2 ml 2,5-Dimethoxytetrahydrofuran zugegeben. Das Gemisch wird 1 Stunde am Rückfluß gekocht, eingedampft und der Rückstand in 20 ml Wasser aufgenommen. Nach weiteren 2 Stunden Rühren wird mit 1N Natronlauge basisch gestellt, die wäßrige Phase mit Methylenchlorid extrahiert und die organische Phase über Natriumsulfat getrocknet. Nach Abziehen des Lösungsmittels wird der Rückstand über eine Kieselgelsäule mit Methylenchlorid/Methanol (4:1) als Laufmittel gereinigt.
Ausbeute: 200 mg (36 % der Theorie),
R_{f}-wert: 0.73 (Kieselgel, Methylenchlorid/Methanol = 4:1)
C₃₁H₃₀N₄O
ESI-Massenspektrum: m/z = 475 [M+H⁺]

### Beispiel 9

### 3-(Z)-{1-[4-(Piperidin-1-yl-methyl)-anilino)-1-phenyl-methyliden}-6-(pyrrolidin-1-yl)-2-indolinon

0.3 g 6-Amino-3-(Z)-{1-[4-(piperidin-1-yl-methyl)-anilino]-1-phenyl-methyliden}-2-indolinon werden in 10 ml Dimethylformamid gelöst und 180 mg Kaliumcarbonat zugegeben. 0.1 ml 1,4-Dibrombutan (gelöst in 1.0 ml Dimethylformamid) werden bei Raumtemperatur zugetropft und das Reaktionsgemisch 12 Stunden bei 80°C gerührt. Nach dem Abkühlen wird der Ansatz auf Wasser gegossen und der entstehende Niederschlag abfiltriert. Ausbeute: 110 mg (33 % der Theorie),
R_{f}-Wert: 0.41 (Kieselgel, Methylenchlorid/Methanol = 9:1)
C₃₁H₃₄N₄O
ESI-Massenspektrum: m/z = 479 [M+H⁺]

Analog Beispiel 9 wird folgende Verbindung hergestellt:
(1) 6-Methoxycarbonylmethylamino-3-(Z)-{1-[4-(piperidin-1-yl-methyl)-anilino]-1-phenyl-methyliden}-2-indolinon
   Hergestellt aus 6-Amino-3-(Z)-{1-[4-(piperidin-1-yl-methyl)-anilino]-1-phenyl-methyliden}-2-indolinon und Bromessigsäuremethylester
   Ausbeute: 230 mg (28 % der Theorie),
   R_{f}-Wert: 0.36 (Kieselgel, Methylenchlorid/Methanol = 9:1)
   C₃₀H₃₂N₄O₃
   ESI-Massenspektrum: m/z = 497 [M+H⁺]

### Beispiel 10

### 6-Carboxymethylamino-3-(Z)-{1-[4-(piperidin-1-yl-methyl)-anilino-1-phenyl-methyliden}-2-indolinon

180 mg 6-Methoxycarbonylmethylamino-3-(Z)-{1-[4-(piperidin-1-yl-methyl)-anilino]-1-phenyl-methyliden}-2-indolinon werden in 10 ml Methanol vorgelegt, 1.0 ml 1N Natronlauge zugegeben und 1 Stunde bei 40°C gerührt. Nach dem Abkühlen wird mit 1.0 ml 1N Salzsäure neutralisiert und das Lösungsmittel abgezogen. Der Rückstand wird in Methylenchlorid und wenig Methanol gelöst und über Natriumsulfat getrocknet.
Ausbeute: 76 mg (44 % der Theorie),
R_{f}-Wert: 0.05 (Kieselgel, Methylenchlorid/Methanol = 9:1)
C₂₉H₃₀N₄O₃
ESI-Massenspektrum: m/z = 483 [M+H⁺]

Analog den vorstehenden Beispielen können folgende Verbindungen hergestellt werden:
(1) 3-(Z)-(1-Anilino-1-phenyl-methyliden)-5,6-dimethoxy-2-indolinon
(2) 3-(Z)-[1-(4-Nitro-anilino)-1-phenyl-methyliden]-5,6-dimethoxy-2-indolinon
(3) 3-(Z)-1-[(4-Fluor-anilino)-1-phenyl-methyliden]-5,6-dimethoxy-2-indolinon
(4) 3-(Z)-[1-(4-Brom-anilino)-1-phenyl-methyliden]-5,6-dimethoxy-2-indolinon
(5) 3-(Z)-[1-(4-Iod-anilino)-1-phenyl-methyliden]-5,6-dimethoxy-2-indolinon
(6) 3-(Z)-[1-(4-cyano-anilino)-1-phenyl-methyliden]-5,6-dimethoxy-2-indolinon
(7) 3-(Z)-(1-(4-Ethoxy-anilino)-1-phenyl-methyliden]-5,6-dimethoxy-2-indolinon
(8) 3-(Z)-[1-(4-Trifluormethyl-anilino)-1-phenyl-methyliden]-5,6-dimethoxy-2-indolinon
(9) 3-(Z)-[1-(4-Methyl-anilino)-1-phenyl-methyliden]-5,6-dimethoxy-2-indolinon
(10) 3-(Z)-(1-(4-Methylmercapto-anilino)-1-phenyl-methyliden]-5,6-dimethoxy-2-indolinon
(11) 3-(Z)-[1-(4-Aminomethyl-anilino)-1-phenyl-methyliden]-5,6-dimethoxy-2-indolinon
(12) 3-(Z)-{1-[4-(Methylaminomethyl)-anilino]-1-phenyl-methyliden}-5,6-dimethoxy-2-indolinon
(13) 3-(Z)-{1-[4-(Isopropylaminomethyl)-anilino]-1-phenyl-methyliden}-5,6-dimethoxy-2-indolinon
(14) 3-(Z)-{1-[4-(Phenylaminomethyl)-anilino]-1-phenyl-methyliden}-5,6-dimethoxy-2-indolinon
(15) 3-(Z)-{1-[4-(Ethylaminomethyl)-anilino]-1-phenyl-methyliden}-5,6-dimethoxy-2-indolinon
(16) 3-(Z)-{1-[4-(Propylaminomethyl)-anilino]-1-phenyl-methyliden}-5,6-dimethoxy-2-indolinon
(17) 3-(Z)-{1-[4-(Butylaminomethyl)-anilino]-1-phenyl-methyliden}-5,6-dimethoxy-2-indolinon
(18) 3-(Z)-{1-[4-(Isobutylaminomethyl)-anilino]-1-phenyl-methyliden}-5,6-dimethoxy-2-indolinon
(19) 3-(Z)-{1-[4-(Cyclohexylaminomethyl)-anilino]-1-phenyl-methyliden}-5,6-dimethoxy-2-indolinon
(20) 3-(Z)-{1-[4-(Benzylaminomethyl)-anilinol-1-phenyl-methyliden}-5,6-dimethoxy-2-indolinon
(21) 3-(Z)-(1-{4-[(N-Ethyl-N-methyl-amino)-methyl]-anilino}-1-phenyl-methyliden)-5,6-dimethoxy-2-indolinon
(22) 3-(Z)-{1-[4-(Diethylaminomethyl)-anilino]-1-phenyl-methyliden}-5,6-dimethoxy-2-indolinon
(23) 3-(Z)-(1-{4-[(N-Methyl-N-propyl-amino)-methyl]-anilino}-1-phenyl-methyliden)-5,6-dimethoxy-2-indolinon
(24) 3-(Z)-(1-{4-[(N-Isopropyl-N-methyl-amino)-methyl]-anilino}-1-phenyl-methyliden)-5,6-dimethoxy-2-indolinon
(25) 3-(Z)-(1-{4-[(N-Ethyl-N-propyl-amino)-methyl]-anilino}-1-phenyl-methyliden)-5,6-dimethoxy-2-indolinon
(26) 3-(Z)-(1-{4-[(N-Ethyl-N-isopropyl-amino)-methyl]-anilino}-1-phenyl-methyliden)-5,6-dimethoxy-2-indolinon
(27) 3-(Z)-{1-[4-(Dipropylaminomethyl)-anilino]-1-phenyl-methyliden}-5,6-dimethoxy-2-indolinon
(28) 3-(Z)-{1-[4-(Diisopropylaminomethyl)-anilino]-1-phenylmethyliden}-5,6-dimethoxy-2-indolinon
(29) 3-(Z)-(1-{4-[(N-Benzyl-N-ethyl-amino)-methyl]-anilino}-1-phenyl-methyliden)-5,6-dimethoxy-2-indolinon
(30) 3- (Z)-{1-[4-(Dibenzylaminomethyl)-anilino]-1-phenyl-methyliden}-5,6-dimethoxy-2-indolinon
(31) 3-(Z)-{1-[4-(3,6-Dihydro-2H-pyridin-1-yl-methyl)-anilino]-1-phenyl-methyliden}-5,6-dimethoxy-2-indolinon
(32) 3-(Z)-{1-[4-(3,5-Dimethyl-piperidin-1-yl-methyl)-anilino]-1-phenyl-methyliden}-5,6-dimethoxy-2-indolinon
(33) 3-(Z)-{1-[4-(Azepan-1-yl-methyl)-anilino]-1-phenyl-methyliden}-5,6-dimethoxy-2-indolinon
(34) 3-(Z)-{1-[4-(Piperazin-1-yl-methyl)-anilino]-1-phenyl-methyliden}-5,6-dimethoxy-2-indolinon
(35) 3-(Z)-{1-[4-(Morpholin-4-yl-methyl)-anilino]-1-phenyl-methyliden}-5,6-dimethoxy-2-indolinon
(36) 3-(Z)-{1-[4-(Thiomorpholin-4-yl-methyl)-anilino]-1-phenyl-methyliden}-5,6-dimethoxy-2-indolinon
(37) 3-(Z)-{1-[4-(1-Oxo-thiomorpholin-4-yl-methyl)-anilino]-1-phenyl-methyliden}-5,6-dimethoxy-2-indolinon
(38) 3-(Z)-{1-(4-(Acetylamino-methyl)-anilino]-1-phenyl-methyliden}-5,6-dimethoxy-2-indolinon
(39) 3-(Z)-{1-[4-(2-Amino-ethyl)-anilinol-1-phenyl-methyliden}-5,6-dimethoxy-2-indolinon
(40) 3-(Z)-{1-[4-(2-Methylamino-ethyl)-anilino]-1-phenyl-methyliden}-5,6-dimethoxy-2-indolinon
(41) 3-(Z)-{1-[4-(2-Ethylamino-ethyl)-anilino]-1-phenyl-methyliden}-5,6-dimethoxy-2-indolinon
(42) 3- (Z)-{1-[4-(2-Diethylamino-ethyl)-anilino]-1-phenyl-methyliden}-5,6-dimethoxy-2-indolinon
(43) 3-(Z)-{1-[4-(2-Piperidin-1-yl-ethyl)-anilino]-1-phenyl-methyliden}-5,6-dimethoxy-2-indolinon
(44) 3-(Z)-{1-[4-(2-Acetylamino-ethyl)-anilino]-1-phenyl-methyliden}-5,6-dimethoxy-2-indolinon
(45) 3- (Z)-{1-[4-(3-Amino-propyl)-anilino]-1-phenyl-methyliden}-5,6-dimethoxy-2-indolinon
(46) 3-(Z)-{1-[4-(3-Dimethylamino-propyl)-anilino]-1-phenyl-methyliden}-5,6-dimethoxy-2-indolinon
(47) 3-(Z)-{1-[4-(N-Aminomethylcarbonyl-N-methyl-amino)-anilino]-1-phenyl-methyliden}-5,6-dimethoxy-2-indolinon
(48) 3-(Z)-{1-[4-(N-Methylaminomethylcarbonyl-N-methyl-amino)-anilino]-1-phenyl-methyliden}-5,6-dimethoxy-2-indolinon.
(49) 3-(Z)-{1-[4-(N-Ethylaminomethylcarbonyl-N-methyl-amino)-anilino]-1-phenyl-methyliden}-5,6-dimethoxy-2-indolinon
(50) 3-(Z)-{1-[4-(N-Diethylaminomethylcarbonyl-N-methyl-amino)-anilino]-1-phenyl-methyliden}-5,6-dimethoxy-2-indolinon
(51) 3-(Z)-(1-{4-[N-(Piperidin-1-yl-methylcarbonyl)-N-methylamino]-anilino}-1-phenyl-methyliden)-5,6-dimethoxy-2-indolinon
(52) 3-(Z)-(1-{4-[N-(Morpholin-4-yl-methylcarbonyl)-N-methylamino]-anilino}-1-phenyl-methyliden)-5,6-dimethoxy-2-indolinon
(53) 3-(Z)-(1-{4-[N-(Piperazin-1-yl-methylcarbonyl)-N-methylamino]-anilino}-1-phenyl-methyliden)-5,6-dimethoxy-2-indolinon
(54) 3-(Z)-(1-{4-[N-(2-Amino-ethyl-carbonyl)-N-methyl-amino]-anilino}-1-phenyl-methyliden)-5,6-dimethoxy-2-indolinon
(55) 3-(Z)-(1-{4-[N-(2-Methylamino-ethyl-carbonyl)-N-methylamino]-anilino}-1-phenyl-methyliden)-5,6-dimethoxy-2-indolinon
(56) 3-(Z)-(1-{4-[N-(2-Diethylamino-ethyl-carbonyl)-N-methylamino]-anilino}-1-phenyl-methyliden)-5,6-dimethoxy-2-indolinon
(57) 3-(Z)-(1-{4-[N-Acetyl-N-(2-aminoethyl)-amino]-anilino}-1-phenyl-methyliden)-5,6-dimethoxy-2-indolinon
(58) 3- (Z) - (1-{4- [N-Acetyl-N- (2-methylamino-ethyl) -amino] -anilino}-1-phenyl-methyliden)-5,6-dimethoxy-2-indolinon
(59) 3- (Z) - (1-{4- [N-Acetyl-N- (3-amino-propyl) -amino] -anilino}-1-phenyl-methyliden)-5,6-dimethoxy-2-indolinon
(60) 3-(Z)-(1-{4-[N-Acetyl-N-(2-methylamino-propyl)-amino]-anilino}-1-phenyl-methyliden)-5,6-dimethoxy-2-indolinon
(61) 3-(Z)-(1-{4-[N-Acetyl-N-(2-piperidin-1-yl-ethyl)-amino]-anilino}-1-phenyl-methyliden)-5,6-dimethoxy-2-indolinon
(62) 3-(Z)-(1-{4-[N-Acetyl-N-(aminocarbonylmethyl)-amino]-anilino}-1-phenyl-methyliden)-5,6-dimethoxy-2-indolinon
(63) 3-(Z)-(1-{4-[N-Acetyl-N-(dimethylaminocarbonylmethyl)-amino]-anilino}-1-phenyl-methyliden)-5,6-dimethoxy-2-indolinon
(64) 3-(Z)-(1-{4-[N-Acetyl-N-(piperidin-1-yl-carbonylmethyl)-amino]-anilino}-1-phenyl-methyliden)-5,6-dimethoxy-2-indolinon
(65) 3- (Z)-(1-{4-[N-Methyl-N-(aminocarbonyl)-amino] -anilino}-1-phenyl-methyliden)-5,6-dimethoxy-2-indolinon
(66) 3-(Z)-(1-{4-[N-Methyl-N-(methylaminocarbonyl)-amino]-anilino}-1-phenyl-methyliden)-5,6-dimethoxy-2-indolinon
(67) 3-(Z)-(1-{4-[N-Methyl-N-(dimethylaminocarbonyl)-amino]-anilino}-1-phenyl-methyliden)-5,6-dimethoxy-2-indolinon
(68) 3-(Z)-(1-(4-[N-Methyl-N-(piperidin-1-yl-carbonyl)-amino] - anilino}-1-phenyl-methyliden)-5,6-dimethoxy-2-indolinon
(69) 3-(Z)-(1-{4-[N-(2-Aminoethyl)-N-methylsulfonyl-amino]-anilino}-1-phenyl-methyliden)-5,6-dimethoxy-2-indolinon
(70) 3-(Z)-(1-{4-[N-(2-Methylamino-ethyl)-N-methylsulfonyl-amino]-anilino}-1-phenyl-methyliden)-5,6-dimethoxy-2-indolinon
(71) 3-(Z)-(1-{4-[N-(2-Ethylamino-ethyl)-N-methylsulfonyl-amino]-anilino}-1-phenyl-methyliden)-5,6-dimethoxy-2-indolinon
(72) 3-(Z)-(1-{4-[N-(2-Diethylamino-ethyl)-N-methylsulfonyl-amino]-anilino}-1-phenyl-methyliden)-5,6-dimethoxy-2-indolinon
(73) 3-(Z)-(1-{4-[N-(2-Pyrrolidin-1-yl-ethyl)-N-methylsulfonyl-amino]-anilino}-1-phenyl-methyliden)-5,6-dimethoxy-2-indolinon
(74) 3-(Z)-(1-{4-[N-(2-Piperidin-1-yl-ethyl)-N-methylsulfonyl-amino]-anilino}-1-phenyl-methyliden)-5,6-dimethoxy-2-indolinon
(75) 3-(Z)-(1-{4-[N-(2-Piperazin-1-yl-ethyl)-N-methylsulfonyl-amino]-anilino}-1-phenyl-methyliden)-5,6-dimethoxy-2-indolinon
(76) 3-(Z)-[1-(4-{N-[2-(Morpholin-4-yl)-ethyl]-N-methylsulfonyl-amino}-anilino)-1-phenyl-methyliden)-5,6-dimethoxy-2-indolinon
(77) 3-(Z)-(1-{4-[N-(Aminocarbonylmethyl)-N-methylsulfonyl-amino]-anilino}-1-phenyl-methyliden)-5,6-dimethoxy-2-indolinon
(78) 3-(Z)-(1-{4-[N-(Methylaminocarbonylmethyl)-N-methylsulfonyl-amino]-anilino}-1-phenyl-methyliden)-5,6-dimethoxy-2-indolinon
(79) 3-(Z)-(1-{4-[N-(Ethylaminocarbonylmethyl)-N-methylsulfonyl-amino]-anilino}-1-phenyl-methyliden)-5,6-dimethoxy-2-indolinon
(80) 3-(Z)-[1-(4-{N-[N-(2-Dimethylamino-ethyl)-N-methyl-amino)-carbonylmethyl]-N-methylsulfonyl-amino}-anilino)-1-phenyl-methyliden]-5,6-dimethoxy-2-indolinon
(81) 3-(Z)-(1-{4-[N-(Diethylaminocarbonylmethyl)-N-methylsulfonyl-amino]-anilino}-1-phenyl-methyliden)-5,6-dimethoxy-2-indolinon
(82) 3-(Z)-(1-{4-[N-(Pyrrolidin-1-yl-carbonylmethyl)-N-methylsulfonyl-amino]-anilino}-1-phenyl-methyliden)-5,6-dimethoxy-2-indolinon
(83) 3-(Z)-(1-{4-[N-(Piperidin-1-yl-carbonylmethyl)-N-methylsulfonyl-amino]-anilino}-1-phenyl-methyliden)-5,6-dimethoxy-2-indolinon
(84) 3-(Z)-(1-{4-[N-(Piperazin-1-yl-carbonylmethyl)-N-methylsulfonyl-amino]-anilino}-1-phenyl-methyliden)-5,6-dimethoxy-2-indolinon
(85) 3-(Z)-[1-(4-{N-[(Morpholin-4-yl)-carbonylmethyl]-N-methylsulfonyl-amino}-anilino)-1-phenyl-methyliden]-5,6-dimethoxy-2-indolinon
(86) 3-(Z)-{1-[4-(2-Dimethylamino-ethoxy)-anilino]-1-phenyl-methyliden}-5,6-dimethoxy-2-indolinon
(87) 3-(Z)-{1-[4-(3-Dimethylamino-propoxy)-anilino]-1-phenyl-methyliden}-5,6-dimethoxy-2-indolinon
(88) 3-(Z)-{1-[4-(2-Aminocarbonyl-ethyl)-anilino]-1-phenyl-methyliden}-5,6-dimethoxy-2-indolinon
(89) 3-(Z)-{1-[4-(Pyridin-2-yl)-anilino]-1-phenyl-methyliden}-5,6-dimethoxy-2-indolinon
(90) 3-(Z)-{1-[4-(Pyridin-3-yl)-anilino]-1-phenyl-methyliden}-5,6-dimethoxy-2-indolinon
(91) 3-(Z)-{1-[4-(Pyridin-4-yl)-anilino]-1-phenyl-methyliden}-5,6-dimethoxy-2-indolinon
(92) 3-(Z)-(1-Anilino-1-phenyl-methyliden)-5,6-diethoxy-2-indolinon
(93) 3-(Z)-[1-(4-Nitro-anilino)-1-phenyl-methyliden]-5, 6-diethoxy-2-indolinon
(94) 3-(Z)-[1-(4-Ethoxycarbonyl-anilino)-1-phenyl-methyliden]-5,6-diethoxy-2-indolinon
(95) 3-(Z)-[1-(4-carboxy-anilino)-1-phenyl-methyliden]-5,6-diethoxy-2-indolinon
(96) 3-(Z)-1-[(4-Fluor-anilino)-1-phenyl-methyliden]-5,6-diethoxy-2-indolinon
(97) 3-(Z)-1-[(4-Chlor-anilino)-1-phenyl-methyliden]-5,6-diethoxy-2-indolinon
(98) 3-(Z)-[1-(4-Brom-anilino)-1-phenyl-methyliden]-5,6-diethoxy-2-indolinon
(99) 3-(Z)-[1-(4-Iod-anilino)-1-phenyl-methyliden]-5,6-diethoxy-2-indolinon
(100) 3-(Z)-[1-(4-Cyano-anilino)-1-phenyl-methyliden]-5,6-diethoxy-2-indolinon
(101) 3- (Z) - [1- (4-Methoxy-anilino) -1-phenyl-methyliden] - 5,6-diethoxy-2-indolinon
(102) 3-(Z)-[1-(4-Ethoxy-anilino)-1-phenyl-methyliden]-5,6-diethoxy-2-indolinon
(103) 3-(Z)-[1-(4-Trifluormethyl-anilino)-1-phenyl-methyliden]-5,6-diethoxy-2-indolinon
(104) 3-(Z)-[1-(4-Methyl-anilino)-1-phenyl-methyliden]-5,6-diethoxy-2-indolinon
(105) 3-(Z)-[1-(4-Methylmercapto-anilino)-1-phenyl-methyliden]-5,6-diethoxy-2-indolinon
(106) 3-(Z)-[1-(4-Aminomethyl-anilino)-1-phenyl-methyliden]-5,6-diethoxy-2-indolinon
(107) 3-(Z)-{1-[4-(Methylaminomethyl)-anilino]-1-phenyl-methyliden}-5,6-diethoxy-2-indolinon
(108) 3-(Z)-{1-[4-(Isopropylaminomethyl)-anilino]-1-phenyl-methyliden}-5,6-diethoxy-2-indolinon
(109) 3-(Z)-{1-[4-(Phenylaminomethyl)-anilino]-1-phenyl-methyliden}-5,6-diethoxy-2-indolinon
(110) 3-(Z)-{1-[4-(Ethylaminomethyl)-anilino]-1-phenyl-methyliden}-5,6-diethoxy-2-indolinon
(111) 3-(Z)-{1-[4-(Propylaminomethyl)-anilino]-1-phenyl-methyliden}-5,6-diethoxy-2-indolinon
(112) 3-(Z)-{1-[4-(Butylaminomethyl)-anilino]-1-phenyl-methyliden}-5,6-diethoxy-2-indolinon
(113) 3-(Z)-{1-[4-(Isobutylaminomethyl)-anilino]-1-phenyl-methyliden}-5,6-diethoxy-2-indolinon
(114) 3-(Z)-{1-[4-(Cyclohexylaminomethyl)-anilino]-1-phenyl-methyliden}-5,6-diethoxy-2-indolinon
(115) 3-(Z)-{1-[4-(Benzylaminomethyl)-anilino]-1-phenyl-methyliden}-5,6-diethoxy-2-indolinon
(116) 3-(Z)-{1-[4-(Dimethylaminomethyl)-anilino]-1-phenyl-methyliden}-5,6-diethoxy-2-indolinon
(117) 3-(Z)-{1-[3-(Dimethylaminomethyl)-anilino]-1-phenyl-methyliden}-5,6-diethoxy-2-indolinon
(118) 3-(Z)-(1-{4-[(N-Ethyl-N-methyl-amino)-methyl]-anilino}-1-phenyl-methyliden)-5,6-diethoxy-2-indolinon
(119) 3-(Z)-{1-[4-(Diethylaminomethyl)-anilino]-1-phenyl-methyliden}-5,6-diethoxy-2-indolinon
(120) 3-(Z)-(1-(4-[(N-Methyl-N-propyl-amino)-methyl]-anilino)-1-phenyl-methyliden)-5,6-diethoxy-2-indolinon
(121) 3-(Z)-(1-{4-[(N-Isopropyl-N-methyl-amino)-methyl]-anilino}-1-phenyl-methyliden)-5,6-diet.hoxy-2-indolinon
(122) 3-(Z)-(1-{4-[(N-Ethyl-N-propyl-amino)-methyl]-anilino}-1-phenyl-methyliden)-5,6-diethoxy-2-indolinon
(123) 3-(Z)-(1-{4-[(N-Ethyl-N-isopropyl-amino)-methyl]-anilino}-1-phenyl-methyliden)-5,6-diethoxy-2-indolinon
(124) 3-(Z)-{1-[4-(Dipropylaminomethyl)-anilino]-1-phenyl-methyliden}-5,6-diethoxy-2-indolinon
(125) 3-(Z)-{1-[4-(Diisopropylaminomethyl)-anilino]-1-phenyl-methyliden}-5,6-diethoxy-2-indolinon
(126) 3-(Z)-(1-{4-[(N-Benzyl-N-methyl-amino)-methyl]-anilino}-1-phenyl-methyliden)-5,6-diethoxy-2-indolinon
(127) 3-(Z)-(1-{4-[(N-Benzyl-N-ethyl-amino)-methyl]-anilino}-1-phenyl-methyliden)-5,6-diethoxy-2-indolinon
(128) 3-(Z)-{1-[4-(Dibenzylaminomethyl)-anilino]-1-phenyl-methyliden}-5,6-diethoxy-2-indolinon
(129) 3-(Z)-{1-[4-(pyrrolidin-1-yl-methyl)-anilino]-1-phenyl-methyliden}-5,6-diethoxy-2-indolinon
(130) 3-(Z)-{1-[4-(3,6-Dihydro-2H-pyridin-1-yl-methyl)-anilino]-1-phenyl-methyliden)-5,6-diethoxy-2-indolinon
(131) 3-(Z)-{1-[4-(2,6-Dimethyl-piperidin-1-yl-methyl)-anilino]-1-phenyl-methyliden}-5,6-diethoxy-2-indolinon
(132) 3-(Z)-{1-[4-(3,5-Dimethyl-piperidin-1-yl-methyl)-anilino]-1-phenyl-methyliden}-5,6-diethoxy-2-indolinon
(133) 3-(Z)-{1-[4-(Azepan-1-yl-methyl)-anilino]-1-phenyl-methyliden}-5,6-diethoxy-2-indolinon
(134) 3-(Z)-{1-[4-(Piperazin-1-yl-methyl)-anilino]-1-phenyl-methyliden}-5,6-diethoxy-2-indolinon
(135) 3-(Z)-{1-[4-(Morpholin-4-yl-methyl)-anilino]-1-phenyl-methyliden}-5,6-diethoxy-2-indolinon
(136) 3-(Z)-{1-[4-(Thiomorpholin-4-yl-methyl)-anilino]-1-phenyl-methyliden}-5,6-diethoxy-2-indolinon
(137) 3-(Z)-{1-[4-(1-Oxo-thiomorpholin-4-yl-methyl)-anilino]-1-phenyl-methyliden}-5,6-diethoxy-2-indolinon
(138) 3-(Z)-{1-[4-(Acetylamino-methyl)-anilino]-1-phenyl-methyliden}-5,6-diethoxy-2-indolinon
(139) 3-(Z)-{1-[4-(2-Amino-ethyl)-anilino]-1-phenyl-methyliden}-5,6-diethoxy-2-indolinon
(140) 3-(Z)-{1-[4-(2-Methylamino-ethyl)-anilino]-1-phenyl-methyliden}-5,6-diethoxy-2-indolinon
(141) 3-(Z)-{1-[4-(2-Ethylamino-ethyl)-anilino]-1-phenyl-methyliden)-5, 6-diethoxy-2-indolinon
(142) 3-(Z)-{1-[4-(2-Dimethylamino-ethyl)-anilino]-1-phenyl-methyliden}-5,6-diethoxy-2-indolinon
(143) 3-(Z)-{1-E4-(2-Diethylamino-ethyl)-anilino]-1-phenyl-methyliden}-5,6-di:ethoxy-2-indolinon
(144) 3-(Z)-{1-[4-(2-Piperidin-1-yl-ethyl)-anilino]-1-phenyl-methyliden}-5,6-diethoxy-2-indolinon
(145) 3-(Z)-(1-{4-[2- (4-Ethoxycarbonyl-piperidin-1-yl)-ethyl]-anilino}-1-phenyl-methyliden)-5,6-diethoxy-2-indolinon
(146) 3-(Z)-(1-{4-[2-(4-Carboxy-piperidin-1-yl)-ethyl]-anilino}-1-phenyl-methyliden)-5,6-diethoxy-2-indolinon
(147) 3-(Z)-(1-{4-[2-(4-Dimethylcarbamoyl-piperidin-1-yl)-ethyl]-anilino}-1-phenyl-methyliden)-5,6-diethoxy-2-indolinon
(148) 3-(Z)-{1-[4-(2-Acetylamino-ethyl)-anilino]-1-phenyl-methyliden}-5,6-diethoxy-2-indolinon
(149) 3-(Z)-{1-[4-(3-Amino-propyl)-anilino]-1-phenyl-methyliden}-5,6-diethoxy-2-indolinon
(150) 3-(Z)-{1-[4-(3-Dimethylamino-propyl)-anilino]-1-phenyl-methyliden}-5,6-diethoxy-2-indolinon
(151) 3-(Z)-{1-[4-(N-Aminomethylcarbonyl-N-methyl-amino)-anilino]-1-phenyl-methyliden}-5,6-diethoxy-2-indolinon
(152) 3-(Z)-{1-[4-(N-Methylaminomethylcarbonyl-N-methyl-amino)-anilino]-1-phenyl-methyliden}-5,6-diethoxy-2-indolinon
(153) 3-(Z)-{1-[4-N-Dimethylaminomethylcarbonyl-N-methyl-amino)-anilino]-1-phenyl-methyliden}-5,6-diethoxy-2-indolinon
(154) 3-(Z)-{1-[4-(N-Ethylaminomethylcarbonyl-N-methyl-amino)-anilino]-1-phenyl-methyliden}-5,6-diethoxy-2-indolinon
(155) 3-(Z)-{1-[4-(N-Diethylaminomethylcarbonyl-N-methyl-amino)-anilino]-1-phenyl-methyliden}-5,6-diethoxy-2-indolinon
(156) 3-(Z)-(1-{4-[N-(Piperidin-1-yl-methylcarbonyl)-N-methylamino]-anilino}-1-phenyl-methyliden)-5,6-diethoxy-2-indolinon
(157) 3-(Z)-(1-{4-[N-(Morpholin-4-yl-methylcarbonyl)-N-methylamino]-anilino}-1-phenyl-methyliden)-5,6-diethoxy-2-indolinon
(158) 3-(Z)-(1-{4-[N-(Piperazin-1-yl-methylcarbonyl)-N-methylamino]-anilino}-1-phenyl-methyliden)-5,6-diethoxy-2-indolinon
(159) 3-(Z)-(1-{4-[N-(2-Amino-ethyl-carbonyl)-N-methyl-amino]-anilino}-1-phenyl-methyliden)-5,6-diethoxy-2-indolinon
(160) 3-(Z)-(1-{4-[N-(2-Methylamino-ethyl-carbonyl)-N-methylamino]-anilino}-1-phenyl-methyliden)-5,6-diethoxy-2-indolinon
(161) 3-(Z)-(1-{4-[N-(2-Diethylamino-ethyl-carbonyl)-N-methylamino]-anilino}-1-phenyl-methyliden)-5,6-diethoxy-2-indolinon
(162) 3-(Z)-(1-{4-[N-Acetyl-N-(2-aminoethyl)-amino]-anilino}-1-phenyl-methyliden)-5,6-diethoxy-2-indolinon
(163) 3-(Z)-(1-{4-[N-Acetyl-N-(2-methylamino-ethyl)-amino]-anilino}-1-phenyl-methyliden)-5,6-diethoxy-2-indolinon
(164) 3-(Z)-(1-{4-[N-Acetyl-N-(2-dimethylaminoethyl)-amino]-anilino}-1-phenyl-methyliden)-5,6-diethoxy-2-indolinon
(165) 3-(Z)-(1-{4-[N-Acetyl-N-(3-amino-propyl)-amino]-anilino}-1-phenyl-methyliden)-5,6-diethoxy-2-indolinon
(166) 3-(Z)-(1-{4-[N-Acetyl-N-(3-dimethylamino-propyl)-amino]-anilino}-1-phenyl-methyliden)-5,6-diethoxy-2-indolinon
(167) 3-(Z)-(1-{4-[N-Acetyl-N-(2-methylamino-propyl)-amino]-anilino}-1-phenyl-methyliden)-5,6-diethoxy-2-indolinon
(168) 3-(Z)-(1-{4-[N-Acetyl-N-(2-piperidin-1-yl-ethyl)-amino]-anilino}-1-phenyl-methyliden)-5,6-diethoxy-2-indolinon
(169) 3-(Z)-(1-{4-[N-Acetyl-N-(aminocarbonylmethyl)-amino]-anilino)-1-phenyl-methyliden)-5,6-diethoxy-2-indolinon
(170) 3-(Z)-(1-{4-[N-Acetyl-N-(dimethylaminocarbonylmethyl)-aminol-anilino}-1-phenyl-methyliden)-5,6-diethoxy-2-indolinon
(171) 3-(Z)-(1-{4-[N-Acetyl-N-(piperidin-1-yl-carbonylmethyl)-amino]-anilino}-1-phenyl-methyliden)-5,6-diethoxy-2-indolinon
(172) 3-(Z)-(1-{4-[N-Methyl-N-(aminocarbonyl)-amino]-anilino}-1-phenyl-methyliden)-5,6-diethoxy-2-indolinon
(173) 3-(Z)-(1-{4-[N-Methyl-N-(methylaminocarbonyl)-amino]-anilino}-1-phenyl-methyliden)-5,6-diethoxy-2-indolinon
(174) 3-(Z)-(1-{4-[N-Methyl-N-(dimethylaminocarbonyl)-amino]-anilino}-1-phenyl-methyliden)-5,6-diethoxy-2-indolinon
(175) 3-(Z)-(1-{4-[N-Methyl-N-(piperidin-1-yl-carbonyl)-amino]-anilino}-1-phenyl-methyliden)-5,6-diethoxy-2-indolinon
(176) 3-(Z)-(1-{4-[N-(2-Aminoethyl)-N-methylsulfonyl-amino]-anilino}-1-phenyl-methyliden)-5,6-diethoxy-2-indolinon
(177) 3-(Z)-(1-{4-[N-(2-Methylamino-ethyl)-N-methylsulfonyl-amino]-anilino}-1-phenyl-methyliden)-5,6-diethoxy-2-indolinon
(178) 3-(Z)-(1-{4-[N-(2-Ethylamino-ethyl)-N-methylsulfonyl-amino]-anilino}-1-phenyl-methyliden)-5,6-diethoxy-2-indolinon
(179) 3-(Z)-(1-{4-[N-(2-Dimethylamino-ethyl)-N-methylsulfonyl-amino]-anilino}-1-phenyl-methyliden)-5,6-diethoxy-2-indolinon
(180) 3-(Z)-(1-{4-[N-(2-Diethylamino-ethyl)-N-methylsulfonyl-amino]-anilino}-1-phenyl-methyliden)-5,6-diethoxy-2-indolinon
(181) 3-(Z)-(1-{4-[N-(2-Pyrrolidin-1-yl-ethyl)-N-methylsulfonyl-amino] -anilino}-1-phenyl-methyliden) -5,6-diethoxy-2-indolinon
(182) 3-(Z)-(1-{4-[N-(2-Piperidin-1-yl-ethyl)-N-methylsulfonyl-amino]-anilino}-1-phenyl-methyliden)-5,6-diethoxy-2-indolinon
(183) 3-(Z)-(1-{4-[N-(2-Piperazin-1-yl-ethyl)-N-methylsulfonyl-amino]-anilino}-1-phenyl-methyliden)-5,6-diethoxy-2-indolinon
(184) 3-(Z)-[1-(4-{N-[2-(Morpholin-4-yl)-ethyl]-N-methylsulfonyl-amino}-anilino)-1-phenyl-methyliden]-5,6-diethoxy-2-indolinon
(185) 3-(Z)-(1-{4-[N-(Aminocarbonylmethyl)-N-methylsulfonyl-amino]-anilino}-1-phenyl-methyliden)-5,6-diethoxy-2-indolinon
(186) 3-(Z)-(1-{4-[N-(Methylaminocarbonylmethyl)-N-methylsulfonyl-amino]-anilino}-1-phenyl-methyliden)-5,6-diethoxy-2-indolinon
(187) 3-(Z)-(1-{4-[N-(Ethylaminocarbonylmethyl)-N-methylsulfonyl-amino]-anilino}-1-phenyl-methyliden)-5,6-diethoxy-2-indolinon
(188) 3-(Z)-[1-(4-{N-1(2-Dimethylamino-ethylamino)-carbonylmethyl]-N-methylsulfonyl-amino}-anilino)-1-phenyl-methyliden]-5,6-diethoxy-2-indolinon
(189) 3-(Z)-[1-(4-{N-[N-(2-Dimethylamino-ethyl)-N-methyl-amino)-carbonylmethyl]-N-methylsulfonyl-amino}-anilino)-1-phenyl-methyliden]-5,6-diethoxy-2-indolinon
(190) 3-(Z)-(1-{4-[N-(Dimethylaminocarbonylmethyl)-N-methylsulfonyl-amino]-anilino}-1-phenyl-methyliden)-5,6-diethoxy-2-indolinon
(191) 3-(Z)-(1-{4-[N-(Diethylaminocarbonylmethyl)-N-methylsulfonyl-amino]-anilino}-1-phenyl-methyliden)-5,6-diethoxy-2-indolinon
(192) 3-(Z)-{1-{4-[N-(Pyrrolidin-1-yl-carbonylmethyl)-N-methylsulfonyl-amino]-anilino}-1-phenyl-methyliden)-5,6-diethoxy-2-indolinon
(193) 3-(Z)-(1-{4-[N-(Piperidin-1-yl-carbonylmethyl)-N-methylsulfonyl-amino]-anilino}-1-phenyl-methyliden)-5,6-dimethoxy-2-indolinon
(194) 3-(Z)-(1-(4-[N-(Piperazin-1-yl-carbonylmethyl)-N-methylsulfonyl-amino]-anilino}-1-phenyl-methyliden)-5,6-diethoxy-2-indolinon
(195) 3- (Z)-[1-[4- {N-[(Morpholin-4-yl) -carbonylmethyl] -N-methylsulfonyl-amino}-anilino]-1-phenyl-methyliden] -5, 6-diethoxy-2-indolinon
(196) 3-(Z)-{1-[4-(2-Dimethylamino-ethoxy)-anilino]-1-phenyl-methyliden}-5,6-diethoxy-2-indolinon
(197) 3-(Z)-{1-[4-(3-Dimethylamino-propoxy)-anilino]-1-phenyl-methyliden}-5,6-diethoxy-2-indolinon
(198) 3-(Z)-{1-[4-(Aminocarbonylmethyl)-anilino]-1-phenyl-methyliden}-5,6-diethoxy-2-indolinon
(199) 3-(Z)-{1-[4-(2-Aminocarbonyl-ethyl)-anilino]-1-phenyl-methyliden}-5,6-diethoxy-2-indolinon
(200) 3-(Z)-{1-[4-(1H-Imidazol-4-yl)-anilino]-1-phenyl-methyliden}-5,6-diethoxy-2-indolinon
(201) 3-(Z)-{1-[4-(pyridin-2-yl)-anilino]-1-phenyl-methyliden}-5,6-diethoxy-2-indolinon
(202) 3-(Z)-{1-[4-(Pyridin-3-yl)-anilino]-1-phenyl-methyliden}-5,6-diethoxy-2-indolinon
(203) 3-(Z)-{1-[4-(Pyridin-4-yl)-anilino]-1-phenyl-methyliden}-5,6-diethoxy-2-indolinon

### Beispiel 12

### Trockenampulle mit 75 mg Wirkstoff pro 10 ml

### Zusammensetzung:

| | |
|---|---|
| Wirkstoff | 75,0 mg |
| Mannitol | 50,0 mg |
| Wasser für Injektionszwecke | ad 10,0 ml |

### Herstellung:

Wirkstoff und Mannitol werden in Wasser gelöst. Nach Abfüllung wird gefriergetrocknet. Die Auflösung zur gebrauchsfertigen Lösung erfolgt mit Wasser für Injektionszwecke.

### Beispiel 13

### Trockenampulle mit 35 mg Wirkstoff pro 2 ml

### Zusammensetzung:

| | |
|---|---|
| Wirkstoff | 35,0 mg |
| Mannitol | 100,0 mg |
| Wasser für Injektionszwecke | ad 2,0 ml |

### Herstellung:

Wirkstoff und Mannitol werden in Wasser gelöst. Nach Abfüllung wird gefriergetrocknet.

Die Auflösung zur gebrauchsfertigen Lösung erfolgt mit Wasser für Injektionszwecke.

### Beispiel 14

### Tablette mit 50 mg Wirkstoff

### Zusammensetzung:

| | |
|---|---|
| (1) Wirkstoff | 50,0 mg |
| (2) Milchzucker | 98,0 mg |
| (3) Maisstärke | 50,0 mg |
| (4) Polyvinylpyrrolidon | 15,0 mg |
| (5) Magnesiumstearat | 2,0 mg |
| | 215,0 mg |

### Herstellung:

(1), (2) und (3) werden gemischt und mit einer wäßrigen Lösung von (4) granuliert. Dem getrockneten Granulat wird (5) zugemischt. Aus dieser Mischung werden Tabletten gepreßt, biplan mit beidseitiger Facette und einseitiger Teilkerbe. Durchmesser der Tabletten: 9 mm.

### Beispiel 15

### Tablette mit 350 mg Wirkstoff

### Zusammensetzung:

| | |
|---|---|
| (1) Wirkstoff | 350,0 mg |
| (2) Milchzucker | 136, 0 mg |
| (3) Maisstärke | 80,0 mg |
| (4) Polyvinylpyrrolidon | 30,0 mg |
| (5) Magnesiumstearat | 4,0 mg |
| | 600,0 mg |

### Herstellung:

(1), (2) und (3) werden gemischt und mit einer wäßrigen Lösung von (4) granuliert. Dem getrockneten Granulat wird (5) zugemischt. Aus dieser Mischung werden Tabletten gepreßt, biplan mit beidseitiger Facette und einseitiger Teilkerbe. Durchmesser der Tabletten: 12 mm.

### Beipspiel 16

### Kapseln mit 50 mg Wirkstoff

### Zusammensetzung:

| | |
|---|---|
| (1) Wirkstoff | 50,0 mg |
| (2) Maisstärke getrocknet | 58,0 mg |
| (3) Milchzucker pulverisiert | 50,0 mg |
| (4) Magnesiumstearat | 2,0 mg |
| | 160,0 mg |

### Herstellung:

(1) wird mit (3) verrieben. Diese Verreibung wird der Mischung aus (2) und (4) unter intensiver Mischung zugegeben.

Diese Pulvermischung wird auf einer Kapselabfüllmaschine in Hartgelatine-Steckkapseln Größe 3 abgefüllt.

### Beispiel 17

### Kapseln mit 350 mg Wirkstoff

### Zusammensetzung:

| | |
|---|---|
| (1) Wirkstoff | 350,0 mg |
| (2) Maisstärke getrocknet | 46,0 mg |
| (3) Milchzucker pulverisiert | 30,0 mg |
| (4) Magnesiumstearat | 4,0 mg |
| | 430,0 mg |

### Herstellung:

(1) wird mit (3) verrieben. Diese Verreibung wird der Mischung aus (2) und (4) unter intensiver Mischung zugegeben.

Diese Pulvermischung wird auf einer Kapselabfüllmaschine in Hartgelatine-Steckkapseln Größe 0 abgefüllt.

### Beispiel 18

### Suppositorien mit 100 mg Wirkstoff

| 1 Zäpfchen enthält: | |
|---|---|
| Wirkstoff | 100,0 mg |
| Polyethylenglykol (M.G. 1500) | 600,0 mg |
| Polyethylenglykol (M.G. 6000) | 460,0 mg |
| Polyethylensorbitanmonostearat | 840,0 mg |
| | 2 000,0 mg |

### Herstellung:

Das Polyethylenglykol wird zusammen mit Polyethylensorbitanmonostearat geschmolzen. Bei 40°C wird die gemahlene Wirksubstanz in der Schmelze homogen dispergiert. Es wird auf 38°C abgekühlt und in schwach vorgekühlte Suppositorienformen ausgegossen.

## Patentansprüche

1. Substituierte Indolinone der allgemeinen Formel 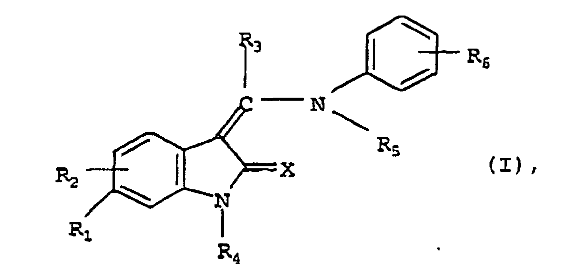
in der
X ein Sauerstoff- oder Schwefelatom,
R₁ eine C₂₋₃-Alkenyl-, C₂₋₃-Alkinyl-, Aryl-, Aryl-C₁₋₃-alkyl-, Heteroaryl-, Heteroaryl-C₁₋₃-alkyl-, Trifluormethyl- oder cyanogruppe,
eine Hydroxy-, C₁₋₃-Alkoxy-, Hydroxy-C₁₋₃-alkyl-, C₁₋₃-Alkoxy-C₁₋₃-alkyl-, Aryloxy- oder Heteroaryloxygruppe,
eine Mercapto-, C₁₋₃-Alkylsulfenyl-, Phenylsulfenyl-, Benzylsulfenyl-, C₁₋₃-Alkylsulfinyl-, Phenylsulfinyl-, Benzylsulfinyl-, C₁₋₃-Alkylsulfonyl-, Phenylpulfonyl-, Benzylsulfonyl-, Sulfo-, C₁₋₃-Alkoxysulfonyl-, Phenoxysulfynyl- oder Benzyloxysulfonylgruppe,
eine Amino-, C₁₋₃-Alkylamino-, Di-(C₁₋₃-Alkyl)-amino-, Hydroxycarbonyl-C₁₋₃-alkylamino-, N-(C₁₋₃-Alkyl)-hydroxycarbonyl-C₁₋₃-alkylamino-, C₁₋₃-Alkoxycarbonyl-C₁₋₃-alkylamino-, N-(C₁₋₃-Alkyl)-C₁₋₃-alkoxycarbonyl-C₁₋₃-alkylamino-, Phenylamino-, N-Phenyl-C₁₋₃-alkylamino-, N,N-Diphenylamino-, Benzylamino-, N-Benzyl-C₁₋₃-alkylamino-, N,N-Dibenzylamino-, C₁₋₃-Alkylcarbonylamino-, Benzoylamino- oder Benzylcarbonylaminogruppe oder eine N-(C₁₋₃-Alkyl)-C₁₋₃-alkylcarbonylaminogruppe, in der die beiden Alkylgruppen durch eine C₂₋₅-a-Alkylenbrücke ersetzt sein können oder in der eine oder beide Alkylgruppen durch eine Phenyl- oder Benzylgruppe ersetzt sein können,
eine C₁₋₃-Alkylsulfonyamino-, Phenylsulfonyamino- oder Benzylsulfonyaminogruppe oder eine N-(C₁₋₃-Alkyl)-C₂₋₃-alkylsulfonylaminogruppe, in der die beiden Alkylgruppen durch eine C₂₋₅-n-Alkylenbrücke ersetzt sein können oder in der eine oder beide Alkylgruppen durch eine Phenyl- oder Benzylgruppe ersetzt sein können,
eine Aminosulfonyl-, C₁₋₃-Alkylaminosulfonyl-, Phenylaminosulfonyl-, Benzylaminosulfonyl-, Di-(C₁₋₃-Alkyl)-aminosulfonyl-N,N-Diphenyl-aminosulfonyl- oder N,N-Dibenzyl-aminosulfonylgruppe,
eine Phosphono-, (C₁₋₃-Alkoxy)PO(H)-, (C₁₋₃-Alkoxy)PO(C₁₋₃-Alkyl)-, (C₁₋₃-Alkoxy)PO(OH)-, Di-(C₁₋₃-Alkoxy)-PO- oder (C₂₋₄-n-Alkylendioxy)-PO-Gruppe,
eine gegebenenfalls durch C₁₋₃-Alkylgruppen mono-, di- oder trisubstituierte Ureidogruppe,
eine 4- bis 7-gliedrige Cycloalkylenimino- oder Cycloalkyleniminosulfonylgruppe, wobei jeweils die Methylengruppe in Position 4 einer 6- oder 7-gliedrigen Cycloalkyleniminogruppe durch ein Sauerstoff- oder Schwefelatom, durch eine Sulfinyl-, Sulfonyl-, -NH- oder -N(C₁₋₃-Alkyl)-Gruppe ersetzt sein kann,
R₂ ein Wasserstoff-, Fluor-, Chlor-, Brom- oder Jodatom,
eine C₁₋₆-Alkyl- oder Trifluormethylgruppe,
eine Hydroxy-, C₁₋₃-Alkoxy-, Mercapto-, C₁₋₃-Alkylsulfenyl-, C₁₋₃-Alkylsulfinyl-, C₁₋₃-Alkylsulfonyl-, Sulfo-, C₁₋₃-Alkoxysulfonyl-, Aminosulfonyl-, C₁₋₃-Alkylaminosulfonyl- oder Di-(C₁₋₃-Alkyl)-aminosulfonylgruppe,
eine Nitro-, Amino-, C₁₋₃-Alkylamino- oder Di-(C₁₋₃-Alkyl)-aminogruppe,
eine C₁₋₃-Alkylcarbonyl-, Cyano-, Carboxy-, C₁₋₃-Alkoxycarbonyl-, Aminocarbonyl-, C₁₋₃-Alkylaminocarbonyl- oder Di-(C₁₋₃-Alkyl)-aminocarbonylgruppe,
eine Phosphono-, (C₁₋₃-Alkoxy)PO(H)-, (C₁₋₃-Alkoxy) PO (C₁₋₃-Alkyl)-, (C₁₋₃-Alkoxy)PO(OH)- oder Di-(C₁₋₃-Alkoxy)-PO-Gruppe,
eine 4- bis 7-gliedrige Cycloalkylenimino-, cycloalkyleniminocarbonyl- oder Cycloalkyleniminosulfonylgruppe, wobei jeweils die Methylengruppe in Position 4 einer 6- oder 7-gliedrigen Cycloalkyleniminogruppe durch ein Sauerstoff- oder Schwefelatom, durch eine Sulfinyl-, Sulfonyl-, -NH- oder -N(C₁₋₃-Alkyl)-Gruppe ersetzt sein kann, oder
R₁ und R₂ zusammen eine Methylendioxy-, Ethylendioxy-, n-Propylen-, n-Butylen- oder 1,4-Butadienylengruppe,
R₃ eine Phenyl-gruppe
R₄ ein Wasserstoffatom, eine C₁₋₃-Alkylgruppe,
eine Hydroxygruppe, eine Acylgruppe, eine C₁₋₁₆-Alkanoylgruppe, eine Allyloxycarbonylgruppe, eine C₁₋₁₆-Alkoxycarbonylgruppe, eine Phenyl-C₁₋₆-alkoxycarbonylgruppe, eine C₁₋₃-Alkylsulfonyl-C₂₋₄-alkoxycarbonyl-, C₁₋₃-Alkoxy-C₂₋₄-alkoxy-C₂₋₄-alkoxycarbonyl- oder RₑCO-O-(R_{f}CR_{g})-O-CO-Gruppe, in der
Rₑ eine C₁₋₈-Alkyl-, C₅₋₇-Cycloalkyl-, Phenyl- oder Phenyl-C₁₋₃-alkylgruppe.
R_{f} ein Wasserstoffatom, eine C₁₋₃-Alkyl-, C₅₋₇-Cycloalkyl- oder Phenylgruppe und
R_{g} ein Wasserstoffatom, eine C₁₋₃-Alkyl- oder RₑCO-O-(R_{f}CR_{g})-O-Gruppe, in der Rₑ bis Rg wie vorstehend erwähnt definiert sind, darstellen,
R₅ ein wasserstoffatom oder eine C₁₋₃-Alkylgruppe und
R₆ ein Wasserstoff-, Fluor-, Chlor-, Brom- oder Jodatom,
eine Trifluormethyl- oder Heteroarylgruppe, eine gegebenenfalls durch 1 bis 3 Fluoratome substituierte C₁₋₃-Alkoxygruppe, eine Amino-c₁₋₃-alkoxy-, C₁₋₃-Alkylamino-C₂₋₃-alkoxy- oder Benzylamino-C₂₋₃-alkoxygruppe, eine CycloalkyleniminoC₂₋₃-alkoxygruppe mit 4 bis 7 Ringgliedern, eine Di-(C₁₋₃-Alkyl)-amino-C₂₋₃-alkoxy- oder C₁₋₃-Alkylmercaptogruppe,
eine Nitro-, Cyano-, Carboxy-, C₁₋₃-Alkoxycarbonyl-, Aminocarbonyl-, C₁₋₃-Alkylaminocarbonyl-, Di-(C₁₋₃-Alkyl)-aminocarbonyl-, Piperidinocarbonyl- oder Tetrazolylgruppe,
eine am Stickstoffatom gegebenenfalls durch eine C₁₋₃-Alkylgruppe substituierte C₁₋₃-Alkylcarbonylaminogruppe,
eine gegebenenfalls an der Iminogruppe durch eine C₁₋₃-Alkylgruppe substituierte Imidazolyl- oder Piperazinogruppe,
eine C₁₋₄-Alkylgruppe, die terminal
durch eine Hydroxy-, C₁₋₃-Alkoxy-, Carboxy-, C₁₋₃-Alkoxycarbonyl-, Amino-, C₁₋₄-Alkylamino-, Di-(C₁₋₄-Alkyl)-amino-, Phenylamino-, N-Phenyl-C₁₋₃-alkylamino-, Phenyl-n-C₁₋₃-alkylamiao-, N-(C₁₋₃-Alkyl)-Phenyl-n-C₁₋₃-alkylamino- oder Di-(Phenyl-n-C₁₋₃-alkyl)-aminogruppe,
durch eine 4- bis 7-gliedrige Cycloalkyleniminogruppe, in der
eine mit der Iminogruppe verknüpfte Methylengruppe durch eine Carbonyl-oder Sulfonylgruppe ersetzt sein kann oder
ein oder zwei Wasserstoffatome jeweils durch eine C₁₋₃-Alkylgruppe ersetzt sein können oder/und
jeweils die Methylengruppe in Position 4 einer 6- oder 7-gliedrigen Cycloalkyleniminogruppe durch eine Carboxy-, C₁₋₃-Alkoxycarbonyl-, Aminocarbonyl-, C₁₋₃-Alkylaminocarbonyl-, Di-(C₁-₃-Alkyl)-aminocarbonyl-, Phenyl-n-C₁₋₃-alkylamino- oder N-(C₁₋₃-Alkyl)-Phenyl-n-C₁₋₃-alkylaminogruppe substituiert oder
durch ein Sauerstoff- oder Schwefelatom, durch eine Sulfinyl-, Sulfonyl-, -NH- oder -N(C₁₋₃-Alkyl)-Gruppe ersetzt sein kann,
durch eine 5- bis 7-gliedrige Cycloalkenyleniminogruppe, in der die Doppelbindung vom Stickstoffatom isoliert ist, durch eine C₄₋₇-Cycloalkylamino-, N-(C₁₋₃-Alkyl)-C₄₋₇-cycloalkylamino- oder C₅₋₇-Cycloalkenylaminogruppe, in der die Position 1 des Rings nicht an der Doppelbindung beteiligt ist und in der das Stickstoffatom durch eine C₁₋₃-Alkylgruppe substituiert sein kann,
durch eine C₁₋₃-Alkylcarbonylamino-, N-(C₁₋₃-Alkyl)-C₁₋₃-alkylcarbonylamino-, Aminocarbonyl-, C₁₋₃-Alkylaminocarbonyl- oder Di-(C₁₋₃-Alkyl)-aminocarbonylgruppe substituiert sein kann,
oder R₆ eine Gruppe der Formel
-N(Rₐ)-CO-(CH₂)ₙ-R_{b} (II),
in der
Rₐ eine C₁₋₃-Alkylgruppe,
n eine der Zahlen 0, 1 oder 2 und
R_{b} eine Amino-, C₁₋₄-Alkylamino-, Phenylamino-, N-(C₁₋₄-Alkyl)-phenylamino-, Benzylamino-, N-(C₁₋₄-Alkyl)-benzylamino-oder Di-(C₁₋₄-Alkyl)-aminogruppe oder eine 4- bis 7-gliedrige Cycloalkyleniminogruppe, wobei jeweils die Methylengruppe in Position 4 einer 6- oder 7-gliedrigen Cycloalkyleniminogruppe durch ein Sauerstoff- oder Schwefelatom, durch eine Sulfinyl-, Sulfonyl-, -NH- oder -N(₁₋₃-Alkyl)-Gruppe ersetzt sein kann, bedeuten,
eine Gruppe der Formel
-N(R_{c})-(CH₂)ₘ-(CO)ₒ-R_{d} (III),
in der
R_{c} eine C₁₋₃-Alkyl-, C₁₋₃-Alkylcarbonyl-, Arylcarbonyl-, Benzylcarbonyl-, C₁₋₃-Alkylsulfonyl-, Arylsulfonyl- oder Benzylsulfonylgruppe,
m eine der Zahlen 1, 2, 3 oder 4,
o eine der Zahlen 0 oder 1 bedeuten und'
R_{d} die für R_{b} vorstehend erwähnten Bedeutungen besitzt oder eine in 1-Position gegebenenfalls durch eine C₁₋₃-Alkylgruppe substituierte Di-(C₁₋₄-Alkyl)-amino-C₁₋₃-alkylaminogruppe darstellt,
oder eine N-(C₁₋₃-Alkyl)-C₁₋₃-alkylsulfonylaminogruppe,
wobei zusätzlich eine vorhandene Carboxy-, Amino- oder Iminogruppe durch einen in-vivo abspaltbaren Rest substituiert sein kann,
wobei dieser Rest eine Hydroxygruppe, eine Acylgruppe, eine C₁₋₁₆₋Alkanoylgruppe, eine Allyloxycarbonylgruppe, eine C₁₋₁₆-Alkoxycarbonylgruppe, eine Phenyl-C₁₋₆-alkoxycarbonylgruppe, eine C₁₋₃-Alkylsulfonyl-C₂₋₄-alkoxycarbonyl-, C₁₋₃-Alkoxy-C₂₋₄-alkoxy-C₂₋₄-alkoxycarbonyl- oder RₑCO-O-(R_{f}CR_{g})-O-CO-Gzuppe, in der
Rₑ eine C₁₋₈-Alkyl-, C₅₋₇-Cyclcalkyl-, Phenyl- oder Phenyl-C₁₋₃-alkylgruppe,
R_{f} ein Wasserstoffatom, eine C₁₋₃-Alkyl-, C₅₋₇₋Cycloalkyl- oder Phenylgruppe und
R_{g} ein Wasserstoffatom, eine C₁₋₃-Alkyl- oder RₑCO-O-(R_{f}CR_{g})-O-Gruppe, in der Rₑ bis R_{g} wie vorstehend erwähnt definiert sind, darstellen,
bedeuten kann, bedeuten, deren Enantiomere und/oder Diastereomere und deren Salze.

2. substituierte Indolinone der allgemeinen Formel I gemäß Anspruch 1, in denen
x ein Sauerstoffatom,
R₁ eine C₁₋₃-Alkoxy-, Trifluormethyl-, Di-(C₁₋₃-Alkyl)-amino-, Pyrrolidino- oder Pyrrologruppe,
eine Amino- oder C₁₋₃-Alkylaminogruppe, in denen jeweils ein Aminwasserstoffatom durch eine C₁₋₃-Alkylcarbonyl-, Phenyl-C₁₋₃-alkylcarbonyl-, Benzoyl-, Aminocarbonyl-, C₁₋₃-Alkylsulfonyl-, Phenylsulfonyl-, Carboxy-C₁₋₃-alkyl- oder C₁₋₃-Alkyloxycarbonyl-C₁₋₃-alkylgruppe ersetzt sein kann, oder
eine gegebenenfalls durch eine C₁₋₃-Alkylgruppe substituierte Phenylgruppe,
R₂ ein Wasserstoffatom oder eine C₁₋₃-Alkoxygruppe oder
R₁ und R₂ zusammen eine Methylendioxygruppe,
R₃ Phenylgruppe,
R₄ ein Wasserstoffatom,
R₅ ein wasserstoffatom und
R₆ ein Wasserstoff-, Fluor-, Chlor-, Brom- oder Jodatom,
eine Trifluormethyl-, 4-(C₁₋₃-Alkyl)-piperazino-, Pyridinyl-, Imidazolyl-, Tetrazolyl-, C₁₋₃-Alkoxy- oder C₁₋₃-Alkylmercaptogruppe,
eine Nitro-, Cyano-, Carboxy- oder C₁₋₃-Alkyloxycarbonylgruppe oder eine am Stickstoffatom gegebenenfalls durch eine C₁₋₃-Alkylgruppe substituierte C₁₋₃-Alkylcarbonylaminogruppe,
eine Piperidinocarbonylgruppe oder eine gegebenenfalls durch eine oder zwei C₁₋₃-Alkylgruppen substituierte Aminocarbonylgruppe,
eine C₁₋₃-Alkylgruppe, die terminal
durch eine Amino-, C₁₋₄-Alkylamino-, Di-(C₁₋₄-Alkyl)-amino-, Phenylamino-, N-Phenyl-C₁₋₃-alkylamino-, Phenyl-n-C₁₋₃-alkylamino-, N-(C₁₋₃-Alkyl)-Phenyl-n-C₁₋₃-alkylamino- oder Di-(Phenyl-n-C₁₋₃-alkyl)-aminogruppe, durch eine Pyrrolidino-, Piperidino-, Hexamethylenimino-, Morpholino-, Thiomorpholino-, 1-Oxido-thiomorpholino- oder Piperazinogruppe,
wobei die Piperidinogruppe zusätzlich durch eine oder zwei C₁₋₃-Alkylgruppen oder durch eine Carboxy-, C₁₋₃-Alkoxycarbonyl-, Aminocarbonyl-, C₁₋₃-Alkylaminocarbonyl- Di-(C₁₋₃-Alkyl)-aminocarbonyl- oder N-(C₁₋₃-Alkyl)-Phenyl-n-C₁₋₃-alkylaminogruppe substituiert sein kann,
durch eine C₅₋₇-Cycloalkylamino- oder C₅₋₇-Cycloalkenylaminogruppe, in der die Position 1 des Rings nicht an der Doppelbindung beteiligt ist,
durch eine C₁₋₃-Alkylcarbonylamino-, N-(C₁₋₃-Alkyl)-C₁₋₃-alkylcarbonylamino-, Carboxy-, C₁₋₃-Alkoxycarbonyl-, Aminocarbonyl-, C₁₋₃-Alkylaminocarbonyl- oder Di-(C₁₋₃-Alkyl)-aminocarbonylgruppe substituiert sein kann,
eine C₁₋₃-Alkoxygruppe, die terminal durch eine Amino-, C₁₋₃-Alkylamino- oder Di-(C₁-₃-Alkyl)-aminogruppe substituiert ist,
eine Gruppe der Formel
-N(Rₐ)-CO-(CH₂)ₙ-R_{b} (II),
in der
Rₐ eine C₁₋₃-Alkylgruppe,
n eine der Zahlen 0, 1 oder 2 und
R_{b} eine Amino-, C₁₋₄-Alkylamino- oder Di-(C₁₋₄-Alkyl)-aminogruppe oder eine Pyrrolidino-, Piperidino-, Hexamethylenimino-, Morpholino-, Thiomorpholino-, 1-Oxido-thiomorpholino- oder Piperazinogruppe bedeuten,
eine Gruppe der Formel
-N(R_{c})-(CH₂)ₘ-(CO)ₒ-R_{d} (III),
in der
R_{c} eine C₁₋₃₋Alkyl-, C₁₋₃-Alkylcarbonyl- oder C₁₋₃-Alkylsulfonylgruppe,
m eine der Zahlen 1, 2, 3 oder 4,
o eine der Zahlen 0 oder 1 bedeuten und
R_{d} die für R_{b} vorstehend erwähnten Bedeutungen besitzt oder eine in 1-Position gegebenenfalls durch eine C₁₋₃-Alkylgruppe substituierte Di-(C₁₋₄-Alkyl)-amino-C₁₋₃-alkylaminogruppe darstellt,
oder eine N-(C₁₋₃-Alkyl)-C₁₋₃-alkylsulfonylaminogruppe,
bedeuten, deren Enantiomere und/oder Diastereomere und deren Salze.

3. Substituierte Indolinone der allgemeinen Formel I gemäß Anspruch 1, in denen
X ein Sauerstoffatom,
R₁ eine Methoxy-, Ethoxy-, Trifluormethyl-, Phenyl-, Methylphenyl-, Dimethylamino-, Pyrrolidino- oder Pyrrologruppe,
eine Aminogruppe, die durch eine Methyl-, Carboxymethyl-, Methoxycarbonylmethyl-, Acetyl-, Phenylacetyl-, Benzoyl-, Methansulfonyl-, Benzolsulfonyl- oder Aminocarbonylgruppe substituiert sein kann,
R₂ ein Wasserstoffatom, eine Methoxy- oder Ethoxygruppe oder
R₁ und R₂ zusammen eine Methylendioxygruppe,
R₃ eine Phenylgruppe,
R₄ ein Wasserstoffatom,
R₅ ein Wasserstoffatom und
R₆ ein Wasserstoff-, Fluor-, Chlor-, Brom- oder Jodatom,
eine Methyl-, Trifluormethyl-, Methoxy-, Ethoxy-, Methylmercapto-, Cyano-, Carboxy-, Methoxycarbonyl-, Ethoxycarbonyl-, Aminocarbonyl-, Dimethylaminocarbonyl-, Piperidinocarbonyl-, Nitro-, 4-Methyl-piperazino-, Imidazolyl-, Pyridinyl- oder Tetrazolylgruppe,
eine endständig durch eine Dimethylaminogruppe substituierte Ethyloxy- oder n-Propyloxygruppe,
eine durch eine Carboxy-, Methoxycarbonyl-, Ethoxycarbonyl-, Aminocarbonyl- oder Dimethylaminocarbonylgruppe substituierte Methyl- oder Ethylgruppe,
eine C₁₋₃-Alkylgruppe, die endständig
durch eine Amino-, C₁₋₄-Alkylamino-, Cyclohexylamino-, Benzylamino- oder Phenylaminogruppe, in denen jeweils ein Wasserstoffatom des Aminstickstoffatoms durch eine C₁₋₃-Alkyl-, Benzyl-, Acetyl- oder Dimethylaminocarbonylgruppe ersetzt sein kann,
durch eine gegebenenfalls durch eine oder zwei Methylgruppen substituierte Piperidinogruppe,
durch eine durch eine Carboxy-, Methoxycarbonyl-, Ethoxycarbonyl- oder Dimethylaminocarbonylgruppe substituierte Piperidinogruppe,
durch eine Pyrrolidino-, 3,4-Dehydro-piperidino-, Hexamethylenimino-, Morpholino-, Thiomorpholino-, 1-Oxo-thiomorpholino- oder Piperazinogruppe substituiert ist,
eine C₁₋₃-Alkylaminogruppe, in der das Wasserstoffatom des Aminstickstoffatoms
durch eine Ethyl- oder n-Propylgruppe, die jeweils endständig durch eine Dimethylaminogruppe substuituiert ist, durch eine C₂₋₃-Alkanoylgruppe, die in 2- oder 3-Stellung durch eine Amino-, C₁₋₃-Alkylamino-, Di-(C₁₋₃-Alkyl)-amino-, Pyrrolidino-, Piperidino-, Morpholino- oder Piperazinogruppe substituiert sein kann,
durch eine Aminocarbonyl-, Methylaminocarbonyl-, Dimethylaminocarbonyl-, Piperidinocarbonyl- oder Methansulfonylgruppe ersetzt ist,
und zusätzlich der C₁₋₃-Alkylteil der C₁₋₃-Alkylaminogruppe
durch eine Aminocarbonylgruppe,
durch eine C₁₋₃-Alkylaminocarbonyl- oder Di-(C₁₋₃-Alkyl)-aminocarbonylgruppe, in denen ein C₂₋₃-Alkylteil endständig zusätzlich durch eine Dimethylaminogruppe substituiert sein kann,
durch eine Pyrrolidinocarbonyl-, Piperidinocarbonyl-, Morpholinocarbonyl- oder Piperazinocarbonylgruppe substituiert sein kann,
wobei der C₂₋₃-Alkylteil der vorstehend erwähnten C₁₋₃-Alkylaminogruppe endständig außerdem durch eine Amino-, C₁₋₃-Alkylamino-, Di-(C₁₋₃-Alkyl)-amino-, Pyrrolidino-, Piperidino-, Morpholino- oder Piperazinogruppe substituiert sein kann,
bedeuten, deren Enantiomere und/oder Diastereomere und deren Salze.

4. Substituierte Indolinone der allgemeinen Formel I gemäß Anspruch 2 oder 3, in denen
X, R₁ und R₃ bis R₆ wie im Anspruch 2 definiert sind und
R₂ ein Wasserstoffatom darstellt,
deren Enantiomere und/oder Diastereomere und deren Salze.

5. Substituierte Indolinone der allgemeinen Formel I gemäß Anspruch 2 oder 3, in denen
X und R₃ bis R₆ wie im Anspruch 2 definiert sind,
R₁ und R₂, die gleich oder verschieden sein können, jeweils eine C₁₋₃-Alkoxygruppe darstellen,
deren Enantiomere und/oder Diastereomere und deren Salze.

6. Substituierte Indolinone der allgemeinen Formel I gemäß Anspruch 1, in denen
X ein Sauerstoffatom,
R₁ eine Amino-, Methoxy- oder Ethoxygruppe,
R₂ ein Wasserstoffatom oder in Position 5 eine Methoxy- oder Ethoxygruppe,
R₃ eine Phenylgruppe,
R₄ und R₅ jeweils ein Wasserstoffatom und
R₆ eine durch eine Methylamino-, Ethylamino-, Piperidino- oder 4-(Dimethylaminocarbonyl)-piperidinogruppe substituierte Methyl- oder Ethylgruppe, wobei das Amino-Wasserstoffatom der Methylamino- und Ethylaminogruppe durch eine Methyl- oder Benzylgruppe ersetzt ist, eine N-Dimethylaminomethylcarbonyl-N-methyl-aminogruppe oder eine N-Acetyl-N-(C₂₋₃-alkyl)-aminogruppe, in der der C₂₋₃-Alkylteil jeweils endständig durch eine Dimethylaminogruppe substituiert ist, bedeuten,
und deren Salze.

7. Folgende substituierte Indolinone der allgemeinen Formel I gemäß Anspruch 1:
(a) 3-(Z)-{1-[4-(Piperidin-1-yl-methyl)-anilino]-1-phenyl-methyliden}-5,6-dimethoxy-2-indolinon,
(b) 3-(Z)-(1-{4-[(N-Benzyl-N-methyl-amino)-methyl]-anilino}-1-phenyl-methyliden)-5,6-dimethoxy-2-indolinon,
(c) 3-(Z)-{1-(4-(Dimethylamino-methyl)-anilino]-1-phenyl-methyliden}-5,6-dimethoxy-2-indolinon,
(d) 3-(Z)-{1-[4-(N-Dimethylaminomethylcarbonyl-N-methyl-amino)-anilino]-1-phenyl-methyliden}-5,6-dimethoxy-2-indolinon,
(e) 3-(Z)-(1-{4-[2-(4-Dimethylcarboxamid-piperidin-1-yl)-ethyl]-anilino}-1-phenyl-methyliden)-5,6-dimethoxy-2-indolinon,
(f) 6-Amino-3- (Z) -{1- [4-(piperidin-1-yl-methyl)-anilino]-1-phenyl-methyliden}-2-indolinon,
(g) 3-(Z)-{1-{4-(N-Acetyl-N-(2-dimethylamino-ethyl)-amino]-anilino}-1-phenyl-methyliden)-5,6-dimethoxy-2-indolinon,
(h) 3-(Z)-(1-{4-[N-Acetyl-N-(3-dimethylamino-propyl)-amino]-anilino}-1-phenyl-methyliden)-5,6-dimethoxy-2-indolinon
und deren Salze.

8. Physiologisch verträgliche Salze der Verbindungen gemäß den Ansprüchen 1 bis 7.

9. Arzneimittel, enthaltend eine Verbindung nach mindestens einem der Ansprüche 1 bis 7 oder ein Salz gemäß Anspruch 8 neben gegebenenfalls einem oder mehreren inerten Trägerstoffen und/oder Verdünnungsmitteln.

10. Verwendung einer Verbindung nach mindestens einem der Ansprüche 1 bis 7 oder ein Salz gemäß Anspruch 8 zur Herstellung eines Arzneimittels, welches zur Behandlung von exzessiven oder anomalen Zellproliferationen geeignet ist.

11. Verfahren zur Herstellung eines Arzneimittels gemäß Anspruch 9, **dadurch gekennzeichnet, daß** auf nichtchemischem Wege eine Verbindung nach mindestens einem der Ansprüche 1 bis 7 oder ein Salz gemäß Anspruch 8 in einen oder mehrere inerte Trägerstoffe und/oder Verdünnungsmittel eingearbeitet wird.

12. Verfahren zur Herstellung der Verbindungen gemäß den Ansprüchen 1 bis 8, **dadurch gekennzeichnet, daß**
eine Verbindung der allgemeinen Formel 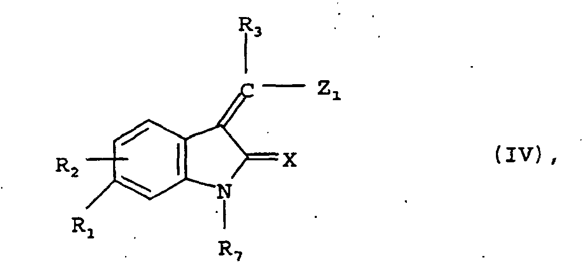
in der
X und R₁ bis R₃ wie in den Ansprüchen 1 bis 7 erwähnt definiert sind,
R₇ ein Wasserstoffatom, eine Schutzgruppe für das Stickstoffatom der Lactamgruppe oder eine Bindung an eine Festphase und
Z₁ ein Halogenatom, eine Hydroxy-, Alkoxy- oder Arylalkoxygruppe bedeuten,
mit einem Amin der allgemeinen Formel 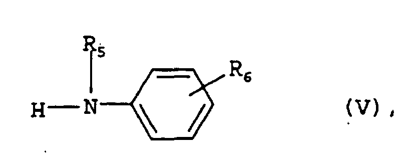
in der
R₅ und R₆ wie in den Ansprüchen 1 bis 7 erwähnt definiert sind, umgesetzt und erforderlichenfalls anschließend eine verwendete Schutzgruppe für das Stickstoffatom der Lactamgruppe oder eine so erhaltene Verbindung von einer Festphase abgespalten wird und
gewünschtenfalls anschließend eine so erhaltene Verbindung der allgemeinen Formel I, die eine Alkoxycarbonylgruppe enthält, mittels Hydrolyse in eine entsprechende Carboxyverbindung übergeführt wird oder
eine so erhaltene Verbindung der allgemeinen Formel I, die eine Amino- oder Alkylaminogruppe enthält, mittels Alkylierung oder reduktiver Alkylierung in eine entsprechende Alkylamino-, Dialkylamino- oder Pyrrolidinoverbindung übergeführt wird oder
eine so erhaltene Verbindung der allgemeinen Formel I, die eine Amino- oder Alkylaminogruppe enthält, mittels Acylierung in eine entsprechende Acylverbindung übergeführt wird oder eine so erhaltene Verbindung der allgemeinen Formel I, die eine Amino- oder Alkylaminogruppe enthält, mittels Sulfonierung in eine entsprechende Sulfonylverbindung übergeführt wird oder
eine so erhaltene Verbindung der allgemeinen Formel I, die eine Aminogruppe enthält, mittels einer Kondensationreaktion in eine entsprechende Pyrroloverbindung übergeführt wird oder
eine so erhaltene Verbindung der allgemeinen Formel I, die eine Carboxygruppe enthält, mittels Veresterung oder Amidierung in eine entsprechende Ester- oder Aminocarbonylverbindung übergeführt wird oder
eine so erhaltene Verbindung der allgemeinen Formel I, die eine Cyanogruppe enthält, Reduktion in eine entsprechende Aminomethylverbindung übergeführt wird oder
eine so erhaltene Verbindung der allgemeinen Formel I, die eine Amino- oder Alkylaminogruppe enthält, mittels Umsetzung mit Cyansäure oder einem entsprechenden Isocyanat in eine entsprechende Ureidoverbindung übergeführt wird und
erforderlichenfalls ein während den Umsetzungen zum Schutze von reaktiven Gruppen verwendeter Schutzrest abgespalten wird oder
gewünschtenfalls anschließend eine so erhaltene Verbindung der allgemeinen Formel I in ihre Stereoisomere aufgetrennt wird oder
eine so erhaltene Verbindung der allgemeinen Formel I in ihre Salze, insbesondere für die pharmazeutische Anwendung in ihre physiologisch verträglichen Salze mit einer anorganischen oder organischen Säure oder Base, übergeführt wird.

13. Verfahren gemäß Anspruch 12, **dadurch gekennzeichnet, daß** zur Herstellung einer Verbindung der allgemeinen Formel I, in der R₁ eine Aminogruppe darstellt, eine Verbindung der allgemeinen Formel 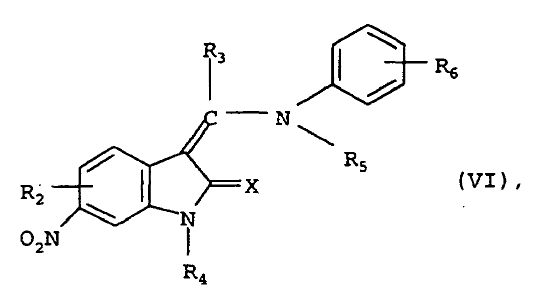 reduziert wird.

14. Verfahren gemäß Anspruch 12, **dadurch gekennzeichnet, daß** zur Herstellung einer Verbindung'der allgemeinen Formel I, in der R₁ oder/und R₂ eine der in den Ansprüchen 1 bis 7 erwähnten substituierten Sulfinyl- oder Sulfonylgruppen darstelit, eine Verbindung der allgemeinen Formel 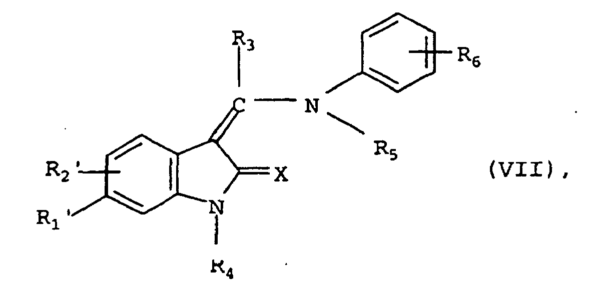
in der
R₃ bis R₆ wie in den Ansprüchen 1 bis 7 erwähnt definiert sind und
eine der Gruppen R₁' und R₂' eine der für R₁ und R₂ in den Ansprüchen 1 bis 6 erwähnten substituierten Mercapto- oder Sulfinylgruppen bedeutet und die andere die für R₁ oder R₂ mit Ausnahme der Mercapto- oder Sulfinylgruppen in den Ansprüchen 1 bis 7 erwähnten Bedeutungen annimmt oder beide Gruppen R_{1'} und R_{2'} eine der für R₁ und R₂ in den Ansprüchen 1 bis 6 erwähnten substituierten Mercapto- oder Sulfinylgruppen darstellen, oxidiert wird.

## Revendications

1. Indolinones substituées de formule générale 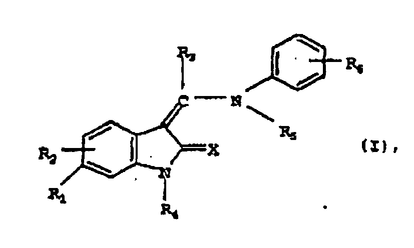
dans laquelle
X est un atome d'oxygène ou un atome de soufre,
R₁ est un groupe alkényle en C₂ à C₃, alkinyle en C₂ à C₃, aryle, aryle-alkyle en C₁ à C₃, hétéroaryle, hétéroaryle-alkyle en C₁ à C₃, trifluorométhyle ou cyano,
un groupe hydroxy, alcoxy en C₁ à C₃, hydroxy-alkyle en C₁ à C₃, alcoxy en C₁ à C₃, alkyle en C₁ à C₃, aryloxy ou hétéroaryloxy,
un groupe mercapto, alkylsulfényle en C₁ à C₃, phénylsulfényle, benzylsulfényle, alkylsulfinyle en C₁ à C₃, phénylsulfinyle, benzylsulfinyle, alkylesulfonyle en C₁ à C₃, phénylsulfonyle, benzylsulfonyle, sulfo, alcoxysulfonyle en C₁ à C₃, phénoxysulfonyle ou benzyloxysulfonyle,
un groupe amino, alkylamino en C₁ à C₃, di-(alkyle en C₁ à C₃)-amino-hydroxycarbonyl-alkylamino en Ci à C₃, N-(alkyle en C₁ à C₃)-hydroxy-carbonyl-alkylamino en C₁ à C₃, alcoxycarbonyle en C₁ à C₃-alkyle en C₁ à C₃-amino, N-(alkyle en C₁ à C₃)-alcoxycarbonyle en C₁ à C₃-alkylamino en C₁ à C₃, phénylamino, N-phényl-alkylamino en C₁ à C₃, N,N-diphénylamino, benzylamino, N-benzylalkylamino en C₁ à C₃, N,N-dibenzylamino, alkylcarbonylamino en C₁ à C₃, benzoylamino ou benzylcarbonyl-amino ou un groupe N-(alkyle en C₁ à C₃)-alkylcarbonylamino en C₁ à C₃, dans lequel les deux groupes alkyle peuvent être substitués par un pont n-alkylène en C₂ à C₅ ou dans lequel un ou les deux groupes alkyle peuvent être substitués par un groupe phényle ou benzyle,
un groupe alkylsulfonylamino en C₁ à C₃, phénylsulfonylamino ou benzylsulfonylamino ou un groupe N-(alkyle en C₁ à C₃)-alkylsulfonylamino en C₁ à C₃, dans lequel les deux groupes alkyle peuvent être substitués par un pont alkylène en C₂ à C₅ ou dans lequel un ou les deux groupes alkyle peuvent être substitués par un groupe phényle ou un groupe benzyle,
un groupe aminosulfonyle, un groupe alkylaminosulfonyle en C₁ à C₃, un groupe phénylaminosulfonyle, benzylaminosulfonyle, di-(alkyle en C₁ à C₃)-aminosulfonyle, N,N-diphényl-aminosulfonyle ou N,N-dibenzyl-aminosulfonyle,
un groupe phosphono, (alcoxy en C₁ à C₃)-PO(H), (alcoxy en C₁ à C₃)PO(alkyle en C₁ à C₃), (alcoxy en C₁ à C₃)PO(OH), di-(alcoxy en C₁ à C₃)-PO ou (n-alkylènedioxy en C₂ à C₄)-PO,
un groupe uréido éventuellement mono-, di-, ou tri-substitué par des groupes alkyle en C₁ à C₃,
un groupe cycloalkylèneimino ou cycloalkylèneiminosulfonyle comportant 4 à 7 chaînons, le groupe méthylène pouvant être respectivement substitué en position 4 d'un groupe cycloalkylèneimino à 6 à 7 chaînons, par un atome d'oxygène ou un atome de soufre, par un groupe sulfinyle, sulfonyle, NH ou N-(alkyle en C₁ à C₃),
R₂ est un atome d'hydrogène, de fluor, de chlore, de brome ou d'iode,
un groupe alkyle en C₁ à C₆ ou un groupe trifluorométhyle,
un groupe hydroxy, alcoxy en C₁ à C₃, mercapto, alkylsulfényle en C₁ à C₃, alkylsulfinyle en C₁ à C₃, alkylsulfonyle en C₁ à C₃, sulfo, alcoxy en C₁ à C₃₋sulfonyle, aminosulfonyle, alkylaminosulfonyle en C₁ à C₃ ou di-(alkyle en C₁ à C₃)-aminosulfonyle,
un groupe nitro, amino, alkylamino en C₁ à C₃ ou di-(alkyle en C₁ à C₃)-amino,
un groupe alkylcarbonyle en C₁ à C₃, cyano, carboxy, alcoxycarbonyle en C₁ à C₃, aminocarbonyle, alkylaminocarbonyle en C₁ à C₃ ou di-(alkyle en C₁ à C₃)-aminocarbonyle,
un groupe phosphono, (alcoxy en C₁ à C₃)PO(H), (alcoxy en C₁ à C₃)PO(alkyle en C₁ à C₃), (alcoxy en C₁ à C₃)PO(OH) ou di-(alcoxy en C₁ à C₃)-PO-,
un groupe cycloalkylèneimino, cycloalkylèneimino-carbonyle ou cycloalkylèneiminosulfonyle comportant 4 à 7 chaînons, le groupe méthylène pouvant être respectivement substitué en position 4 d'un groupe cycloalkylèneimino comportant 6 à 7 chaînons un atome d'oxygène ou un atome de soufre, par un groupe sulfinyle, sulfonyle, NH ou N-(alkyle en C₁ à C₃), ou
R₁ et R₂ sont conjointement un groupe méthylènedioxy, éthylènedioxy, n-propylène, n-butylène ou 1,4-butadiénylène,
R₃ est un groupe phényle,
R₄ est un atome d'hydrogène, un groupe alkyle en C₁ à C₃, un groupe hydroxy, un groupe acyle, un groupe alcanoyle en C₁ à C₁₆, un groupe allyloxycarbonyle, un groupe alcoxycarbonyle en C₁ à C₁₆, un groupe phényle-alcoxycarbonyle en C₁ à C₆, un groupe alkylsulfonyle en C₁ à C₃-alcoxycarbonyle en C₂ à C₄, alcoxy en C₁ à C₃₋alcoxy en C₂ à C₄-alcoxycarbonyle en C₂ à C₄ ou un groupe RₑCO-O-(R_{f}CR_{g}-O-CO,
dans lequel
Rₑ est un groupe alkyle en C₁ à C₈, cycloalkyle en C₅ à C₇, phényle ou phényl-alkyle en C₁ à C₃,
R_{f} est un atome d'hydrogène, un groupe alkyle en C₁ à C₃, un groupe cycloalkyle en C₅ à C₇ ou un groupe phényle, et
R_{g} est un atome d'hydrogène, un groupe alkyle en C₁ à C₃ ou
un groupe RₑCO-O-(R_{f}R_{g})-O- dans lequel Rₑ à Rg sont tels que définis précédemment,
R₅ est un atome d'hydrogène ou un groupe alkyle en C₁ à C₃ et
R₆ est un atome d'hydrogène, de fluor, de chlore, de brome ou d'iode,
un groupe trifluorométhyle ou hétéroaryle, un groupe alcoxy en C₁ à C₃ éventuellement substitués par 1 à 3 atomes de fluor, un groupe amino-alcoxy en C₁ à C₃, alkylamino en C₁ à C₃-alcoxy en C₂ à C₃ ou un groupe benzylamino-alcoxy en C₂ à C₃, un groupe cycloalkylèneimino-alcoxy en C₂ à C₃ comportant 4 à 7 chaînons du cycle, un groupe di-(alkyle en C₁ à C₃)-amino-alcoxy en C₂ à C₃ ou un groupe alkylmercapto en C₁ à C₃,
un groupe nitro, cyano, carboxy, alcoxycarbonyle en C₁ à C₃, amino-carbonyle, alkylaminocarbonyle en C₁ à C₃, di-(alkyle en C₁ à C₃)-amino-carbonyle, pipéridinocarbonyle ou tétrazolyle,
un groupe alkyle en C₁ à C₃-carbonylamino éventuellement substitué par un groupe alkyle en C₁ à C₃ sur l'atome d'azote,
un groupe imidazolyle ou pipérazino éventuellement substitué par un groupe alkyle en C₁ à C₃, sur le groupe imino,
un groupe alkyle en C₁ à C₄, qui peut être substitué au niveau terminal par un groupe hydroxy, alcoxy en C₁ à C₃, carboxy, alcoxy en C₁ à C₃, carbonyle, amino, alkylamino en C₁ à C₄, di-(alkyle en C₁ à C₄)-amino, phénylamino, N-phényl-alkylamino en C₁ à C₃, phényl-n-alkylamino en C₁à C₃, N-(alkyle en C₁ à C₃)-phényl-n-alkyle en C₁ à C₃-amino ou di-(phényl-n-alkyle en C₁ à C₃)-amino,
par un groupe cycloalkylèneimino comportant 4 à 7 chaînons, dans lequel
un groupe méthylène lié au groupe imino peut être substitué par un groupe carbonyle ou sulfonyle, ou
un ou deux atomes d'hydrogène peuvent être respectivement substitués par un groupe alkyle en C₁ à C₃ ou/et
respectivement, le groupe méthylène en position 4 d'un groupe cycloalkylèneimino comportant 6 ou 7 chaînons peut être substitué par un groupe carboxy, alcoxycarbonyle en C₁ à C₃, aminocarbonyle, alkylaminocarbonyle en C₁ à C₃, di-(alkyle en C₁ à C₃)-aminocarbonyle, phényl-n-alkylamino en C₁ à C₃ ou N-(alkyle en C₁ à C₃)-phényl-n-alkylamino en C₁ à C₃, ou
par un atome d'oxygène ou un atome de soufre, par un groupe sulfinyle, sulfonyle, NH ou N-(alkyle en C₁ à C₃),
par un groupe cycloalkénylèneimino comportant 5 à 7 chaînons, dans lequel la double liaison est isolée de l'atome d'azote,
par un groupe cycloalkylamino en C₄ à C₇, N-(alkyle en C₁ à C₃), cycloalkylamino en C₄ à C₇ ou cycloalkénylamino en C₅ à C₇, dans lequel la position 1 du cycle ne fait pas partie de la double liaison et dans lequel l'atome d'azote peut être substitué par un groupe alkyle en C₁ à C₃,
par un groupe alkylcarbonylamino en C₁ à C₃, N-(alkyle en C₁ à C₃)-alkylcarbonylamino en C₁ à C₃, aminocarbonyle, alkyle en C₁ à C₃-aminocarbonyle
ou di-(alkyle en C₁ à C₃)-aminocarbonyle,
ou R₆ est un groupe de formule
-N(Ra₎-CO-(CH₂)ₙ-R_{b} (II),
dans laquelle
Rₐ est un groupe alkyle en C₁ à C₃,
n est l'un des nombres 0, 1 ou 2 et
R_{b} est un groupe amino, alkylamino en C₁ à C₄, phénylamino, N-(alkyle en C₁ à C₄)-phénylamino, benzylamino, N-(alkyle en C₁ en C₄)-benzylamino ou di-(alkyle en C₁ à C₄)-amino ou un groupe cycloalkylèneimino comportant 4 à 7 chaînons, le groupe méthylène en position 4 d'un groupe cycloalkylèneimino comportant 6 ou 7 chaînons pouvant être respectivement substitué par un atome d'oxygène ou de soufre, par un groupe sulfinyle, sulfonyle, NH ou N-(alkyle en C₁ à C₃), un groupe de formule
-N(R_{c})-(CH₂)ₘ-(CO)ₒ-R_{d} (III),
dans laquelle R_{c} est un groupe alkyle en C₁ à C₃, alkylcarbonyle en C₁ à C₃, arylcarbonyle, benzylcarbonyle, alkylsulfonyle en C₁ à C₃, arylsulfonyle ou benzylsulfonyle,
m est l'un des nombres 1, 2, 3 ou 4,
o est l'un des nombres 0 ou 1 et
R_{d} qui a les significations précédemment mentionnées pour R_{b} ou représente un groupe di-(alkyle en C₁ à C₄)-amino-alkylamino en C₁ à C₃ éventuellement substitué en position 1 par un groupe alkyle en C₁ à C₃,
ou un groupe N-(alkyle en C1 à C3)-alkylsulfonylamino en C₁ à C₃,
dans lequel en outre, un groupe carboxy, amino ou imino peut être substitué par un radical pouvant être scindé *in vivo,*
ce radical étant un groupe hydroxy, un groupe acyle, un groupe alcanoyle en C₁ à C₁₆, un groupe allyloxycarbonyle, un groupe alcoxycarbonyle en C₁ à C₁₆, un groupe phényle-alcoxycarbonyle en C₁ à C₆, un groupe alkylsulfonyle en C₁ à C₃₋alcoxycarbonyle en C₂ à C₄, un groupe alcoxy en C₁ à C₃-alcoxy en C₂ à C₄₋alcoxycarbonyle en C₂ à C₄ ou un groupe RₑCO-O-(R_{f}CR_{g})-O-CO-, dans lequel
Rₑ peut être un groupe alkyle en C₁ à C₈, cycloalkyle en C₅ à C₇, phényle ou phényl-alkyle en C₁ à C₃,
R_{f} peut être un atome d'hydrogène, un groupe alkyle en C₁ à C₃, cycloalkyle en C₅ à C₇ ou phényle, et
R_{g} peut être un atome d'hydrogène, un groupe alkyle en C₁ à C₃ ou un groupe RₑCO-O-(R_{f}CR_{g})-O, dans lequel Rₑ à Rg sont tels que définis précédemment,
leurs énantiomères et/ou diastéréomères et leurs sels.

2. Indolinones substituées de formule générale I selon la revendication 1, dans lesquelles
X est un atome d'oxygène,
R₁ est un groupe alcoxy en C₁ à C₃, trifluorométhyle, di-(alkyle en C₁ à C₃)-amino, pyrrolidino ou pyrrolo,
un groupe amino ou alkylamino en C₁ à C₃, dans lequel respectivement un atome d'hydrogène aminé peut être remplacé par un groupe alkylcarbonyle en C₁ à C₃, phényle, alkylcarbonyle en C₁ à C₃, benzoyle, aminocarbonyle, alkylsulfonyle en C₁ à C₃, phénylsulfonyle, carboxy-alkyle en C₁ à C₃ ou alkyle en C₁ à C₃-oxycarbonyle alkyle en C₁ à C3, ou
un groupe phényle éventuellement substitué par un groupe alkyle en C₁ à C₃,
R₂ est un atome d'hydrogène ou un groupe alcoxy en C₁ à C₃ ou
R₁ et R₂ conjointement sont un groupe méthylènedioxyde,
R₃ est un groupe phényle,
R₄ est un atome d'hydrogène,
R₅ est un atome d'hydrogène et
R₆ est un atome d'hydrogène, de fluor, de chlore, de brome ou d'iode,
un groupe trifluorométhyle, 4-(alkyle en C₁ à C₃)-pipérazino, pyridinyle, imidazolyle, tétrazolyle, alcoxy en C₁ à C₃ ou alkylmercapto en C₁ à C₃,
un groupe nitro, cyano, carboxy ou alcoxycarbonyle en C₁ à C₃ ou un groupe alkylcarbonylamino en C₁ à C₃ éventuellement substitué au niveau d'un atome d'azote, par un groupe alkyle en C₁ à C₃,
un groupe pipéridonocarbonyle ou un groupe aminocarbonyle éventuellement substitué par un ou deux groupes alkyle en C₁ à C₃,
un groupe alkyle en C₁ à C₃ qui est substitué au niveau terminal
par un groupe amino, alkylamino en C₁ à C₄, di-(alkyle en C₁ à C₄)-aminophénylamino, N-phényl-alkylamino en C₁ à C₃, phényl-n-alkyle en C₁ à C₃-amino, N-(alkyle en C₁ à C₃)-phényl-n-alkylamino en C₁ à C₃ ou di-(péhnyl-n-alkyle en C₁ à C₃)-amino, par un groupe pyrrolidino, pipéridino, hexaméthylèneimino, morpholino, thiomorpholino, 1-oxydo-thiomorpholino ou pipérazino,
le groupe pipéridino pouvant en outre être substitué par un ou deux groupes alkyle en C₁ à C₃ ou par un groupe carboxy-alcoxycarbonyle en C₁ à C₃, aminocarbonyle, alkylaminocarbonyle en C₁ à C₃, di-(alkyle en C₁ à C₃)-aminocarbonyle ou N-(alkyle en C₁ à C₃)-phényl-n-alkylamino en C₁ à C₃,
par un groupe cycloalkylamino en C₅ à C₇ ou cycloalkénylamino en C₅ à C₇, dans lequel la position 1 du cycle ne fait pas partie de la double liaison,
par un groupe alkylcarbonylamino en C₁ à C₃, N-(alkyle en C₁ à C₃)-alkylcarbonylamino en C₁ à C₃, carboxy, alcoxycarbonyle en C₁ à C₃, aminocarbonyle, alkylaminocarbonyle en C₁ à C₃ ou di-(alkyle en C₁ à C₃)-aminocarbonyle,
un groupe alcoxy en C₁ à C₃ qui peut être substitué au niveau terminal par un groupe amino-alkylamino en C₁ à C₃ ou di-(alkyle en C₁ à C₃)-amino, un groupe de formule
-N(Rₐ)-CO-(CH₂)ₙ-R_{b} (II)
dans laquelle
Rₐ est un groupe alkyle en C₁ à C₃,
n est l'un des nombres 0, 1 ou 2 et
R_{b} est un groupe amino, alkylamino en C₁ à C₄ ou di-(alkyle en C₁ à C₄)-amino ou un groupe pyrrolidino, pipéridino, hexaméthylèneimino, morpholino, thiomorpholino, 1-oxydo-thiomorpholino ou pipérazino,
un groupe de formule
-N(Rₑ)-(CH₂)ₘ-(CO)ₒ-R_{d} (III),
dans laquelle R_{c} est un groupe alkyle en C₁ à C₃, alkylcarbonyle en C₁ à C₃ ou alkylsulfonyle en C₁ à C₃,
m est l'un des nombres 1, 2, 3 ou 4,
o est l'un des nombres 0 ou 1, et
R_{d} qui possède la signification mentionnée précédemment pour R_{b} ou est un groupe di-(alkyle en C₁ à C₄)-amino-alkylamino en C₁ à C₃ éventuellement substitué en position 1 par un groupe alkylamino en C₁ à C₃ ou un groupe N-(alkyle en C₁ à C₃)-alkylsulfonylamino en C₁ à C₃, leurs énantiomères et diastéréo-isomères et leurs sels.

3. Indolinones substituées de formule générale I selon la revendication 1, dans lesquelles
X est un atome d'oxygène
R₁ est un groupe méthoxy, éthoxy, trifluorométhyle, phényle, méthylphényle, diméthylamino, pyrrolidino ou pyrrolo,
un groupe amino qui peut être substitué par un groupe méthyle, carboxyméthyle, méthoxycarbonylméthyle, acétyle, phénylacétyle, benzoyle, méthanesulfonyle, benzolsulfonyle ou aminocarbonyle,
R₂ est un atome d'hydrogène, un groupe méthoxy ou éthoxy ou
R₁ et R₂ conjointement sont un groupe méthylènedioxy,
R₃ est un groupe phényle,
R₄ est un atome d'hydrogène,
R₅ est un atome d'hydrogène et
R₆ est un atome d'hydrogène, de fluor, de chlore, de brome ou d'iode,
un groupe méthyle, trifluorométhyle, méthoxy, éthoxy, méthylmercapto, cyano, carboxy, méthoxycarbonyle, éthoxycarbonyle, aminocarbonyle, diméthylaminocarbonyle, pipéridinocarbonyle, nitro, 4-méthyl-pipérazino, imidazolyle, pyridinyle ou tétrazolyle,
un groupe éthyloxy ou n-propyloxy substitué au niveau terminal par un groupe diméthylamino,
un groupe méthyle ou éthyle substitué par un groupe carboxy, méthoxycarbonyle, éthoxycarbonyle, aminocarbonyle ou diméthylaminocarbonyle,
un groupe alkyle en C₁ à C₃ qui est substitué au niveau terminal
par un groupe amino, alkylamino en C₁ à C₄, cyclohexylamino, benzylamino ou phénylamino, dans lesquels respectivement un atome d'hydrogène de l'atome amino d'azote peut être remplacé par un groupe alkyle en C₁ à C₃, benzyle, acétyle ou diméthylaminocarbonyle,
par un groupe pipéridino éventuellement substitué par un ou deux groupes méthyle, par un groupe pipéridino substitué par un groupe carboxy, méthoxycarbonyle, éthoxycarbonyle ou diméthylaminocarbonyle,
par un groupe pyrrolidino, 3,4-déshydro-pipéridino, hexaméthylène-imino, morpholino, thiomorpholino, 1-oxo-thio-morpholino ou pipérazino,
un groupe alkylamino en C₁ à C₃, dans lequel l'atome d'hydrogène de l'atome d'azote est substitué
par un groupe éthyle ou n-propyle qui est respectivement substitué au niveau terminal par un groupe diméthylamino,
par un groupe alcanoyle en C₂ à C₃ qui peut être substitué par un groupe amino, alkylamino en C₁ à C₃, di-(alkyle en C₁ à C₃), amino, pyrrolidino, pipéridino, morpholino ou pipérazino,
par un groupe aminocarbonyle, méthylaminocarbonyle, diméthylaminocarbonyle, pipéridinocarbonyle ou méthanesulfonyle,
et en outre, la partie alkyle en C₁ à C₃ du groupe alkylamino en C₁ à C₃ peut être substituée
par un groupe aminocarbonyle,
par un groupe alkylaminocarbonyle en C₁ à C₃ ou un groupe di-(alkyle en C₁ à C₃)-aminocarbonyle dans lesquels une partie alkyle en C₂ à C₃ peut en outre être substituée au niveau terminal par un groupe diméthylamino,
par un groupe pyrrolidinocarbonyle, pipéridinocarbonyle, morpholinocarbonyle ou pipérazinocarbonyle,
la partie alkyle en C₂ à C₃ du groupe alkylamino en C₁ à C₃ mentionné précédemment pouvant en outre être substituée au niveau terminal par un groupe amino, alkylamino en C₁ à C₃, di-(alkyle en C₁ à C₃)-amino, pyrrolidino, pipéridino, morpholino ou pipérazino,
leurs énantiomères et/ou leurs diastéréomères et leurs sels.

4. Indolinones substituées de formule générale I selon la revendication 2 ou 4, dans lesquelles
X, R₁ et R₃ à R₆ sont tels que définis à la revendication 2 et
R₂ est un atome d'hydrogène,
leurs énantiomères et/ou diastéréomères et leurs sels.

5. Indolinones substituées de formule générale I selon la revendication 2 ou 3, dans lesquelles
X et R₃ à R₆ sont tels que définis à la revendication 2,
R₁ et R₂ qui peuvent être identiques ou différents, sont respectivement un groupe alcoxy en C₁ à C₃,
leurs énantiomères et/ou leurs diastéréomères et leurs sels.

6. Indolinones substituées de formule générale I selon la revendication 1, dans lesquelles
X est un atome d'oxygène
R₁ est un groupe amino, méthoxy ou éthoxy,
R₂ est un atome d'hydrogène ou un groupe méthoxy ou éthoxy en position 5,
R₃ est un groupe phényle,
R₄ et R₅ sont respectivement un atome d'hydrogène et
R₆ est un groupe méthyle ou éthyle substitué par un groupe méthylamino, éthylamino, pipéridino ou 4-(diméthylaminocarbonyl)-pipéridino, l'atome d'azote du groupe méthylamino et éthylamino étant substitué par un groupe méthyle ou benzyle, un groupe N-diméthylaminoéthylcarbonyl-N-méthyl-amino ou un groupe N-acétyl-N-(alkyle en C₂ à C₃)-amino, dans lequel la partie alkyle en C₂ à C₃ est substituée respectivement au niveau terminal par un groupe diméthylamino,
et leurs sels.

7. Indolinones substituées suivantes de formule générale I selon la revendication 1 :
(a) 3-(Z)-(1-[4-(pipéridine-1-yl-méthyl)-anilino)-1-phényl-méthylidène)-5,6-diméthoxy-2-indolinone,
(b) 3-(Z)-(1-{4-[(N-benzyl-N-méthyl-amino)-méthyl]-anilino}-1-phényl-méthylidène)-5,6-diméthoxy-2-indolinone
(c) 3-(Z)- {1-(4-(diméthylamino-méthyl)-anilino]-1-phényl-méthylidène}-5,6-diméthoxy-2-indolinone,
(d) 3-(Z)-{1-[4-(N-diméthylaminométhylcarbonyl-N-méthyl-amino)-anilino]-1-phényl-méthylidène)-5,6-diméthoxy-2-indolinone,
(e) 3-(Z)-(1-(4-[2-(4-diméthylcarboxamide-pipéridine-1-yl)-éthyl]-anilino}-1-phényl-méthylidène)-5,6-diméthoxy-2-indolinone,
(f) 6-amino-3-(Z)-{1-[4-(pipéridine-1-yl-méthyl)-anilino]-1-phényl-méthylidène)-2-indolinone,
(g) 3-(Z)-(1-{4-[N-acétyl-N-(2-diméthylamino-éthyl)-amino]-anilino)-1-phényl-méthylidène)-5,6-diméthoxy-2-indolinone,
(h) 3-(Z)-(1-(4-[N-acétyl-N-(3-diméthylamino-propyl)-amino}-anilino)-1-phényl-méthylidène)-5,6-diméthoxy-2-indolinone,
et leurs sels.

8. Sels physiologiquement acceptables des composés selon les revendications 1 à 7.

9. Médicament contenant un composé selon au moins l'une quelconque des revendications 1 à 7, ou un sel selon la revendication 8, en outre éventuellement un ou plusieurs véhicules et/ou diluants.

10. Utilisation d'un composé selon au moins l'une quelconque des revendications 1 à 7, ou un sel selon la revendication 8 pour la fabrication d'un médicament qui est approprié pour le traitement des proliférations cellulaires excessives ou anormales.

11. Procédé pour la fabrication d'un médicament selon la revendication 9, **caractérisé en ce que**, de manière non chimique, un composé selon au moins l'une quelconque des revendications 1 à 7 ou un sel selon la revendication 8 est intégré dans un ou plusieurs véhicules et/ou diluants inertes.

12. Procédé pour la fabrication des composés selon les revendications 1 à 8,
**caractérisé en ce que**
un composé de formule générale 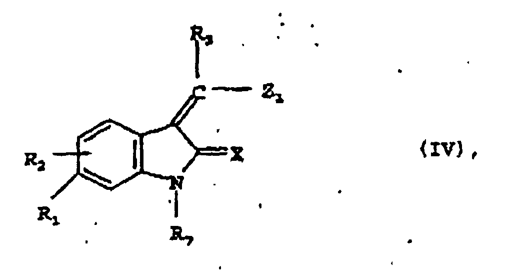
dans laquelle
X et R₁ à R₃ sont tels que définis dans les revendications 1 à 7,
R₇ est un atome d'hydrogène, un groupe protecteur de l'atome d'azote du groupe lactame ou une liaison à une phase solide et
Z₁ est un atome d'halogène, un groupe hydroxy, alcoxy ou arylalcoxy,
est transformé
avec une amine de formule générale 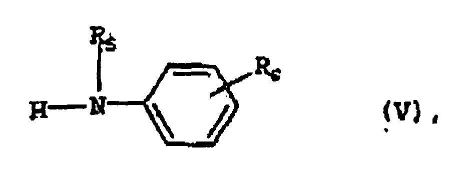
dans laquelle
R₅ et R₆ sont tels que définis dans les revendications 1 à 7, transformés et si nécessaire, un groupe protecteur utilisé pour l'atome d'azote du groupe lactame ou un composé obtenu de cette façon est ensuite séparé d'une phase solide, et éventuellement ensuite, un composé de formule générale I obtenu de cette façon, qui contient un groupe alcoxycarbonyle, est transformé au moyen d'une hydrolyse en un composé carboxy correspondant, ou
un composé de formule générale I obtenu de cette façon, qui contient un groupe amino ou alkylamino, est transformé par une alkylation ou une alkylation réductrice en un composé alkylamino, dialkylamino ou pyrrolidino correspondant, ou
un composé de formule générale I obtenu de cette façon, qui contient un groupe amino ou alkylamino, est transformé par acylation en un composé acyle correspondant ou
un composé de formule générale I obtenu de cette façon, qui contient un groupe amino ou alkylamino, est transformé par sulfonation en un composé sulfonyle correspondant, ou
un composé de formule générale I obtenu de cette façon, qui contient un groupe amino, est transformé par une réaction de condensation, en un composé pyrrolo correspondant, ou
un composé de formule générale I obtenu de cette façon, qui contient un groupe carboxy, est transformé par estérification ou amidation en un composé ester ou aminocarbonyle correspondant, ou
un composé de formule générale I obtenu de cette façon, qui contient un groupe cyano, est transformé par réduction en un composé aminométhyle correspondant, ou
un composé de formule générale I obtenu de cette façon, qui contient un groupe amino ou alkylamino, est transformé par réaction avec de l'acide cyanhydrique ou un isocyanate, en un composé uréido correspondant, et
si nécessaire, un groupe protecteur utilisé pendant les transformations pour la protection des groupes réactifs est séparé, ou
si on le souhaite, par la suite, un composé de formule générale I obtenu de cette façon, est séparé en ses stéréo-isomères, ou
un composé de formule générale I obtenu de cette façon, est transformé en ses sels, en particulier pour l'utilisation pharmaceutique, en ses sels physiologiquement acceptables, avec un acide ou une base anorganique ou organique.

13. Procédé selon la revendication 12, **caractérisé en ce que**, pour la fabrication d'un composé de formule générale I, dans laquelle R₁ est un groupe amino, un composé de formule générale 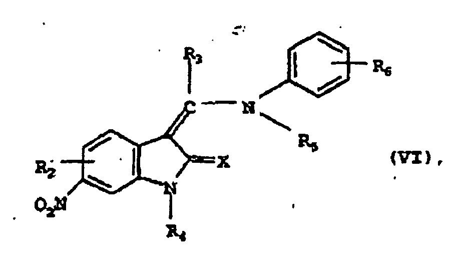
est réduit.

14. Procédé selon la revendication 12, **caractérisé en ce que** pour la fabrication d'un composé de formule générale I, dans laquelle R₁ et/ou R₂ sont l'un des groupes sulfinyle ou sulfonyle substitués mentionnés dans les revendications 1 à 7, un composé de formule générale 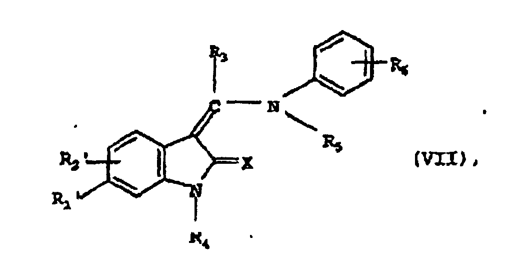
dans laquelle
R₃ à R₆ sont tels que définis dans les revendications 1 à 7, et
l'un des groupes R₁' et R₂' est l'un des groupes mercapto ou sulfinyle substitués mentionnés dans les revendications 1 à 6 pour R₁ et R₂ et l'autre a les significations mentionnées dans les revendications 1 à 7 pour R₁ ou R₂, à l'exception des groupes mercapto ou sulfinyle, ou les deux groupes R₁' et R₂' sont l'un des groupes mercapto ou sulfinyle substitués, mentionnés pour R₁ et R₂ dans les revendications 1 à 6,
est oxydé.

## Claims

1. Substituted indolinones of general formula 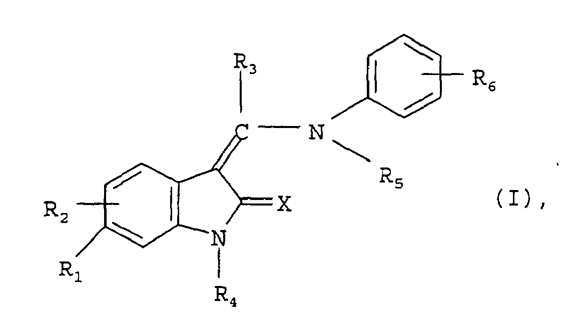
X denotes an oxygen or sulphur atom,
R₁ denotes a C₂₋₃-alkenyl, C₂₋₃-alkynyl, aryl, aryl-C₁₋₃-alkyl, heteroaryl, heteroaryl-C₁₋₃-alkyl, trifluoromethyl or cyano group,
a hydroxy, C₁₋₃-alkoxy, hydroxy-C₁₋₃-alkyl, C₁₋₃-alkoxy-C₁₋₃-alkyl, aryloxy or heteroaryloxy group,
a mercapto, C₁₋₃-alkylsulphenyl, phenylsulphenyl, benzylsulphenyl, C₁₋₃-alkylsulphinyl, phenylsulphinyl, benzylsulphinyl, C₁₋₃-alkylsulphonyl, phenylsulphonyl, benzylsulphonyl, sulpho, C₁₋₃-alkoxysulphonyl, phenoxysulphonyl or benzyloxysulphonyl group,
an amino, C₁₋₃-alkylamino, di-(C₁₋₃-alkyl)-amino, hydroxycarbonyl-C₁₋₃-alkylamino, N-(C₁₋₃-alkyl) hydroxycarbonyl-C₁₋₃-alkylamino, C₁₋₃-alkoxycarbonyl-C₁₋₃-alkylamino, N-(C_{1 -3}-alkyl)-C₁₋₃-alkoxycarbonyl-C₁₋₃-alkylamino, phenylamino, N-phenyl-C₁₋₃-alkylamino, N,N-diphenylamino, benzylamino, N-benzyl-C₁₋₃-alkylamino, N,N-dibenzylamino, C₁₋₃-alkylcarbonylamino, benzoylamino or benzylcarbonylamino group or an N-(C₁₋₃-alkyl)-C₁₋₃-alkylcarbonylamino group wherein the two alkyl groups may be replaced by a C₂₋₅-n-alkylene bridge or wherein one or both alkyl groups may be replaced by a phenyl or benzyl group,
a C₁₋₃-alkylsulphonylamino, phenylsulphonylamino or benzylsulphonylamino group or an N-(C₁₋₃-alkyl)-C₁₋₃-alkylsulphonylamino group wherein the two alkyl groups may be replaced by a C₂₋₅-n-alkylene bridge or wherein one or both alkyl groups may be replaced by a phenyl or benzyl group,
an aminosulphonyl, C₁₋₃-akylaminosulphonyl, phenylaminosulphonyl, benzylaminosulphonyl, di-(C₁₋₃-alkyl)-aminosulphonyl, N,N-diphenyl-aminosulphonyl or N,N-dibenzyl-aminosulphonyl group,
a phosphono, (C₁₋₃-alkoxy)PO(H), (C₁₋₃-alkoxy)PO(C₁₋₃-alkyl), (C₁₋₃-alkoxy)PO(OH), di-(C₁₋₃-alkoxy)-PO or (C₂₋₄-n-alkylenedioxy)-PO group,
a ureido group optionally mono-, di- or trisubstituted by C₁₋₃-alkyl groups,
a 4- to 7-membered cycloalkyleneimino or cycloalkyleneiminosulphonyl group, wherein in each case the methylene group in the 4 position of a 6- or 7-membered cycloalkyleneimino group may be replaced by an oxygen or sulphur atom, by a sulphinyl, sulphonyl, -NH or-N(C₁₋₃-alkyl) group,
R₂ denotes a hydrogen, fluorine, chlorine, bromine or iodine atom,
a C₁₋₆-alkyl or trifluoromethyl group,
a hydroxy, C₁₋₃-alkoxy, mercapto, C₁₋₃-alkylsulphenyl, C₁₋₃-alkylsulphinyl, C₁₋₃-alkylsulphonyl, sulpho, C₁₋₃-alkoxysulphonyl, aminosulphonyl, C₁₋₃-alkylaminosulphonyl or di-(C₁₋₃-alkyl)-aminosulphonyl group,
a nitro, amino, C₁₋₃-alkylamino or di-(C₁₋₃-alkyl)-amino group,
a C₁₋₃-alkylcarbonyl, cyano, carboxy, C₁₋₃-alkoxycarbonyl, aminocarbonyl, C₁₋₃-alkylaminocarbonyl or di-(C₁₋₃-alkyl)-aminocarbonyl group,
a phosphono, (C₁₋₃-alkoxy)PO(H), (C₁₋₃-alkoxy)PO(C₁₋₃-alkyl), (C₁₋₃-alkoxy)PO(OH) or di-(C₁₋₃-alkoxy)-PO group,
a 4- to 7-membered cycloalkyleneimino, cycloalkyleneiminocarbonyl or cycloalkyleneiminosulphonyl group, wherein in each case the methylene group in the 4 position of a 6- or 7-membered cycloalkyleneimino group may be replaced by an oxygen or sulphur atom, by a sulphinyl, sulphonyl, -NH or -N(C₁₋₃-alkyl) group; or
R₁ and R₂ together denote a methylenedioxy, ethylenedioxy, n-propylene, n-butylene or 1,4-butadienylene group,
R₃ denotes a phenyl group,
R₄ denotes a hydrogen atom, a C₁₋₃-alkyl group, a hydroxy group, an acyl group, a C₁₋₁₆-alkanoyl group, an allyloxycarbonyl group, a C₁₋₁₆₋alkoxycarbonyl group, a phenyl-C₁₋₆-alkoxycarbonyl group, a C₁₋₃₋alkylsulphonyl-C₂₋₄-alkoxycarbonyl, C₁₋₃-alkoxy-C₂₋₄-alkoxy-C₂₋₄₋alkoxycarbonyl or RₑCO-O-(R_{f}CR_{g})-O-CO group wherein
Rₑ denotes a C₁₋₈-alkyl, C₅₋₇-cycloalkyl, phenyl or phenyl-C₁₋₃-alkyl group,
R_{f} denotes a hydrogen atom, a C₁₋₃-alkyl, C₅₋₇-cycloalkyl or phenyl group and
R_{g} denotes a hydrogen atom, a C₁₋₃-alkyl or RₑCO-O-(R_{f}CR_{g}-O- group wherein Rₑ to R_{g} are as hereinbefore defined,
R₅ denotes a hydrogen atom or a C₁₋₃-alkyl group and
R₆ denotes a hydrogen, fluorine, chlorine, bromine or iodine atom,
a trifluoromethyl or heteroaryl group, a C₁₋₃-alkoxy group optionally substituted by 1 to 3 fluorine atoms, an amino-C₁₋₃-alkoxy, C₁₋₃-alkylamino-C₂₋₃-alkoxy or benzylamino-C₂₋₃-alkoxy group, a cycloalkyleneimino-C₂₋₃-alkoxy group with 4 to 7 ring members, a di-(C₁₋₃-alkyl)-amino-C₂₋₃-alkoxy or C₁₋₃-alkylmercapto group,
a nitro, cyano, carboxy, C₁₋₃-alkoxycarbonyl, aminocarbonyl, C₁₋₃-alkylaminocarbonyl, di-(C₁₋₃-alkyl)-aminocarbonyl, piperidinocarbonyl or tetrazolyl group,
a C₁₋₃-alkylcarbonylamino group optionally substituted at the nitrogen atom by a C₁₋₃-alkyl group,
an imidazolyl or piperazino group optionally substituted at the imino group by a C₁₋₃-alkyl group,
a C₁₋₄-alkyl group, which may be terminally substituted
by a hydroxy, C₁₋₃-alkoxy, carboxy, C₁₋₃-alkoxycarbonyl, amino, C₁₋₄-alkylamino, di-(C₁₋₄-alkyl)-amino, phenylamino, N-phenyl-C₁₋₃₋alkylamino, phenyl-n-C₁₋₃-alkylamino, N-(C₁₋₃-alkyl)-phenyl-n-C₁₋₃-alkylamino or di-(phenyl-n-C₁₋₃-alkyl)-amino group,
by a 4- to 7-membered cycloalkyleneimino group wherein
a methylene group linked to the imino group may be replaced by a carbonyl or sulphonyl group or
one or two hydrogen atoms may each be replaced by a C₁₋₃-alkyl group and/or
in each case the methylene group in the 4 position of a 6- or 7- membered cycloalkyleneimino group may be substituted by a carboxy, C₁₋₃-alkoxycarbonyl, aminocarbonyl, C₁₋₃-alkylaminocarbonyl, di-(C₁₋₃-alkyl)-aminocarbonyl, phenyl-n-C₁₋₃-alkylamino or N-(C₁₋₃-alkyl) phenyl-n-C₁₋₃-alkylamino group or
may be replaced by an oxygen or sulphur atom, by a sulphinyl, sulphonyl, -NH or -N(C₁₋₃-alkyl) group,
by a 5- to 7-membered cycloalkenyleneimino group wherein the double bond is isolated from the nitrogen atom,
by a C₄₋₇-cycloalkylamino, N-(C₁₋₃-alkyl)-C₄₋₇-cycloalkylamino or C₅₋₇-cycloalkenylamino group wherein position 1 of the ring is not involved in the double bond and wherein the nitrogen atom may be substituted by a C₁₋₃-alkyl group,
by a C₁₋₃-alkylcarbonylamino, N-(C₁₋₃-alkyl)-C₁₋₃-alkylcarbonylamino, aminocarbonyl, C₁₋₃-alkylaminocarbonyl or di-(C₁₋₃-alkyl)-aminocarbonyl group,
or R₆ denotes a group of formula
-N(Rₐ)-CO-(CH₂)ₙ-R_{b} (II),
wherein
Rₐ denotes a C₁₋₃-alkyl group,
n one of the numbers 0, 1 or 2 and
R_{b} denotes an amino, C₁₋₄-alkylamino, phenylamino, N-(C₁₋₄-alkyl)-phenylamino, benzylamino, N-(C₁₋₄-alkyl)-benzylamino or di-(C₁₋₄-alkyl)-amino group or a 4-to 7-membered cycloalkyleneimino group, wherein in each case the methylene group in the 4 position of a 6- or 7-membered cycloalkyleneimino group may be replaced by an oxygen or sulphur atom, by a sulphinyl, sulphonyl, -NH or -N(C₁₋₃-alkyl) group,
a group of formula
-N(R_{c})-(CH₂)m-(CO)ₒ-R_{d} (III),
wherein
R_{c} denotes a C₁₋₃-alkyl; C₁₋₃-alkylcarbonyl, arylcarbonyl, benzylcarbonyl, C₁₋₃-alkylsulphonyl, arylsulphonyl or benzylsulphonyl group,
m denotes one of the numbers 1, 2, 3 or 4,
o denotes one of the numbers 0 or 1 and
R_{d} has the meanings given for R_{b} hereinbefore or denotes a di-(C₁₋₄-alkyl)-amino-C₁₋₃-alkylamino group optionally substituted in the 1 position by a C₁₋₃-alkyl group,
or an N-(C₁₋₃-alkyl)-C₁₋₃-alkylsulphonylamino group,
whilst additionally any carboxy, amino or imino group present may be substituted by a group which can be cleaved *in vivo,*
where this group may be a hydroxy group, an acyl group, a C₁₋₁₆₋alkanoyl group, an allyloxycarbonyl group, a C₁₋₁₆-alkoxycarbonyl group, a phenyl-C₁₋₆-alkoxycarbonyl group, a C₁₋₃-alkylsulphonyl-C₂₋₄₋alkoxycarbonyl, C₁₋₃-alkoxy-C₂₋₄-alkoxy-C₂₋₄-alkoxycarbonyl or RₑCOO-(R_{f}CR_{g})-O-CO group wherein
Rₑ denotes a C₁₋₈-akyl, C₅₋₇-cycloalkyl, phenyl or phenyl-C₁₋₃₋alkyl group,
R_{f} denotes a hydrogen atom, a C₁₋₃-alkyl, C₅₋₇-cycloalkyl or phenyl group and
R_{g} denotes a hydrogen atom, a C₁₋₃-alkyl or RₑCO-O-(R_{f}CR_{g})-O- group wherein Rₑ to Rg are as hereinbefore defined,
the enantiomers and/or diastereomers and the salts thereof.

2. Substituted indolinones of general formula I according to claim 1, wherein
X denotes an oxygen atom,
R₁ denotes a C₁₋₃-alkoxy, trifluoromethyl, di-(C₁₋₃-alkyl)-amino, pyrrolidino or pyrrolo group,
an amino or C₁₋₃-alkylamino group wherein an amino-hydrogen atom may be replaced by a C₁₋₃-alkylcarbonyl, phenyl-C₁₋₃-alkylcarbonyl, benzoyl, aminocarbonyl, C₁₋₃-alkylsulphonyl, phenylsulphonyl, carboxy-C₁₋₃-alkyl or C₁₋₃-alkyloxycarbonyl-C₁₋₃-alkyl group, or
a phenyl group optionally substituted by a C₁₋₃-alkyl group,
R₂ denotes a hydrogen atom or a C₁₋₃-alkoxy group or
R₁ and R₂ together denote a methylenedioxy group,
R₃ denotes a phenyl group,
R₄ denotes a hydrogen atom,
R₅ denotes a hydrogen atom and
R₆ denotes a hydrogen, fluorine, chlorine, bromine or iodine atom,
a trifluoromethyl, 4-(C₁₋₃-alkyl)-piperazino, pyridinyl, imidazolyl, tetrazolyl, C₁₋₃-alkoxy or C₁₋₃-alkylmercapto group,
a nitro, cyano, carboxy or C₁₋₃-alkyloxycarbonyl group or a C₁₋₃-alkylcarbonylamino group optionally substituted at the nitrogen atom by a C₁₋₃-alkyl group,
a piperidinocarbonyl group or an aminocarbonyl group optionally substituted by one or two C₁₋₃-alkyl groups,
a C₁₋₃-alkyl group, which may be terminally substituted
by an amino, C₁₋₄-alkylamino, di-(C₁₋₄-alkyl)-amino, phenylamino, N-phenyl-C₁₋₃-alkylamino, phenyl-n-C₁₋₃-alkylamino, N-(C₁₋₃-alkyl)-phenyl-n-C₁₋₃-alkylamino or di-(phenyl-n-C₁₋₃-alkyl)-amino group, by a pyrrolidino, piperidino, hexamethyleneimino, morpholino, thiomorpholino, 1-oxido-thiomorpholino or piperazino group,
whilst the piperidino group may additionally be substituted by one or two C₁₋₃-alkyl groups or by a carboxy, C₁₋₃-alkoxycarbonyl, aminocarbonyl, C₁₋₃-alkylaminocarbonyl- di-(C₁₋₃-alkyl)-aminocarbonyl or N-(C₁₋₃-alkyl)-phenyl-n-C₁₋₃-alkylamino group,
by a C₅₋₇-cycloalkylamino or C₅₋₇-cycloalkenylamino group wherein position 1 of the ring is not involved in the double bond,
by a C₁₋₃-alkylcarbonylamino, N-(C₁₋₃-alkyl)-C₁₋₃-alkylcarbonylamino, carboxy, C₁₋₃-alkoxycarbonyl, aminocarbonyl, C₁₋₃-alkylaminocarbonyl or di-(C₁₋₃-alkyl)-aminocarbonyl group,
a C₁₋₃-alkoxy group, which is terminally substituted by an amino, C₁₋₃-alkylamino or di-(C₁₋₃-alkyl)-amino group,
a group of formula
-N(Rₐ)-CO-(CH₂)ₙ-R_{b} (II),
wherein
Rₐ denotes a C₁₋₃-alkyl group,
n denotes one of the numbers 0, 1 or 2 and
R_{b} denotes an amino, C₁₋₄-alkylamino or di-(C₁₋₄-alkyl)-amino group or a pyrrolidino, piperidino, hexamethyleneimino, morpholino, thiomorpholino, 1-oxido-thiomorpholino or piperazino group,
a group of formula
-N(R_{c})-(CH₂)ₘ-(CO)ₒ-R_{d} (III),
wherein
R_{c} denotes a C₁₋₃-alkyl, C₁₋₃-alkylcarbonyl or C₁₋₃-alkylsulphonyl group,
m denotes one of the numbers 1, 2, 3 or 4,
o denotes one of the numbers 0 or 1 and
R_{d} has the meanings given for R_{b} hereinbefore or denotes a di-(C₁₋₄-alkyl)-amino-C₁₋₃-alkylamino group optionally substituted in the 1 position by a C₁₋₃-alkyl group,
or an N-(C₁₋₃-alkyl)-C₁₋₃-alkylsulphonylamino group,
the enantiomers and/or diastereomers and the salts thereof.

3. Substituted indolinones of general formula I according to claim 1, wherein
X denotes an oxygen atom,
R₁ denotes a methoxy, ethoxy, trifluoromethyl, phenyl, methylphenyl, dimethylamino, pyrrolidino or pyrrolo group,
an amino group which may be substituted by a methyl, carboxymethyl, methoxycarbonylmethyl, acetyl, phenylacetyl, benzoyl, methanesulphonyl, benzenesulphonyl or aminocarbonyl group,
R₂ denotes a hydrogen atom, a methoxy or ethoxy group or
R₁ and R₂ together denote a methylenedioxy group,
R₃ denotes a phenyl group,
R₄ denotes a hydrogen atom,
R₅ denotes a hydrogen atom and
R₆ denotes a hydrogen, fluorine, chlorine, bromine or iodine atom,
a methyl, trifluoromethyl, methoxy, ethoxy, methylmercapto, cyano, carboxy, methoxycarbonyl, ethoxycarbonyl, aminocarbonyl, dimethylaminocarbonyl, piperidinocarbonyl, nitro, 4-methyl-piperazino, imidazolyl, pyridinyl or tetrazolyl group,
an ethyloxy or n-propyloxy group terminally substituted by a dimethylamino group,
a methyl or ethyl group substituted by a carboxy, methoxycarbonyl, ethoxycarbonyl, aminocarbonyl or dimethylaminocarbonyl group,
a C₁₋₃-alkyl group, which may be terminally substituted
by an amino, C₁₋₄-alkylamino, cyclohexylamino, benzylamino or phenylamino group wherein a hydrogen atom of the amino-nitrogen atom may be replaced in each case by a C₁₋₃-alkyl, benzyl, acetyl or dimethylaminocarbonyl group,
by a piperidino group optionally substituted by one or two methyl groups,
by a piperidino group substituted by a carboxy, methoxycarbonyl, ethoxycarbonyl or dimethylaminocarbonyl group,
by a pyrrolidino, 3,4-dehydro-piperidino, hexamethyleneimino, morpholino, thiomorpholino, 1-oxo-thiomorpholino or piperazino group,
a C₁₋₃-alkylamino group wherein the hydrogen atom of the amino-nitrogen atom is replaced
by an ethyl or n-propyl group, each of which is terminally substituted by a dimethylamino group,
by a C₂₋₃-alkanoyl group which may be substituted in the 2 or 3 position by an amino, C₁₋₃-alkylamino, di-(C₁₋₃-alkyl)-amino, pyrrolidino, piperidino, morpholino or piperazino group,
by an aminocarbonyl, methylaminocarbonyl, dimethylaminocarbonyl, piperidinocarbonyl or methanesulphonyl group,
and additionally the C₁₋₃-alkyl moiety of the C₁₋₃-alkylamino group may be substituted
by an aminocarbonyl group,
by a C₁₋₃-alkylaminocarbonyl or di-(C₁₋₃-alkyl)-aminocarbonyl group wherein a C₂₋₃-alkyl moiety may additionally be terminally substituted by a dimethylamino group,
by a pyrrolidinocarbonyl, piperidinocarbonyl, morpholinocarbonyl or piperazinocarbonyl group,
whilst the C₂₋₃-alkyl moiety of the abovementioned C₁₋₃-alkylamino group may also be terminally substituted by an amino, C₁₋₃-alkylamino, di-(C₁₋₃-alkyl)-amino, pyrrolidino, piperidino, morpholino or piperazino group,
the enantiomers and/or diastereomers and the salts thereof.

4. Substituted indolinones of general formula I according to claim 2 or 3, wherein
X, R₁ and R₃ to R₆ are as defined in claim 2 and
R₂ denotes a hydrogen atom,
the enantiomers and/or diastereomers and the salts thereof.

5. Substituted indolinones of general formula I according to claim 2 or 3, wherein
X and R₃ to R₆ are as defined in claim 2,
R₁ and R₂, which may be identical or different, each denote a C₁₋₃-alkoxy group,
the enantiomers and/or diastereomers and the salts thereof.

6. Substituted indolinones of general formula I according to claim 1, wherein
X denotes an oxygen atom,
R₁ denotes an amino, methoxy or ethoxy group,
R₂ denotes a hydrogen atom or in position 5 a methoxy or ethoxy group,
R₃ denotes a phenyl group,
R₄ and R₅ each denote a hydrogen atom and
R₆ denotes a methyl or ethyl group substituted by a methylamino, ethylamino, piperidino or 4-(dimethylaminocarbonyl)-piperidino group, wherein the amino-hydrogen atom of the methylamino- and ethylamino group is replaced by a methyl or benzyl group, an N-dimethylaminomethylcarbonyl-N-methylamino group or an N-acetyl-N-(C₂₋₃-alkyl)-amino group wherein the C₂₋₃-alkyl moiety in each case is terminally substituted by a dimethylamino group,
and the salts thereof.

7. The following substituted indolinones of general formula I according to claim 1:
(a) 3-(Z)-{1-[4-(piperidin-1-yl-methyl)-anilino]-1-phenyl-methylidene)-5,6-dimethoxy-2-indolinone,
(b) 3-(Z)-(1-{4-[(N-benzyl-N-methyl-amino)-methyl]-anilino}-1-phenyl-methylidene)-5,6-dimethoxy-2-indolinone,
(c) 3-(Z)-{1-(4-(dimethylamino-methyl)-anilino]-1-phenyl-methylidene}-5,6-dimethoxy-2-indolinone,
(d) 3-(Z)-{1-[4-(N-dimethylaminomethylcarbonyl-N-methyl-amino)-anilino]-1-phenyl-methylidene}-5,6-dimethoxy-2-indolinone,
(e) 3-(Z)-(1-(4-[2-(4-dimethylcarboxamide-piperidin-1-yl)-ethyl]-anilino)-1-phenyl-methylidene)-5,6-dimethoxy-2-indolinone,
(f) 6-amino-3-(Z)-{1-[4-(piperidin-1-yl-methyl)-anilino]-1-phenyl-methylidene}-2-indolinone,
(g) 3-(Z)-(1-{4-[N-acetyl-N-(2-dimethylamino-ethyl)-amino]-anilino}-1-phenyl-methylidene)-5, 6-dimethoxy-2-indolinone,
(h) 3-(Z)-(1-{4-[N-acetyl-N-(3-dimethylamino-propyl)-amino]-anilino}-1-phenyl-methylidene)-5,6-dimethoxy-2-indolinone
and the salts thereof.

8. Physiologically acceptable salts of the compounds according to claim 1 to 7.

9. Pharmaceutical compositions containing a compound according to at least one of claims 1 to 7 or a salt according to claim 8 optionally together with one or more inert carriers and/or diluents.

10. Use of a compound according to at least one of claims 1 to 7 or a salt according to claim 8 for preparing a pharmaceutical composition which is suitable for treating excessive or anomalous cell proliferation.

11. Process for preparing a pharmaceutical composition according to claim 9, **characterised in that** a compound according to at least one of claims 1 to 7 or a salt according to claim 8 is incorporated in one or more inert carriers and/or diluents by a non-chemical method.

12. Process for preparing the compounds according to claims 1 to 8, **characterised in that**
a compound of general formula 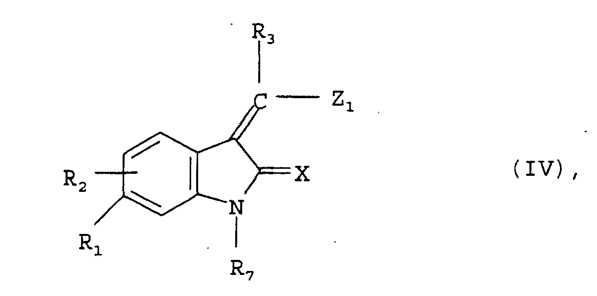
wherein
X and R₁ to R₃ are defined as in claims 1 to 7,
R₇ denotes a hydrogen atom, a protecting group for the nitrogen atom of the lactam group or a bond to a solid phase and
Z₁ denotes a halogen atom, a hydroxy, alkoxy or arylalkoxy group,
is reacted with an amine of general formula 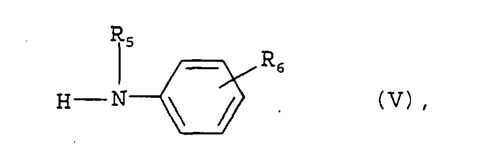
wherein
R₅ and R₆ are defined as in claims 1 to 7, and if necessary any protecting group used for the nitrogen atom of the lactam group is cleaved or a compound thus obtained is cleaved from a solid phase, and
subsequently, if desired, a compound of general formula I thus obtained which contains an alkoxycarbonyl group is converted by hydrolysis into a corresponding carboxy compound or
a compound of general formula I thus obtained which contains an amino or alkylamino group is converted by alkylation or reductive alkylation into a corresponding alkylamino, dialkylamino or pyrrolidino compound or
a compound of general formula I thus obtained which contains an amino or alkylamino group is converted by acylation into a corresponding acyl compound or
a compound of general formula I thus obtained which contains an amino or alkylamino group is converted by sulphonation into a corresponding sulphonyl compound or
a compound of general formula I thus obtained which contains an amino group is converted by a condensation reaction into a corresponding pyrrolo compound or
a compound of general formula I thus obtained which contains a carboxy group is converted by esterification or amidation into a corresponding ester or aminocarbonyl compound or
a compound of general formula I thus obtained which contains a cyano group, is converted by reduction into a corresponding aminomethyl compound or
a compound of general formula I thus obtained which contains an amino or alkylamino group is converted by reaction with cyanic acid or a corresponding isocyanate into a corresponding ureido compound and
if necessary any protecting group used to protect reactive groups during the reactions is cleaved or
if desired a compound of general formula I thus obtained is subsequently resolved into the stereoisomers thereof or
a compound of general formula I thus obtained is converted into the salts thereof, in particular for pharmaceutical use into the physiologically acceptable salts thereof with an inorganic or organic acid or base.

13. Process according to claim 12, **characterised in that**, in order to prepare a compound of general formula I wherein R₁ denotes an amino group, a compound of general formula 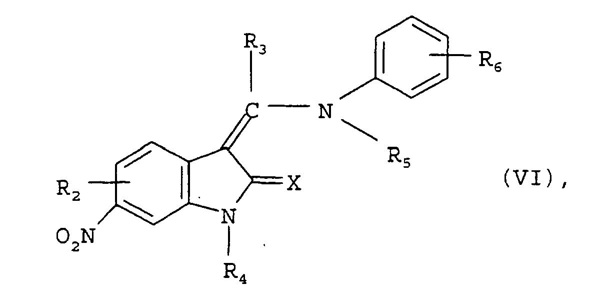
is reduced.

14. Process according to claim 12, **characterised in that**, in order to prepare a compound of general formula I wherein R₁ and/or R₂ denotes one of the substituted sulphinyl or sulphonyl groups mentioned in claims 1 to 7, a compound of general formula 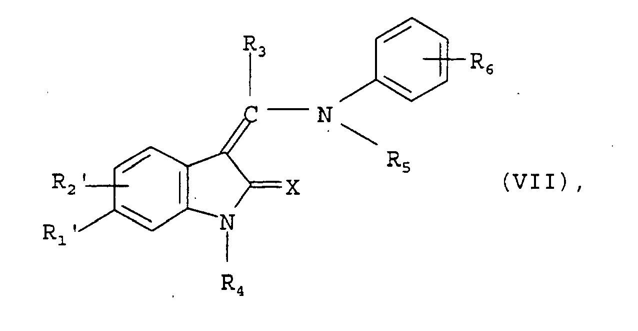
wherein
R₃ to R₆ are defined as in claims 1 to 7 and
one of the groups R₁' and R₂' denotes one of the substituted mercapto or sulphinyl groups mentioned for R₁ and R₂ in claims 1 to 6 and the other assumes the meanings given for R₁ or R₂ in claims 1 to 7 with the exception of the mercapto or sulphinyl groups or both groups R₁' and R₂' denote one of the substituted mercapto or sulphinyl groups mentioned for R₁ and R₂ in claims 1 to 6, is oxidised.
